# EUROPEAN PATENT APPLICATION

(11) **EP 3 991 741 A1**
(43) Date of publication of application: **04.05.2022**
(21) Application number: 20205209.8
(22) Date of filing: 02.11.2020
(51) Int. Cl.: A61K 35/766, C12N 7/00, C12N 15/86, A61P 35/00, A61K 35/768, A61K 48/00, A61K 49/00, A61K 38/17, C07K 14/705

(54) **AN IMMUNE CHECKPOINT-MODULATING VSV-NDV HYBRID VIRUS FOR ONCOLYTIC VIRUS IMMONOTHERAPY OF CANCER**

(71) Applicant: Klinikum rechts der Isar der Technischen Universität München, 81675 München (DE)
(72) Inventor: Altomonte, Jennifer, 81249 München (DE); Krabbe, Teresa, 80796 München (DE)
(74) Representative: Engelhard, Markus

(57) **Abstract**

The present invention relates to a recombinant oncolytic virus. The present invention further relates to a nucleic acid encoding a recombinant oncolytic virus. The present invention also relates to a vector comprising a nucleic acid encoding a recombinant oncolytic virus. Furthermore, the present invention relates to a pharmaceutical composition comprising a recombinant oncolytic virus. The present invention further relates a virus, a nucleic acid, a vector, and a pharmaceutical composition for use in medicine, such as for use in the prevention and/or treatment of cancer. Moreover, the present invention relates to the use of a virus, a nucleic acid, a vector, and a pharmaceutical composition as gene delivery tool, (noninvasive) imaging of virus biodistribution, and/or for tumor detection.

SEQUENCE LISTING

<110> Klinikum rechts der Isar der Technischen Universität München

<120> An immune checkpoint-modulating VSV-NDV hybrid virus for oncolytic
virus immunotherapy of cancer

<130> K31866EP

<160> 26

<170> BiSSAP 1.3.6

<210> 1
<211> 288
<212> PRT
<213> Homo sapiens

<220>
<223> Human PD1

<400> 1
Met Gln Ile Pro Gln Ala Pro Trp Pro Val Val Trp Ala Val Leu Gln
1               5                   10                  15
Leu Gly Trp Arg Pro Gly Trp Phe Leu Asp Ser Pro Asp Arg Pro Trp
            20                  25                  30
Asn Pro Pro Thr Phe Ser Pro Ala Leu Leu Val Val Thr Glu Gly Asp
        35                  40                  45
Asn Ala Thr Phe Thr Cys Ser Phe Ser Asn Thr Ser Glu Ser Phe Val
    50                  55                  60
Leu Asn Trp Tyr Arg Met Ser Pro Ser Asn Gln Thr Asp Lys Leu Ala
65                  70                  75                  80
Ala Phe Pro Glu Asp Arg Ser Gln Pro Gly Gln Asp Cys Arg Phe Arg
                85                  90                  95
Val Thr Gln Leu Pro Asn Gly Arg Asp Phe His Met Ser Val Val Arg
            100                 105                 110
Ala Arg Arg Asn Asp Ser Gly Thr Tyr Leu Cys Gly Ala Ile Ser Leu
        115                 120                 125
Ala Pro Lys Ala Gln Ile Lys Glu Ser Leu Arg Ala Glu Leu Arg Val
    130                 135                 140
Thr Glu Arg Arg Ala Glu Val Pro Thr Ala His Pro Ser Pro Ser Pro
145                 150                 155                 160
Arg Pro Ala Gly Gln Phe Gln Thr Leu Val Val Gly Val Val Gly Gly
                165                 170                 175
Leu Leu Gly Ser Leu Val Leu Leu Val Trp Val Leu Ala Val Ile Cys
            180                 185                 190
Ser Arg Ala Ala Arg Gly Thr Ile Gly Ala Arg Arg Thr Gly Gln Pro
        195                 200                 205
Leu Lys Glu Asp Pro Ser Ala Val Pro Val Phe Ser Val Asp Tyr Gly
    210                 215                 220
Glu Leu Asp Phe Gln Trp Arg Glu Lys Thr Pro Glu Pro Pro Val Pro
225                 230                 235                 240
Cys Val Pro Glu Gln Thr Glu Tyr Ala Thr Ile Val Phe Pro Ser Gly
                245                 250                 255
Met Gly Thr Ser Ser Pro Ala Arg Arg Gly Ser Ala Asp Gly Pro Arg
            260                 265                 270
Ser Ala Gln Pro Leu Arg Pro Glu Asp Gly His Cys Ser Trp Pro Leu
        275                 280                 285

<210> 2

<211> 170
<212> PRT
<213> Homo sapiens

<220>
<223> Human PD1 (soluble)

<400> 2
Met Gln Ile Pro Gln Ala Pro Trp Pro Val Val Trp Ala Val Leu Gln
1               5                   10                  15
Leu Gly Trp Arg Pro Gly Trp Phe Leu Asp Ser Pro Asp Arg Pro Trp
            20                  25                  30
Asn Pro Pro Thr Phe Ser Pro Ala Leu Leu Val Val Thr Glu Gly Asp
        35                  40                  45
Asn Ala Thr Phe Thr Cys Ser Phe Ser Asn Thr Ser Glu Ser Phe Val
    50                  55                  60
Leu Asn Trp Tyr Arg Met Ser Pro Ser Asn Gln Thr Asp Lys Leu Ala
65                  70                  75                  80
Ala Phe Pro Glu Asp Arg Ser Gln Pro Gly Gln Asp Cys Arg Phe Arg
                85                  90                  95
Val Thr Gln Leu Pro Asn Gly Arg Asp Phe His Met Ser Val Val Arg
            100                 105                 110
Ala Arg Arg Asn Asp Ser Gly Thr Tyr Leu Cys Gly Ala Ile Ser Leu
        115                 120                 125
Ala Pro Lys Ala Gln Ile Lys Glu Ser Leu Arg Ala Glu Leu Arg Val
    130                 135                 140
Thr Glu Arg Arg Ala Glu Val Pro Thr Ala His Pro Ser Pro Ser Pro
145                 150                 155                 160
Arg Pro Ala Gly Gln Phe Gln Thr Leu Val
                165                 170

<210> 3
<211> 170
<212> PRT
<213> Artificial Sequence

<220>
<223> High Affinity PD1 (soluble)

<400> 3
Met Gln Ile Pro Gln Ala Pro Trp Pro Val Val Trp Ala Val Leu Gln
1               5                   10                  15
Leu Gly Trp Arg Pro Gly Trp Phe Leu Asp Ser Pro Asp Arg Pro Trp
            20                  25                  30
Asn Pro Pro Thr Phe Ser Pro Ala Leu Leu Val Val Thr Glu Gly Asp
        35                  40                  45
Asn Ala Thr Phe Thr Cys Ser Phe Ser Asn Thr Ser Glu Ser Phe Val
    50                  55                  60
Leu Asn Trp Tyr Arg Met Ser Pro Ser Asn Gln Thr Asp Lys Leu Ala
65                  70                  75                  80
Ala Phe Pro Glu Asp Arg Ser Gln Pro Gly Gln Asp Cys Arg Phe Arg
                85                  90                  95
Val Thr Gln Leu Pro Asn Gly Arg Asp Phe His Met Ser Val Val Arg
            100                 105                 110
Ala Arg Arg Asn Asp Ser Gly Thr Tyr Leu Cys Gly Ala Ile Ser Leu
        115                 120                 125
Ala Pro Lys Leu Gln Ile Lys Glu Ser Leu Arg Ala Glu Leu Arg Val
    130                 135                 140
Thr Glu Arg Arg Ala Glu Val Pro Thr Ala His Pro Ser Pro Ser Pro
145                 150                 155                 160
Arg Pro Ala Gly Gln Phe Gln Thr Leu Val

165                 170

<210> 4
<211> 553
<212> PRT
<213> Artificial Sequence

<220>
<223> NDV F3aa-modified fusion protein with L289A (amino acid sequence)

<400> 4
Met Gly Ser Arg Pro Phe Thr Lys Asn Pro Ala Pro Met Met Leu Thr
1               5                   10                  15
Ile Arg Val Ala Leu Val Leu Ser Cys Ile Cys Pro Ala Asn Ser Ile
            20                  25                  30
Asp Gly Arg Pro Phe Ala Ala Ala Gly Ile Val Val Thr Gly Asp Lys
        35                  40                  45
Ala Val Asn Ile Tyr Thr Ser Ser Gln Thr Gly Ser Ile Ile Val Lys
    50                  55                  60
Leu Leu Pro Asn Leu Pro Lys Asp Lys Glu Ala Cys Ala Lys Ala Pro
65                  70                  75                  80
Leu Asp Ala Tyr Asn Arg Thr Leu Thr Thr Leu Leu Thr Pro Leu Gly
                85                  90                  95
Asp Ser Ile Arg Arg Ile Gln Glu Ser Val Thr Thr Ser Gly Gly Arg
            100                 105                 110
Arg Gln Arg Arg Phe Ile Gly Ala Ile Ile Gly Gly Val Ala Leu Gly
        115                 120                 125
Val Ala Thr Ala Ala Gln Ile Thr Ala Ala Ala Ala Leu Ile Gln Ala
    130                 135                 140
Lys Gln Asn Ala Ala Asn Ile Leu Arg Leu Lys Glu Ser Ile Ala Ala
145                 150                 155                 160
Thr Asn Glu Ala Val His Glu Val Thr Asp Gly Leu Ser Gln Leu Ala
                165                 170                 175
Val Ala Val Gly Lys Met Gln Gln Phe Val Asn Asp Gln Phe Asn Lys
            180                 185                 190
Thr Ala Gln Glu Leu Asp Cys Ile Lys Ile Ala Gln Gln Val Gly Val
        195                 200                 205
Glu Leu Asn Leu Tyr Leu Thr Glu Leu Thr Thr Val Phe Gly Pro Gln
    210                 215                 220
Ile Thr Ser Pro Ala Leu Asn Lys Leu Thr Ile Gln Ala Leu Tyr Asn
225                 230                 235                 240
Leu Ala Gly Gly Asn Met Asp Tyr Leu Leu Thr Lys Leu Gly Ile Gly
                245                 250                 255
Asn Asn Gln Leu Ser Ser Leu Ile Gly Ser Gly Leu Ile Thr Gly Asn
            260                 265                 270
Pro Ile Leu Tyr Asp Ser Gln Thr Gln Leu Leu Gly Ile Gln Val Thr
        275                 280                 285
Ala Pro Ser Val Gly Asn Leu Asn Asn Met Arg Ala Thr Tyr Leu Glu
    290                 295                 300
Thr Leu Ser Val Ser Thr Thr Arg Gly Phe Ala Ser Ala Leu Val Pro
305                 310                 315                 320
Lys Val Val Thr Gln Val Gly Ser Val Ile Glu Glu Leu Asp Thr Ser
                325                 330                 335
Tyr Cys Ile Glu Thr Asp Leu Asp Leu Tyr Cys Thr Arg Ile Val Thr
            340                 345                 350
Phe Pro Met Ser Pro Gly Ile Tyr Ser Cys Leu Ser Gly Asn Thr Ser
        355                 360                 365
Ala Cys Met Tyr Ser Lys Thr Glu Gly Ala Leu Thr Thr Pro Tyr Met
    370                 375                 380
Thr Ile Lys Gly Ser Val Ile Ala Asn Cys Lys Met Thr Thr Cys Arg
385                 390                 395                 400
Cys Val Asn Pro Pro Gly Ile Ile Ser Gln Asn Tyr Gly Glu Ala Val

                    405                     410                     415
Ser Leu Ile Asp Lys Gln Ser Cys Asn Val Leu Ser Leu Gly Gly Ile
                420                     425                     430
Thr Leu Arg Leu Ser Gly Glu Phe Asp Val Thr Tyr Gln Lys Asn Ile
            435                     440                     445
Ser Ile Gln Asp Ser Gln Val Ile Ile Thr Gly Asn Leu Asp Ile Ser
    450                     455                     460
Thr Glu Leu Gly Asn Val Asn Asn Ser Ile Ser Asn Ala Leu Asn Lys
465                     470                     475                     480
Leu Glu Glu Ser Asn Arg Lys Leu Asp Lys Val Asn Val Lys Leu Thr
                485                     490                     495
Ser Thr Ser Ala Leu Ile Thr Tyr Ile Val Leu Thr Ile Ile Ser Leu
            500                     505                     510
Val Phe Gly Ile Leu Ser Leu Ile Leu Ala Cys Tyr Leu Met Tyr Lys
        515                     520                     525
Gln Lys Ala Gln Gln Lys Thr Leu Leu Trp Leu Gly Asn Asn Thr Leu
    530                     535                     540
Asp Gln Met Arg Ala Thr Thr Lys Met
545                     550

<210> 5
<211> 577
<212> PRT
<213> Newcastle disease virus


<220>
<223> NDV HN protein (amino acid sequence)

<400> 5
Met Asp Arg Ala Val Ser Gln Val Ala Leu Glu Asn Asp Glu Arg Glu
1               5                   10                  15
Ala Lys Asn Thr Trp Arg Leu Ile Phe Arg Ile Ala Ile Leu Phe Leu
            20                  25                  30
Thr Val Val Thr Leu Ala Ile Ser Val Ala Ser Leu Leu Tyr Ser Met
        35                  40                  45
Gly Ala Ser Thr Pro Ser Asp Leu Val Gly Ile Pro Thr Arg Ile Ser
    50                  55                  60
Arg Ala Glu Glu Lys Ile Thr Ser Thr Leu Gly Ser Asn Gln Asp Val
65                  70                  75                  80
Val Asp Arg Ile Tyr Lys Gln Val Ala Leu Glu Ser Pro Leu Ala Leu
                85                  90                  95
Leu Asn Thr Glu Thr Thr Ile Met Asn Ala Ile Thr Ser Leu Ser Tyr
            100                 105                 110
Gln Ile Asn Gly Ala Ala Asn Asn Ser Gly Trp Gly Ala Pro Ile His
        115                 120                 125
Asp Pro Asp Tyr Ile Gly Gly Ile Gly Lys Glu Leu Ile Val Asp Asp
    130                 135                 140
Ala Ser Asp Val Thr Ser Phe Tyr Pro Ser Ala Phe Gln Glu His Leu
145                 150                 155                 160
Asn Phe Ile Pro Ala Pro Thr Thr Gly Ser Gly Cys Thr Arg Ile Pro
                165                 170                 175
Ser Phe Asp Met Ser Ala Thr His Tyr Cys Tyr Thr His Asn Val Ile
            180                 185                 190
Leu Ser Gly Cys Arg Asp His Ser His Ser His Gln Tyr Leu Ala Leu
        195                 200                 205
Gly Val Leu Arg Thr Ser Ala Thr Gly Arg Val Phe Phe Ser Thr Leu
    210                 215                 220
Arg Ser Ile Asn Leu Asp Asp Thr Gln Asn Arg Lys Ser Cys Ser Val
225                 230                 235                 240
Ser Ala Thr Pro Leu Gly Cys Asp Met Leu Cys Ser Lys Ala Thr Glu
                245                 250                 255
Thr Glu Glu Glu Asp Tyr Asn Ser Ala Val Pro Thr Arg Met Val His

260                 265                 270
Gly Arg Leu Gly Phe Asp Gly Gln Tyr His Glu Lys Asp Leu Asp Val
        275                 280                 285
Thr Thr Leu Phe Gly Asp Trp Val Ala Asn Tyr Pro Gly Val Gly Gly
    290                 295                 300
Gly Ser Phe Ile Asp Ser Arg Val Trp Phe Ser Val Tyr Gly Gly Leu
305                 310                 315                 320
Lys Pro Asn Thr Pro Ser Asp Thr Val Gln Glu Gly Lys Tyr Val Ile
                325                 330                 335
Tyr Lys Arg Tyr Asn Asp Thr Cys Pro Asp Glu Gln Asp Tyr Gln Ile
            340                 345                 350
Arg Met Ala Lys Ser Ser Tyr Lys Pro Gly Arg Phe Gly Gly Lys Arg
        355                 360                 365
Ile Gln Gln Ala Ile Leu Ser Ile Lys Val Ser Thr Ser Leu Gly Glu
    370                 375                 380
Asp Pro Val Leu Thr Val Pro Pro Asn Thr Val Thr Leu Met Gly Ala
385                 390                 395                 400
Glu Gly Arg Ile Leu Thr Val Gly Thr Ser His Phe Leu Tyr Gln Arg
                405                 410                 415
Gly Ser Ser Tyr Phe Ser Pro Ala Leu Leu Tyr Pro Met Thr Val Ser
            420                 425                 430
Asp Lys Thr Ala Thr Leu His Ser Pro Tyr Thr Phe Asn Ala Phe Thr
        435                 440                 445
Arg Pro Gly Ser Ile Pro Cys Gln Ala Ser Ala Arg Cys Pro Asn Ser
    450                 455                 460
Cys Val Thr Gly Val Tyr Thr Asp Pro Tyr Pro Leu Ile Phe Tyr Arg
465                 470                 475                 480
Asn His Thr Leu Arg Gly Val Phe Gly Thr Met Leu Asp Gly Glu Gln
                485                 490                 495
Ala Arg Leu Asn Pro Ala Ser Ala Val Phe Asp Ser Thr Ser Arg Ser
            500                 505                 510
Arg Ile Thr Arg Val Ser Ser Ser Ser Ile Lys Ala Ala Tyr Thr Thr
        515                 520                 525
Ser Thr Cys Phe Lys Val Val Lys Thr Asn Lys Thr Tyr Cys Leu Ser
    530                 535                 540
Ile Ala Glu Ile Ser Asn Thr Leu Phe Gly Glu Phe Arg Ile Val Pro
545                 550                 555                 560
Leu Leu Val Glu Ile Leu Lys Asp Asp Gly Val Arg Glu Ala Arg Ser
                565                 570                 575
Gly

<210> 6
<211> 510
<212> DNA
<213> Artificial Sequence

<220>
<223> High affinity PD1 (soluble) nucleic acid sequence

<400> 6
atgcagatcc cacaggcgcc ctggccagtc gtctgggcgg tgctacaact gggctggcgg      60

ccaggatggt tcttagactc cccagacagg ccctggaacc cccccacctt ctccccagcc     120

ctgctcgtgg tgaccgaagg ggacaacgcc accttcacct gcagcttctc caacacatcg     180

gagagcttcg tgctaaactg gtaccgcatg agccccagca accagacgga caagctggcc     240

gccttccccg aggaccgcag ccagcccggc caggactgcc gcttccgtgt cacacaactg     300

cccaacgggc gtgacttcca catgagcgtg gtcagggccc ggcgcaatga cagcggcacc     360

tacctctgtg gggccatctc cctggccccc aagctgcaga tcaaagagag cctgcgggca     420

gagctcaggg tgacagagag aagggcagaa gtgcccacag cccaccccag cccctcaccc     480

aggccagccg gccagttcca aaccctggtg                                      510

<210> 7
<211> 684
<212> DNA
<213> Artificial Sequence

<220>
<223> Human IgG1-Fc; CH2 and CH3 domains of the human IgG1 heavy chain
      and the hinge region.

<400> 7
gacaaaactc acacatgccc accgtgccca gcacctgaac tcctgggggg accgtcagtc      60

ttcctcttcc ccccaaaacc caaggacacc ctcatgatct cccggacccc tgaggtcaca     120

tgcgtggtgg tggacgtgag ccacgaagac cctgaggtca agttcaactg gtacgtggac     180

ggcgtggagg tgcataatgc caagacaaag ccgcgggagg agcagtacaa cagcacgtac     240

cgtgtggtca gcgtcctcac cgtcctgcac caggactggc tgaatggcaa ggagtacaag     300

tgcaaggtct ccaacaaagc cctcccagcc cccatcgaga aaaccatctc caaagccaaa     360

gggcagcccc gagaaccaca ggtgtacacc ctgcccccat cccgggagga gatgaccaag     420

aaccaggtca gcctgacctg cctggtcaaa ggcttctatc ccagcgacat cgccgtggag     480

tgggagagca atgggcagcc ggagaacaac tacaagacca cgcctcccgt gctggactcc     540

gacggctcct tcttcctcta cagcaagctc accgtggaca agagcaggtg gcagcagggg     600

aacgtcttct catgctccgt gatgcacgag gctctgcaca accactacac gcagaagagc     660

ctctccctgt ctccgggtaa atga                                            684

<210> 8
<211> 1200
<212> DNA
<213> Artificial Sequence

<220>
<223> High affinity soluble PD1-IgG1-Fc fusion gene

<400> 8
atgcagatcc cacaggcgcc ctggccagtc gtctgggcgg tgctacaact gggctggcgg      60

ccaggatggt tcttagactc cccagacagg ccctggaacc cccccacctt ctccccagcc     120

ctgctcgtgg tgaccgaagg ggacaacgcc accttcacct gcagcttctc caacacatcg     180

gagagcttcg tgctaaactg gtaccgcatg agccccagca accagacgga caagctggcc     240

gccttccccg aggaccgcag ccagcccggc caggactgcc gcttccgtgt cacacaactg     300

cccaacgggc gtgacttcca catgagcgtg gtcagggccc ggcgcaatga cagcggcacc 360

tacctctgtg gggccatctc cctggccccc aagctgcaga tcaaagagag cctgcgggca 420

gagctcaggg tgacagagag aagggcagaa gtgcccacag cccaccccag cccctcaccc 480

aggccagccg gccagttcca aaccctggtg ctcgaggaca aaactcacac atgcccaccg 540

tgcccagcac ctgaactcct ggggggaccg tcagtcttcc tcttcccccc aaaacccaag 600

gacaccctca tgatctcccg gacccctgag gtcacatgcg tggtggtgga cgtgagccac 660

gaagaccctg aggtcaagtt caactggtac gtggacggcg tggaggtgca taatgccaag 720

acaaagccgc gggaggagca gtacaacagc acgtaccgtg tggtcagcgt cctcaccgtc 780

ctgcaccagg actggctgaa tggcaaggag tacaagtgca aggtctccaa caaagccctc 840

ccagccccca tcgagaaaac catctccaaa gccaaagggc agccccgaga accacaggtg 900

tacaccctgc ccccatcccg ggaggagatg accaagaacc aggtcagcct gacctgcctg 960

gtcaaaggct tctatcccag cgacatcgcc gtggagtggg agagcaatgg gcagccggag 1020

aacaactaca agaccacgcc tcccgtgctg gactccgacg gctccttctt cctctacagc 1080

aagctcaccg tggacaagag caggtggcag caggggaacg tcttctcatg ctccgtgatg 1140

cacgaggctc tgcacaacca ctacacgcag aagagcctct ccctgtctcc gggtaaatga 1200

<210> 9
<211> 17346
<212> DNA
<213> Artificial Sequence

<220>
<223> vector nucleic acid sequence

<400> 9
cacctaaatt gtaagcgtta atattttgtt aaaattcgcg ttaaattttt gttaaatcag 60

ctcatttttt aaccaatagg ccgaaatcgg caaaatccct tataaatcaa aagaatagac 120

cgagataggg ttgagtgttg ttccagtttg gaacaagagt ccactattaa agaacgtgga 180

ctccaacgtc aaagggcgaa aaaccgtcta tcagggcgat ggcccactac gtgaaccatc 240

accctaatca agttttttgg ggtcgaggtg ccgtaaagca ctaaatcgga accctaaagg 300

gagcccccga tttagagctt gacggggaaa gccggcgaac gtggcgagaa aggaagggaa 360

gaaagcgaaa ggagcgggcg ctagggcgct ggcaagtgta gcggtcacgc tgcgcgtaac 420

caccacaccc gccgcgctta atgcgccgct acagggcgcg tcccattcgc cattcaggct 480

gcgcaactgt tgggaagggc gatcggtgcg ggcctcttcg ctattacgcc agctggcgaa 540

agggggatgt gctgcaaggc gattaagttg ggtaacgcca gggttttccc agtcacgacg 600

ttgtaaaacg acggccagtg aattgtaata cgactcacta taggacgaag acaaacaaac 660

cattattatc attaaaaggc tcaggagaaa ctttaacagt aatcaaaatg tctgttacag 720

tcaagagaat cattgacaac acagtcgtag ttccaaaact tcctgcaaat gaggatccag 780

tggaataccc ggcagattac ttcagaaaat caaaggagat tcctctttac atcaatacta 840

caaaaagttt gtcagatcta agaggatatg tctaccaagg cctcaaatcc ggaaatgtat 900

caatcataca tgtcaacagc tacttgtatg gagcattaaa ggacatccgg ggtaagttgg 960

ataaagattg gtcaagtttc ggaataaaca tcgggaaagc aggggataca atcggaatat 1020

ttgaccttgt atccttgaaa gccctggacg gcgtacttcc agatggagta tcggatgctt 1080

ccagaaccag cgcagatgac aaatggttgc ctttgtatct acttggctta tacagagtgg 1140

gcagaacaca aatgcctgaa tacagaaaaa agctcatgga tgggctgaca aatcaatgca 1200

aaatgatcaa tgaacagttt gaacctcttg tgccagaagg tcgtgacatt tttgatgtgt 1260

ggggaaatga cagtaattac acaaaaattg tcgctgcagt ggacatgttc ttccacatgt 1320

tcaaaaaaca tgaatgtgcc tcgttcagat acggaactat tgtttccaga ttcaaagatt 1380

gtgctgcatt ggcaacattt ggacacctct gcaaaataac cggaatgtct acagaagatg 1440

taacgacctg gatcttgaac cgagaagttg cagatgaaat ggtccaaatg atgcttccag 1500

gccaagaaat tgacaaggcc gattcataca tgccttattt gatcgacttt ggattgtctt 1560

ctaagtctcc atattcttcc gtcaaaaacc ctgccttcca cttctggggg caattgacag 1620

ctcttctgct cagatccacc agagcaagga atgcccgaca gcctgatgac attgagtata 1680

catctcttac tacagcaggt ttgttgtacg cttatgcagt aggatcctct gccgacttgg 1740

cacaacagtt ttgtgttgga gataacaaat acactccaga tgatagtacc ggaggattga 1800

cgactaatgc accgccacaa ggcagagatg tggtcgaatg gctcggatgg tttgaagatc 1860

aaaacagaaa accgactcct gatatgatgc agtatgcgaa aagagcagtc atgtcactgc 1920

aaggcctaag agagaagaca attggcaagt atgctaagtc agaatttgac aaatgaccct 1980

ataattctca gatcacctat tatatattat gctacatatg aaaaaaacta acagatatca 2040

tggataatct cacaaaagtt cgtgagtatc tcaagtccta ttctcgtctg gatcaggcgg 2100

taggagagat agatgagatc gaagcacaac gagctgaaaa gtccaattat gagttgttcc 2160

aagaggatgg agtggaagag catactaagc cctcttattt tcaggcagca gatgattctg 2220

acacagaatc tgaaccagaa attgaagaca atcaaggctt gtatgcacca gatccagaag 2280

ctgagcaagt tgaaggcttt atacaggggc ctttagatga ctatgcagat gaggaagtgg 2340

atgttgtatt tacttcggac tggaaacagc ctgagcttga atctgacgag catggaaaga 2400

ccttacggtt gacatcgcca gagggtttaa gtggagagca gaaatcccag tggctttcga 2460

cgattaaagc agtcgtgcaa agtgccaaat actggaatct ggcagagtgc acatttgaag 2520

catcgggaga aggggtcatt atgaaggagc gccagataac tccggatgta tataaggtca 2580

ctccagtgat gaacacacat ccgtcccaat cagaagcagt atcagatgtt tggtctctct 2640

caaagacatc catgactttc caacccaaga aagcaagtct tcagcctctc accatatcct 2700

tggatgaatt gttctcatct agaggagagt tcatctctgt cggaggtgac ggacgaatgt 2760

ctcataaaga ggccatcctg ctcggcctga gatacaaaaa gttgtacaat caggcgagag 2820

tcaaatattc tctgtagact atgaaaaaaa gtaacagata tcacgatcta agtgttatcc 2880

caatccattc atcatgagtt ccttaaagaa gattctcggt ctgaagggga aaggtaagaa 2940

atctaagaaa ttagggatcg caccaccccc ttatgaagag gacactagca tggagtatgc 3000

tccgagcgct ccaattgaca aatcctattt tggagttgac gagatggaca cctatgatcc 3060

gaatcaatta agatatgaga aattcttctt tacagtgaaa atgacggtta gatctaatcg 3120

tccgttcaga acatactcag atgtggcagc cgctgtatcc cattgggatc acatgtacat 3180

cggaatggca gggaaacgtc ccttctacaa aatcttggct tttttgggtt cttctaatct 3240

aaaggccact ccagcggtat tggcagatca aggtcaacca gagtatcacg ctcactgcga 3300

aggcagggct tatttgccac ataggatggg gaagacccct cccatgctca atgtaccaga 3360

gcacttcaga agaccattca atataggtct ttacaaggga acgattgagc tcacaatgac 3420

catctacgat gatgagtcac tggaagcagc tcctatgatc tgggatcatt tcaattcttc 3480

caaattttct gatttcagag agaaggcctt aatgtttggc ctgattgtcg agaaaaaggc 3540

atctggagcg tgggtcctgg actctatcgg ccacttcaaa tgagctagtc taacttctag 3600

cttctgaaca atccccggtt tactcagtct cccctaattc cagcctctcg aacaactaat 3660

atcctgtctt ttctatccct atgaaaaaaa ctaacagaga tcgatctgtt tacgcgtcga 3720

gatgggctcc agaccttcta ccaagaaccc agcacctatg atgctgacta tccgggtcgc 3780

gctggtactg agttgcatct gcccggcaaa ctccattgat ggcaggcctc ttgcagctgc 3840

aggaattgtg gttacaggag acaaagcagt caacatatac acctcatccc agacaggatc 3900

aatcatagtt aagctcctcc cgaatctgcc caaggataag gaggcatgtg cgaaagcccc 3960

cttggatgca tacaacagga cattgaccac tttgctcacc ccccttggtg actctatccg 4020

taggatacaa gagtctgtga ctacatctgg agggcggaga cagaggcgct ttataggcgc 4080

cattattggc ggtgtggctc ttggggttgc aactgccgca caaataacag cggccgcagc 4140

tctgatacaa gccaaacaaa atgctgccaa catcctccga cttaaagaga gcattgccgc 4200

aaccaatgag gctgtgcatg aggtcactga cggattatcg caactagcag tggcagttgg 4260

gaagatgcag cagtttgtta atgaccaatt taataaaaca gctcaggaat tagactgcat 4320

caaaattgca cagcaagttg gtgtagagct caacctgtac ctaaccgaat tgactacagt 4380

attcggacca caaatcactt cacctgcctt aaacaagctg actattcagg cactttacaa 4440

tctagctggt gggaatatgg attacttatt gactaagtta ggtataggga acaatcaact 4500

cagctcatta atcggtagcg gcttaatcac cggtaaccct attctatacg actcacagac 4560

tcaactcttg ggtatacagg taactgcacc ttcagtcggg aacctaaata atatgcgtgc 4620

cacctacttg gaaaccttat ccgtaagcac aaccagggga tttgcctcgg cacttgtccc 4680

aaaagtggtg acacaggtcg gttctgtgat agaagaactt gacacctcat actgtataga 4740

aactgactta gatttatatt gtacaagaat agtaacgttc cctatgtccc ctggtattta 4800

ctcctgcttg agcggcaata catcggcctg tatgtactca aagaccgaag gcgcacttac 4860

tacaccatat atgactatca aaggctcagt catcgctaac tgcaagatga caacatgtag 4920

atgtgtaaac cccccgggta tcatatcgca aaactatgga gaagccgtgt ctctaataga 4980

taaacaatca tgcaatgttt tatccttagg cgggataact ttaaggctca gtggggaatt 5040

cgatgtaact tatcagaaga atatctcaat acaagattct caagtaataa taacaggcaa 5100

tcttgatatc tcaactgagc ttgggaatgt caacaactcg atcagtaatg ctttgaataa 5160

gttagaggaa agcaacagaa aactagacaa agtcaatgtc aaactgacca gcacatctgc 5220

tctcattacc tatatcgttt tgactatcat atctcttgtt tttggtatac ttagcctgat 5280

tctagcatgc tacctaatgt acaagcaaaa ggcgcaacaa aagaccttat tatggcttgg 5340

gaataatacc ctagatcaga tgagagccac tacaaaaatg tgagctagct taattaatat 5400

gaaaaaaact aacagatatc atggaccgcg ccgttagcca agttgcgtta gagaatgatg 5460

aaagagaggc aaaaaataca tggcgcttga tattccggat tgcaatctta ttcttaacag 5520

tagtgacctt ggctatatct gtagcctccc ttttatatag catgggggct agcacaccta 5580

gcgatcttgt aggcataccg actaggattt ccagggcaga agaaaagatt acatctacac 5640

ttggttccaa tcaagatgta gtagatagga tatataagca agtggccctt gagtctccat 5700

tggcattgtt aaatactgag accacaatta tgaacgcaat aacatctctc tcttatcaga 5760

ttaatggagc tgcaaacaac agcgggtggg ggcacctatt tcatgaccca gattatatag 5820

gggggatagg caaagaactc attgtagatg atgctagtga tgtcacatca ttctatccct 5880

ctgcatttca agaacatctg aattttatcc cggcgcctac tacaggatca ggttgcactc 5940

gaataccctc atttgacatg agtgctaccc attactgcta cacccataat gtaatattgt 6000

ctggatgcag agatcactca cactcacatc agtatttagc acttggtgtg ctccggacat 6060

ctgcaacagg gagggtattc ttttctactc tgcgttccat caacctggac gacacccaaa 6120

atcggaagtc ttgcagtgtg agtgcaactc ccctgggttg tgatatgctg tgctcgaaag 6180

ccacggagac agaggaagaa gattataact cagctgtccc tacgcggatg gtacatggga 6240

ggttagggtt cgacggccaa tatcacgaaa aggacctaga tgtcacaaca ttattcgggg 6300

actgggtggc caactaccca ggagtagggg gtggatcttt tattgacagc cgcgtatggt 6360

tctcagtcta cggaggttta aaacccaata cacccagtga cactgtacag gaagggaaat 6420

atgtgatata caagcgatac aatgacacat gcccagatga gcaagactac cagattcgaa 6480

tggccaagtc ttcgtataag cctggacggt ttggtgggaa acgcatacag caggctatct 6540

tatctatcaa agtgtcaaca tccttaggcg aagacccggt actgactgta ccgcccaaca 6600

cagtcacact catgggggcc gaaggcagaa ttctcacagt agggacatcc catttcttgt 6660

atcagcgagg gtcatcatac ttctctcccg cgttattata tcctatgaca gtcagcaaca 6720

aaacagccac tcttcatagt ccttatacat tcaatgcctt cactcggcca ggtagtatcc 6780

cttgccaggc ttcagcaaga tgccccaact cgtgtgttac tggagtctat acagatccat 6840

atcccctaat cttctataga aaccacacct tgcgaggggt attcgggaca atgcttgatg 6900

gtgaacaagc aagacttaac cctgcgtctg cagtattcga tagcacatcc cgcagtcgca 6960

taactcgagt gagttcaagc agcatcaaag cagcatacac aacatcaact tgttttaaag 7020

tggtcaagac caataagacc tattgtctca gcattgctga aatatctaat actctcttcg 7080

gagaattcag aatcgtcccg ttactagttg agatcctcaa agatgacggg gttagagaag 7140

ccaggtctgg ctaggtttaa acagctaggc gcgcctatga aaaaaactaa cagcaatcat 7200

gcagatccca caggcgccct ggccagtcgt ctgggcggtg ctacaactgg gctggcggcc 7260

aggatggttc ttagactccc cagacaggcc ctggaacccc cccaccttct ccccagccct 7320

gctcgtggtg accgaagggg acaacgccac cttcacctgc agcttctcca acacatcgga 7380

gagcttcgtg ctaaactggt accgcatgag ccccagcaac cagacggaca agctggccgc 7440

cttccccgag gaccgcagcc agcccggcca ggactgccgc ttccgtgtca cacaactgcc 7500

caacgggcgt gacttccaca tgagcgtggt cagggcccgg cgcaatgaca gcggcaccta 7560

cctctgtggg gccatctccc tggcccccaa gctgcagatc aaagagagcc tgcgggcaga 7620

gctcagggtg acagagagaa gggcagaagt gcccacagcc caccccagcc cctcacccag 7680

gccagccggc cagttccaaa ccctggtgct cgaggacaaa actcacacat gcccaccgtg 7740

cccagcacct gaactcctgg ggggaccgtc agtcttcctc ttccccccaa aacccaagga 7800

caccctcatg atctcccgga cccctgaggt cacatgcgtg gtggtggacg tgagccacga 7860

agaccctgag gtcaagttca actggtacgt ggacggcgtg gaggtgcata atgccaagac 7920

aaagccgcgg gaggagcagt acaacagcac gtaccgtgtg gtcagcgtcc tcaccgtcct 7980

gcaccaggac tggctgaatg gcaaggagta caagtgcaag gtctccaaca aagccctccc 8040

agcccccatc gagaaaacca tctccaaagc caaagggcag ccccgagaac cacaggtgta 8100

caccctgccc ccatcccggg aggagatgac caagaaccag gtcagcctga cctgcctggt 8160

caaaggcttc tatcccagcg acatcgccgt ggagtgggag agcaatgggc agccggagaa 8220

caactacaag accacgcctc ccgtgctgga ctccgacggc tccttcttcc tctacagcaa 8280

gctcaccgtg gacaagagca ggtggcagca ggggaacgtc ttctcatgct ccgtgatgca 8340

cgaggctctg cacaaccact acacgcagaa gagcctctcc ctgtctccgg gtaaatgacc 8400

tagggctagc ggcctcaatt atatttgagt ttttaatttt tatgaaaaaa actaacagca 8460

atcatggaag tccacgattt tgagaccgac gagttcaatg atttcaatga agatgactat 8520

gccacaagag aattcctgaa tcccgatgag cgcatgacgt acttgaatca tgctgattac 8580

aacctgaatt ctcctctaat tagtgatgat attgacaatt taatcaggaa attcaattct 8640

cttccaattc cctcgatgtg ggatagtaag aactgggatg gagttcttga gatgttaacg 8700

tcatgtcaag ccaatcccat cccaacatct cagatgcata aatggatggg aagttggtta 8760

atgtctgata atcatgatgc cagtcaaggg tatagttttt tacatgaagt ggacaaagag 8820

gcagaaataa catttgacgt ggtggagacc ttcatccgcg gctggggcaa caaaccaatt 8880

gaatacatca aaaaggaaag atggactgac tcattcaaaa ttctcgctta tttgtgtcaa 8940

aagtttttgg acttacacaa gttgacatta atcttaaatg ctgtctctga ggtggaattg 9000

ctcaacttgg cgaggacttt caaaggcaaa gtcagaagaa gttctcatgg aacgaacata 9060

tgcaggatta gggttcccag cttgggtcct acttttattt cagaaggatg ggcttacttc 9120

aagaaacttg atattctaat ggaccgaaac tttctgttaa tggtcaaaga tgtgattata 9180

gggaggatgc aaacggtgct atccatggta tgtagaatag acaacctgtt ctcagagcaa 9240

gacatcttct cccttctaaa tatctacaga attggagata aaattgtgga gaggcaggga 9300

aatttttctt atgacttgat taaaatggtg gaaccgatat gcaacttgaa gctgatgaaa 9360

ttagcaagag aatcaaggcc tttagtccca caattccctc attttgaaaa tcatatcaag 9420

acttctgttg atgaaggggc aaaaattgac cgaggtataa gattcctcca tgatcagata 9480

atgagtgtga aaacagtgga tctcacactg gtgatttatg gatcgttcag acattggggt 9540

catcctttta tagattatta cactggacta gaaaaattac attcccaagt aaccatgaag 9600

aaagatattg atgtgtcata tgcaaaagca cttgcaagtg atttagctcg gattgttcta 9660

tttcaacagt tcaatgatca taaaaagtgg ttcgtgaatg gagacttgct ccctcatgat 9720

catcccttta aaagtcatgt taaagaaaat acatggccca cagctgctca agttcaagat 9780

tttggagata aatggcatga acttccgctg attaaatgtt ttgaaatacc cgacttacta 9840

gacccatcga taatatactc tgacaaaagt cattcaatga ataggtcaga ggtgttgaaa 9900

catgtccgaa tgaatccgaa cactcctatc cctagtaaaa aggtgttgca gactatgttg 9960

gacacaaagg ctaccaattg gaaagaattt cttaaagaga ttgatgagaa gggcttagat 10020

gatgatgatc taattattgg tcttaaagga aaggagaggg aactgaagtt ggcaggtaga 10080

tttttctccc taatgtcttg gaaattgcga gaatactttg taattaccga atatttgata 10140

aagactcatt tcgtccctat gtttaaaggc ctgacaatgg cggacgatct aactgcagtc 10200

attaaaaaga tgttagattc ctcatccggc caaggattga agtcatatga ggcaatttgc 10260

atagccaatc acattgatta cgaaaaatgg aataaccacc aaaggaagtt atcaaacggc 10320

ccagtgttcc gagttatggg ccagttctta ggttatccat ccttaatcga gagaactcat 10380

gaattttttg agaaaagtct tatatactac aatggaagac cagacttgat gcgtgttcac 10440

aacaacacac tgatcaattc aacctcccaa cgagtttgtt ggcaaggaca agagggtgga 10500

ctggaaggtc tacggcaaaa aggatggagt atcctcaatc tactggttat tcaaagagag 10560

gctaaaatca gaaacactgc tgtcaaagtc ttggcacaag gtgataatca agttatttgc 10620

acacagtata aaacgaagaa atcgagaaac gttgtagaat tacagggtgc tctcaatcaa 10680

atggtttcta ataatgagaa aattatgact gcaatcaaaa tagggacagg gaagttagga 10740

cttttgataa atgacgatga gactatgcaa tctgcagatt acttgaatta tggaaaaata 10800

ccgattttcc gtggagtgat tagagggtta gagaccaaga gatggtcacg agtgacttgt 10860

gtcaccaatg accaaatacc cacttgtgct aatataatga gctcagtttc cacaaatgct 10920

ctcaccgtag ctcattttgc tgagaaccca atcaatgcca tgatacagta caattatttt 10980

gggacatttg ctagactctt gttgatgatg catgatcctg ctcttcgtca atcattgtat 11040

gaagttcaag ataagatacc gggcttgcac agttctactt tcaaatacgc catgttgtat 11100

ttggaccctt ccattggagg agtgtcgggc atgtctttgt ccaggttttt gattagagcc 11160

ttcccagatc ccgtaacaga aagtctctca ttctggagat tcatccatgt acatgctcga 11220

agtgagcatc tgaaggagat gagtgcagta tttggaaacc ccgagatagc caagtttcga 11280

ataactcaca tagacaagct agtagaagat ccaacctctc tgaacatcgc tatgggaatg 11340

agtccagcga acttgttaaa gactgaggtt aaaaaatgct taatcgaatc aagacaaacc 11400

atcaggaacc aggtgattaa ggatgcaacc atatatttgt atcatgaaga ggatcggctc 11460

agaagtttct tatggtcaat aaatcctctg ttccctagat ttttaagtga attcaaatca 11520

ggcacttttt tgggagtcgc agacgggctc atcagtctat ttcaaaattc tcgtactatt 11580

cggaactcct ttaagaaaaa gtatcatagg gaattggatg atttgattgt gaggagtgag 11640

gtatcctctt tgacacattt agggaaactt catttgagaa ggggatcatg taaaatgtgg 11700

acatgttcag ctactcatgc tgacacatta agatacaaat cctgggggcg tacagttatt 11760

gggacaactg taccccatcc attagaaatg ttgggtccac aacatcgaaa agagactcct 11820

tgtgcaccat gtaacacatc agggttcaat tatgtttctg tgcattgtcc agacgggatc 11880

catgacgtct ttagttcacg gggaccattg cctgcttatc tagggtctaa aacatctgaa 11940

tctacatcta ttttgcagcc ttgggaaagg gaaagcaaag tcccactgat taaaagagct 12000

acacgtctta gagatgctat ctcttggttt gttgaacccg actctaaact agcaatgact 12060

atactttcta acatccactc tttaacaggc gaagaatgga ccaaaaggca gcatgggttc 12120

aaaagaacag ggtctgccct tcataggttt tcgacatctc ggatgagcca tggtgggttc 12180

gcatctcaga gcactgcagc attgaccagg ttgatggcaa ctacagacac catgagggat 12240

ctgggagatc agaatttcga ctttttattc caagcaacgt tgctctatgc tcaaattacc 12300

accactgttg caagagacgg atggatcacc agttgtacag atcattatca tattgcctgt 12360

aagtcctgtt tgagacccat agaagagatc accctggact caagtatgga ctacacgccc 12420

ccagatgtat cccatgtgct gaagacatgg aggaatgggg aaggttcgtg gggacaagag 12480

ataaaacaga tctatccttt agaagggaat tggaagaatt tagcacctgc tgagcaatcc 12540

tatcaagtcg gcagatgtat aggttttcta tatggagact tggcgtatag aaaatctact 12600

catgccgagg acagttctct atttcctcta tctatacaag gtcgtattag aggtcgaggt 12660

ttcttaaaag ggttgctaga cggattaatg agagcaagtt gctgccaagt aatacaccgg 12720

agaagtctgg ctcatttgaa gaggccggcc aacgcagtgt acggaggttt gatttacttg 12780

attgataaat tgagtgtatc acctccattc ctttctctta ctagatcagg acctattaga 12840

gacgaattag aaacgattcc ccacaagatc ccaacctcct atccgacaag caaccgtgat 12900

atgggggtga ttgtcagaaa ttacttcaaa taccaatgcc gtctaattga aaagggaaaa 12960

tacagatcac attattcaca attatggtta ttctcagatg tcttatccat agacttcatt 13020

ggaccattct ctatttccac caccctcttg caaatcctat acaagccatt tttatctggg 13080

aaagataaga atgagttgag agagctggca aatctttctt cattgctaag atcaggagag 13140

gggtgggaag acatacatgt gaaattcttc accaaggaca tattattgtg tccagaggaa 13200

atcagacatg cttgcaagtt cgggattgct aaggataata ataaagacat gagctatccc 13260

ccttggggaa gggaatccag agggacaatt acaacaatcc ctgtttatta tacgaccacc 13320

ccttacccaa agatgctaga gatgcctcca agaatccaaa atcccctgct gtccggaatc 13380

aggttgggcc aattaccaac tggcgctcat tataaaattc ggagtatatt acatggaatg 13440

ggaatccatt acaggacttc cttgagttgt ggagacggct ccggagggat gactgctgca 13500

ttactacgag aaaatgtgca tagcagagga atattcaata gtctgttaga attatcaggg 13560

tcagtcatgc gaggcgcctc tcctgagccc cccagtgccc tagaaacttt aggaggagat 13620

aaatcgagat gtgtaaatgg tgaaacatgt tgggaatatc catctgactt atgtgaccca 13680

aggacttggg actatttcct ccgactcaaa gcaggcttgg ggcttcaaat tgatttaatt 13740

gtaatggata tggaagttcg ggattcttct actagcctga aaattgagac gaatgttaga 13800

aattatgtgc accggatttt ggatgagcaa ggagttttaa tctacaagac ttatggaaca 13860

tatatttgtg agagcgaaaa gaatgcagta acaatccttg gtcccatgtt caagacggtc 13920

gacttagttc aaacagaatt tagtagttct caaacgtctg aagtatatat ggtatgtaaa 13980

ggtttgaaga aattaatcga tgaacccaat cccgattggt cttccatcaa tgaatcctgg 14040

aaaaacctgt acgcattcca gtcatcagaa caggaatttg ccagagcaaa gaaggttagt 14100

acatacttta ccttgacagg tattccctcc caattcattc ctgatccttt tgtaaacatt 14160

gagactatgc tacaaatatt cggagtaccc acgggtgtgt ctcatgcggc tgccttaaaa 14220

tcatctgata gacctgcaga tttattgacc attagccttt tttatatggc gattatatcg 14280

tattataaca tcaatcatat cagagtagga ccgatacctc cgaacccccc atcagatgga 14340

attgcacaaa atgtggggat cgctataact ggtataagct tttggctgag tttgatggag 14400

aaagacattc cactatatca acagtgttta gcagttatcc agcaatcatt cccgattagg 14460

tgggaggctg tttcagtaaa aggaggatac aagcagaagt ggagtactag aggtgatggg 14520

ctcccaaaag atacccgaat ttcagactcc ttggccccaa tcgggaactg gatcagatct 14580

ctggaattgg tccgaaacca agttcgtcta aatccattca atgagatctt gttcaatcag 14640

ctatgtcgta cagtggataa tcatttgaaa tggtcaaatt tgcgaagaaa cacaggaatg 14700

attgaatgga tcaatagacg aatttcaaaa gaagaccggt ctatactgat gttgaagagt 14760

gacctacacg aggaaaactc ttggagagat taaaaaatca tgaggagact ccaaacttta 14820

agtatgaaaa aaactttgat ccttaagacc ctcttgtggt tttatttttt tatctggttt 14880

tgtggtcttc gtgggtcggc atggcatctc cacctcctcg cggtccgacc tgggcatccg 14940

aaggaggacg tcgtccactc ggatggctaa gggagggccc cccgcggggc tgctaacaaa 15000

gcccgaaagg aagctgagtt ggctgctgcc accgctgagc aataactagc ataacccctt 15060

ggggcctcta aacgggtctt gaggggtttt ttgctgaaag gaggaactat atccggatcg 15120

agacctcgat actagtgcgg tggagctcca gcttttgttc cctttagtga gggttaattt 15180

cgagcttggc gtaatcatgg tcatagctgt ttcctgtgtg aaattgttat ccgctcacaa 15240

ttccacacaa catacgagcc ggaagcataa agtgtaaagc ctggggtgcc taatgagtga 15300

gctaactcac attaattgcg ttgcgctcac tgcccgcttt ccagtcggga aacctgtcgt 15360

gccagctgca ttaatgaatc ggccaacgcg cggggagagg cggtttgcgt attgggcgct 15420

cttccgcttc ctcgctcact gactcgctgc gctcggtcgt tcggctgcgg cgagcggtat 15480

cagctcactc aaaggcggta atacggttat ccacagaatc aggggataac gcaggaaaga 15540

acatgtgagc aaaaggccag caaaaggcca ggaaccgtaa aaaggccgcg ttgctggcgt 15600

ttttccatag gctccgcccc cctgacgagc atcacaaaaa tcgacgctca agtcagaggt 15660

ggcgaaaccc gacaggacta taaagatacc aggcgtttcc ccctggaagc tccctcgtgc 15720

gctctcctgt tccgaccctg ccgcttaccg gatacctgtc cgcctttctc ccttcgggaa 15780

gcgtggcgct ttctcatagc tcacgctgta ggtatctcag ttcggtgtag gtcgttcgct 15840

ccaagctggg ctgtgtgcac gaaccccccg ttcagcccga ccgctgcgcc ttatccggta 15900

actatcgtct tgagtccaac ccggtaagac acgacttatc gccactggca gcagccactg 15960

gtaacaggat tagcagagcg aggtatgtag gcggtgctac agagttcttg aagtggtggc 16020

ctaactacgg ctacactaga aggacagtat ttggtatctg cgctctgctg aagccagtta 16080

ccttcggaaa aagagttggt agctcttgat ccggcaaaca aaccaccgct ggtagcggtg 16140

gtttttttgt ttgcaagcag cagattacgc gcagaaaaaa aggatctcaa gaagatcctt 16200

tgatcttttc tacggggtct gacgctcagt ggaacgaaaa ctcacgttaa gggattttgg 16260

tcatgagatt atcaaaaagg atcttcacct agatcctttt aaattaaaaa tgaagtttta 16320

aatcaatcta aagtatatat gagtaaactt ggtctgacag ttaccaatgc ttaatcagtg 16380

aggcacctat ctcagcgatc tgtctatttc gttcatccat agttgcctga ctccccgtcg 16440

tgtagataac tacgatacgg gagggcttac catctggccc cagtgctgca atgataccgc 16500

gagacccacg ctcaccggct ccagatttat cagcaataaa ccagccagcc ggaagggccg 16560

agcgcagaag tggtcctgca actttatccg cctccatcca gtctattaat tgttgccggg 16620

aagctagagt aagtagttcg ccagttaata gtttgcgcaa cgttgttgcc attgctacag 16680

gcatcgtggt gtcacgctcg tcgtttggta tggcttcatt cagctccggt tcccaacgat 16740

caaggcgagt tacatgatcc cccatgttgt gcaaaaaagc ggttagctcc ttcggtcctc 16800

cgatcgttgt cagaagtaag ttggccgcag tgttatcact catggttatg gcagcactgc 16860

ataattctct tactgtcatg ccatccgtaa gatgcttttc tgtgactggt gagtactcaa 16920

ccaagtcatt ctgagaatag tgtatgcggc gaccgagttg ctcttgcccg gcgtcaatac 16980

gggataatac cgcgccacat agcagaactt taaaagtgct catcattgga aaacgttctt 17040

cggggcgaaa actctcaagg atcttaccgc tgttgagatc cagttcgatg taacccactc 17100

gtgcacccaa ctgatcttca gcatctttta ctttcaccag cgtttctggg tgagcaaaaa 17160

caggaaggca aaatgccgca aaaaagggaa taagggcgac acggaaatgt tgaatactca 17220

tactcttcct ttttcaatat tattgaagca tttatcaggg ttattgtctc atgagcggat 17280

acatatttga atgtatttag aaaaataaac aaataggggt tccgcgcaca tttccccgaa 17340

aagtgc 17346

<210> 10
<211> 422
<212> PRT
<213> Artificial Sequence

<220>

<223> N protein

<400> 10
Met Ser Val Thr Val Lys Arg Ile Ile Asp Asn Thr Val Val Val Pro
1               5                   10                  15
Lys Leu Pro Ala Asn Glu Asp Pro Val Glu Tyr Pro Ala Asp Tyr Phe
            20                  25                  30
Arg Lys Ser Lys Glu Ile Pro Leu Tyr Ile Asn Thr Thr Lys Ser Leu
        35                  40                  45
Ser Asp Leu Arg Gly Tyr Val Tyr Gln Gly Leu Lys Ser Gly Asn Val
    50                  55                  60
Ser Ile Ile His Val Asn Ser Tyr Leu Tyr Gly Ala Leu Lys Asp Ile
65                  70                  75                  80
Arg Gly Lys Leu Asp Lys Asp Trp Ser Ser Phe Gly Ile Asn Ile Gly
                85                  90                  95
Lys Ala Gly Asp Thr Ile Gly Ile Phe Asp Leu Val Ser Leu Lys Ala
            100                 105                 110
Leu Asp Gly Val Leu Pro Asp Gly Val Ser Asp Ala Ser Arg Thr Ser
        115                 120                 125
Ala Asp Asp Lys Trp Leu Pro Leu Tyr Leu Leu Gly Leu Tyr Arg Val
    130                 135                 140
Gly Arg Thr Gln Met Pro Glu Tyr Arg Lys Lys Leu Met Asp Gly Leu
145                 150                 155                 160
Thr Asn Gln Cys Lys Met Ile Asn Glu Gln Phe Glu Pro Leu Val Pro
                165                 170                 175
Glu Gly Arg Asp Ile Phe Asp Val Trp Gly Asn Asp Ser Asn Tyr Thr
            180                 185                 190
Lys Ile Val Ala Ala Val Asp Met Phe Phe His Met Phe Lys Lys His
        195                 200                 205
Glu Cys Ala Ser Phe Arg Tyr Gly Thr Ile Val Ser Arg Phe Lys Asp
    210                 215                 220
Cys Ala Ala Leu Ala Thr Phe Gly His Leu Cys Lys Ile Thr Gly Met
225                 230                 235                 240
Ser Thr Glu Asp Val Thr Thr Trp Ile Leu Asn Arg Glu Val Ala Asp
                245                 250                 255
Glu Met Val Gln Met Met Leu Pro Gly Gln Glu Ile Asp Lys Ala Asp
            260                 265                 270
Ser Tyr Met Pro Tyr Leu Ile Asp Phe Gly Leu Ser Ser Lys Ser Pro
        275                 280                 285
Tyr Ser Ser Val Lys Asn Pro Ala Phe His Phe Trp Gly Gln Leu Thr
    290                 295                 300
Ala Leu Leu Leu Arg Ser Thr Arg Ala Arg Asn Ala Arg Gln Pro Asp
305                 310                 315                 320
Asp Ile Glu Tyr Thr Ser Leu Thr Thr Ala Gly Leu Leu Tyr Ala Tyr
                325                 330                 335
Ala Val Gly Ser Ser Ala Asp Leu Ala Gln Gln Phe Cys Val Gly Asp
            340                 345                 350
Asn Lys Tyr Thr Pro Asp Asp Ser Thr Gly Gly Leu Thr Thr Asn Ala
        355                 360                 365
Pro Pro Gln Gly Arg Asp Val Val Glu Trp Leu Gly Trp Phe Glu Asp
    370                 375                 380
Gln Asn Arg Lys Pro Thr Pro Asp Met Met Gln Tyr Ala Lys Arg Ala
385                 390                 395                 400
Val Met Ser Leu Gln Gly Leu Arg Glu Lys Thr Ile Gly Lys Tyr Ala
                405                 410                 415
Lys Ser Glu Phe Asp Lys
            420

<210> 11
<211> 265
<212> PRT
<213> Artificial Sequence

<220>
<223> P protein

<400> 11
Met Asp Asn Leu Thr Lys Val Arg Glu Tyr Leu Lys Ser Tyr Ser Arg
1               5                   10                  15
Leu Asp Gln Ala Val Gly Glu Ile Asp Glu Ile Glu Ala Gln Arg Ala
            20                  25                  30
Glu Lys Ser Asn Tyr Glu Leu Phe Gln Glu Asp Gly Val Glu Glu His
        35                  40                  45
Thr Lys Pro Ser Tyr Phe Gln Ala Ala Asp Asp Ser Asp Thr Glu Ser
    50                  55                  60
Glu Pro Glu Ile Glu Asp Asn Gln Gly Leu Tyr Ala Pro Asp Pro Glu
65                  70                  75                  80
Ala Glu Gln Val Glu Gly Phe Ile Gln Gly Pro Leu Asp Asp Tyr Ala
                85                  90                  95
Asp Glu Glu Val Asp Val Val Phe Thr Ser Asp Trp Lys Gln Pro Glu
            100                 105                 110
Leu Glu Ser Asp Glu His Gly Lys Thr Leu Arg Leu Thr Ser Pro Glu
        115                 120                 125
Gly Leu Ser Gly Glu Gln Lys Ser Gln Trp Leu Ser Thr Ile Lys Ala
    130                 135                 140
Val Val Gln Ser Ala Lys Tyr Trp Asn Leu Ala Glu Cys Thr Phe Glu
145                 150                 155                 160
Ala Ser Gly Glu Gly Val Ile Met Lys Glu Arg Gln Ile Thr Pro Asp
                165                 170                 175
Val Tyr Lys Val Thr Pro Val Met Asn Thr His Pro Ser Gln Ser Glu
            180                 185                 190
Ala Val Ser Asp Val Trp Ser Leu Ser Lys Thr Ser Met Thr Phe Gln
        195                 200                 205
Pro Lys Lys Ala Ser Leu Gln Pro Leu Thr Ile Ser Leu Asp Glu Leu
    210                 215                 220
Phe Ser Ser Arg Gly Glu Phe Ile Ser Val Gly Gly Asp Gly Arg Met
225                 230                 235                 240
Ser His Lys Glu Ala Ile Leu Leu Gly Leu Arg Tyr Lys Lys Leu Tyr
                245                 250                 255
Asn Gln Ala Arg Val Lys Tyr Ser Leu
            260                 265

<210> 12
<211> 229
<212> PRT
<213> Artificial Sequence



<220>
<223> M protein

<400> 12
Met Ser Ser Leu Lys Lys Ile Leu Gly Leu Lys Gly Lys Gly Lys Lys
1               5                   10                  15
Ser Lys Lys Leu Gly Ile Ala Pro Pro Pro Tyr Glu Glu Asp Thr Ser
            20                  25                  30
Met Glu Tyr Ala Pro Ser Ala Pro Ile Asp Lys Ser Tyr Phe Gly Val
        35                  40                  45
Asp Glu Met Asp Thr Tyr Asp Pro Asn Gln Leu Arg Tyr Glu Lys Phe
    50                  55                  60
Phe Phe Thr Val Lys Met Thr Val Arg Ser Asn Arg Pro Phe Arg Thr
65                  70                  75                  80
Tyr Ser Asp Val Ala Ala Ala Val Ser His Trp Asp His Met Tyr Ile
                85                  90                  95
Gly Met Ala Gly Lys Arg Pro Phe Tyr Lys Ile Leu Ala Phe Leu Gly

                100                 105                 110
Ser Ser Asn Leu Lys Ala Thr Pro Ala Val Leu Ala Asp Gln Gly Gln
        115                 120                 125
Pro Glu Tyr His Ala His Cys Glu Gly Arg Ala Tyr Leu Pro His Arg
    130                 135                 140
Met Gly Lys Thr Pro Pro Met Leu Asn Val Pro Glu His Phe Arg Arg
145                 150                 155                 160
Pro Phe Asn Ile Gly Leu Tyr Lys Gly Thr Ile Glu Leu Thr Met Thr
                165                 170                 175
Ile Tyr Asp Asp Glu Ser Leu Glu Ala Ala Pro Met Ile Trp Asp His
            180                 185                 190
Phe Asn Ser Ser Lys Phe Ser Asp Phe Arg Glu Lys Ala Leu Met Phe
        195                 200                 205
Gly Leu Ile Val Glu Lys Lys Ala Ser Gly Ala Trp Val Leu Asp Ser
    210                 215                 220
Ile Gly His Phe Lys
225

<210> 13
<211> 399
<212> PRT
<213> Artificial Sequence

<220>
<223> sPD1-Fc protein

<400> 13
Met Gln Ile Pro Gln Ala Pro Trp Pro Val Val Trp Ala Val Leu Gln
1               5                   10                  15
Leu Gly Trp Arg Pro Gly Trp Phe Leu Asp Ser Pro Asp Arg Pro Trp
            20                  25                  30
Asn Pro Pro Thr Phe Ser Pro Ala Leu Leu Val Val Thr Glu Gly Asp
        35                  40                  45
Asn Ala Thr Phe Thr Cys Ser Phe Ser Asn Thr Ser Glu Ser Phe Val
    50                  55                  60
Leu Asn Trp Tyr Arg Met Ser Pro Ser Asn Gln Thr Asp Lys Leu Ala
65                  70                  75                  80
Ala Phe Pro Glu Asp Arg Ser Gln Pro Gly Gln Asp Cys Arg Phe Arg
                85                  90                  95
Val Thr Gln Leu Pro Asn Gly Arg Asp Phe His Met Ser Val Val Arg
            100                 105                 110
Ala Arg Arg Asn Asp Ser Gly Thr Tyr Leu Cys Gly Ala Ile Ser Leu
        115                 120                 125
Ala Pro Lys Leu Gln Ile Lys Glu Ser Leu Arg Ala Glu Leu Arg Val
    130                 135                 140
Thr Glu Arg Arg Ala Glu Val Pro Thr Ala His Pro Ser Pro Ser Pro
145                 150                 155                 160
Arg Pro Ala Gly Gln Phe Gln Thr Leu Val Leu Glu Asp Lys Thr His
                165                 170                 175
Thr Cys Pro Pro Cys Pro Ala Pro Glu Leu Leu Gly Gly Pro Ser Val
            180                 185                 190
Phe Leu Phe Pro Pro Lys Pro Lys Asp Thr Leu Met Ile Ser Arg Thr
        195                 200                 205
Pro Glu Val Thr Cys Val Val Val Asp Val Ser His Glu Asp Pro Glu
    210                 215                 220
Val Lys Phe Asn Trp Tyr Val Asp Gly Val Glu Val His Asn Ala Lys
225                 230                 235                 240
Thr Lys Pro Arg Glu Glu Gln Tyr Asn Ser Thr Tyr Arg Val Val Ser
                245                 250                 255
Val Leu Thr Val Leu His Gln Asp Trp Leu Asn Gly Lys Glu Tyr Lys
            260                 265                 270
Cys Lys Val Ser Asn Lys Ala Leu Pro Ala Pro Ile Glu Lys Thr Ile

275                 280                 285
Ser Lys Ala Lys Gly Gln Pro Arg Glu Pro Gln Val Tyr Thr Leu Pro
    290                 295                 300
Pro Ser Arg Glu Glu Met Thr Lys Asn Gln Val Ser Leu Thr Cys Leu
305                 310                 315                 320
Val Lys Gly Phe Tyr Pro Ser Asp Ile Ala Val Glu Trp Glu Ser Asn
                325                 330                 335
Gly Gln Pro Glu Asn Asn Tyr Lys Thr Thr Pro Pro Val Leu Asp Ser
            340                 345                 350
Asp Gly Ser Phe Phe Leu Tyr Ser Lys Leu Thr Val Asp Lys Ser Arg
        355                 360                 365
Trp Gln Gln Gly Asn Val Phe Ser Cys Ser Val Met His Glu Ala Leu
    370                 375                 380
His Asn His Tyr Thr Gln Lys Ser Leu Ser Leu Ser Pro Gly Lys
385                 390                 395

<210> 14
<211> 2109
<212> PRT
<213> Artificial Sequence

<220>
<223> L protein

<400> 14
Met Glu Val His Asp Phe Glu Thr Asp Glu Phe Asn Asp Phe Asn Glu
1               5                   10                  15
Asp Asp Tyr Ala Thr Arg Glu Phe Leu Asn Pro Asp Glu Arg Met Thr
            20                  25                  30
Tyr Leu Asn His Ala Asp Tyr Asn Leu Asn Ser Pro Leu Ile Ser Asp
        35                  40                  45
Asp Ile Asp Asn Leu Ile Arg Lys Phe Asn Ser Leu Pro Ile Pro Ser
    50                  55                  60
Met Trp Asp Ser Lys Asn Trp Asp Gly Val Leu Glu Met Leu Thr Ser
65                  70                  75                  80
Cys Gln Ala Asn Pro Ile Pro Thr Ser Gln Met His Lys Trp Met Gly
                85                  90                  95
Ser Trp Leu Met Ser Asp Asn His Asp Ala Ser Gln Gly Tyr Ser Phe
            100                 105                 110
Leu His Glu Val Asp Lys Glu Ala Glu Ile Thr Phe Asp Val Val Glu
        115                 120                 125
Thr Phe Ile Arg Gly Trp Gly Asn Lys Pro Ile Glu Tyr Ile Lys Lys
    130                 135                 140
Glu Arg Trp Thr Asp Ser Phe Lys Ile Leu Ala Tyr Leu Cys Gln Lys
145                 150                 155                 160
Phe Leu Asp Leu His Lys Leu Thr Leu Ile Leu Asn Ala Val Ser Glu
                165                 170                 175
Val Glu Leu Leu Asn Leu Ala Arg Thr Phe Lys Gly Lys Val Arg Arg
            180                 185                 190
Ser Ser His Gly Thr Asn Ile Cys Arg Ile Arg Val Pro Ser Leu Gly
        195                 200                 205
Pro Thr Phe Ile Ser Glu Gly Trp Ala Tyr Phe Lys Lys Leu Asp Ile
    210                 215                 220
Leu Met Asp Arg Asn Phe Leu Leu Met Val Lys Asp Val Ile Ile Gly
225                 230                 235                 240
Arg Met Gln Thr Val Leu Ser Met Val Cys Arg Ile Asp Asn Leu Phe
                245                 250                 255
Ser Glu Gln Asp Ile Phe Ser Leu Leu Asn Ile Tyr Arg Ile Gly Asp
            260                 265                 270
Lys Ile Val Glu Arg Gln Gly Asn Phe Ser Tyr Asp Leu Ile Lys Met
        275                 280                 285
Val Glu Pro Ile Cys Asn Leu Lys Leu Met Lys Leu Ala Arg Glu Ser

290                 295                 300
Arg Pro Leu Val Pro Gln Phe Pro His Phe Glu Asn His Ile Lys Thr
305                 310                 315                 320
Ser Val Asp Glu Gly Ala Lys Ile Asp Arg Gly Ile Arg Phe Leu His
                325                 330                 335
Asp Gln Ile Met Ser Val Lys Thr Val Asp Leu Thr Leu Val Ile Tyr
            340                 345                 350
Gly Ser Phe Arg His Trp Gly His Pro Phe Ile Asp Tyr Tyr Thr Gly
        355                 360                 365
Leu Glu Lys Leu His Ser Gln Val Thr Met Lys Lys Asp Ile Asp Val
    370                 375                 380
Ser Tyr Ala Lys Ala Leu Ala Ser Asp Leu Ala Arg Ile Val Leu Phe
385                 390                 395                 400
Gln Gln Phe Asn Asp His Lys Lys Trp Phe Val Asn Gly Asp Leu Leu
                405                 410                 415
Pro His Asp His Pro Phe Lys Ser His Val Lys Glu Asn Thr Trp Pro
            420                 425                 430
Thr Ala Ala Gln Val Gln Asp Phe Gly Asp Lys Trp His Glu Leu Pro
        435                 440                 445
Leu Ile Lys Cys Phe Glu Ile Pro Asp Leu Leu Asp Pro Ser Ile Ile
    450                 455                 460
Tyr Ser Asp Lys Ser His Ser Met Asn Arg Ser Glu Val Leu Lys His
465                 470                 475                 480
Val Arg Met Asn Pro Asn Thr Pro Ile Pro Ser Lys Lys Val Leu Gln
                485                 490                 495
Thr Met Leu Asp Thr Lys Ala Thr Asn Trp Lys Glu Phe Leu Lys Glu
            500                 505                 510
Ile Asp Glu Lys Gly Leu Asp Asp Asp Asp Leu Ile Ile Gly Leu Lys
        515                 520                 525
Gly Lys Glu Arg Glu Leu Lys Leu Ala Gly Arg Phe Phe Ser Leu Met
    530                 535                 540
Ser Trp Lys Leu Arg Glu Tyr Phe Val Ile Thr Glu Tyr Leu Ile Lys
545                 550                 555                 560
Thr His Phe Val Pro Met Phe Lys Gly Leu Thr Met Ala Asp Asp Leu
                565                 570                 575
Thr Ala Val Ile Lys Lys Met Leu Asp Ser Ser Ser Gly Gln Gly Leu
            580                 585                 590
Lys Ser Tyr Glu Ala Ile Cys Ile Ala Asn His Ile Asp Tyr Glu Lys
        595                 600                 605
Trp Asn Asn His Gln Arg Lys Leu Ser Asn Gly Pro Val Phe Arg Val
    610                 615                 620
Met Gly Gln Phe Leu Gly Tyr Pro Ser Leu Ile Glu Arg Thr His Glu
625                 630                 635                 640
Phe Phe Glu Lys Ser Leu Ile Tyr Tyr Asn Gly Arg Pro Asp Leu Met
                645                 650                 655
Arg Val His Asn Asn Thr Leu Ile Asn Ser Thr Ser Gln Arg Val Cys
            660                 665                 670
Trp Gln Gly Gln Glu Gly Gly Leu Glu Gly Leu Arg Gln Lys Gly Trp
        675                 680                 685
Ser Ile Leu Asn Leu Leu Val Ile Gln Arg Glu Ala Lys Ile Arg Asn
    690                 695                 700
Thr Ala Val Lys Val Leu Ala Gln Gly Asp Asn Gln Val Ile Cys Thr
705                 710                 715                 720
Gln Tyr Lys Thr Lys Lys Ser Arg Asn Val Val Glu Leu Gln Gly Ala
                725                 730                 735
Leu Asn Gln Met Val Ser Asn Asn Glu Lys Ile Met Thr Ala Ile Lys
            740                 745                 750
Ile Gly Thr Gly Lys Leu Gly Leu Leu Ile Asn Asp Asp Glu Thr Met
        755                 760                 765
Gln Ser Ala Asp Tyr Leu Asn Tyr Gly Lys Ile Pro Ile Phe Arg Gly
    770                 775                 780
Val Ile Arg Gly Leu Glu Thr Lys Arg Trp Ser Arg Val Thr Cys Val
785                 790                 795                 800

Thr Asn Asp Gln Ile Pro Thr Cys Ala Asn Ile Met Ser Ser Val Ser
                805                 810                 815
Thr Asn Ala Leu Thr Val Ala His Phe Ala Glu Asn Pro Ile Asn Ala
            820                 825                 830
Met Ile Gln Tyr Asn Tyr Phe Gly Thr Phe Ala Arg Leu Leu Leu Met
        835                 840                 845
Met His Asp Pro Ala Leu Arg Gln Ser Leu Tyr Glu Val Gln Asp Lys
    850                 855                 860
Ile Pro Gly Leu His Ser Ser Thr Phe Lys Tyr Ala Met Leu Tyr Leu
865                 870                 875                 880
Asp Pro Ser Ile Gly Gly Val Ser Gly Met Ser Leu Ser Arg Phe Leu
                885                 890                 895
Ile Arg Ala Phe Pro Asp Pro Val Thr Glu Ser Leu Ser Phe Trp Arg
            900                 905                 910
Phe Ile His Val His Ala Arg Ser Glu His Leu Lys Glu Met Ser Ala
        915                 920                 925
Val Phe Gly Asn Pro Glu Ile Ala Lys Phe Arg Ile Thr His Ile Asp
    930                 935                 940
Lys Leu Val Glu Asp Pro Thr Ser Leu Asn Ile Ala Met Gly Met Ser
945                 950                 955                 960
Pro Ala Asn Leu Leu Lys Thr Glu Val Lys Lys Cys Leu Ile Glu Ser
                965                 970                 975
Arg Gln Thr Ile Arg Asn Gln Val Ile Lys Asp Ala Thr Ile Tyr Leu
            980                 985                 990
Tyr His Glu Glu Asp Arg Leu Arg Ser Phe Leu Trp Ser Ile Asn Pro
        995                 1000                1005
Leu Phe Pro Arg Phe Leu Ser Glu Phe Lys Ser Gly Thr Phe Leu Gly
    1010                1015                1020
Val Ala Asp Gly Leu Ile Ser Leu Phe Gln Asn Ser Arg Thr Ile Arg
1025                1030                1035                1040
Asn Ser Phe Lys Lys Lys Tyr His Arg Glu Leu Asp Asp Leu Ile Val
                1045                1050                1055
Arg Ser Glu Val Ser Ser Leu Thr His Leu Gly Lys Leu His Leu Arg
            1060                1065                1070
Arg Gly Ser Cys Lys Met Trp Thr Cys Ser Ala Thr His Ala Asp Thr
        1075                1080                1085
Leu Arg Tyr Lys Ser Trp Gly Arg Thr Val Ile Gly Thr Thr Val Pro
    1090                1095                1100
His Pro Leu Glu Met Leu Gly Pro Gln His Arg Lys Glu Thr Pro Cys
1105                1110                1115                1120
Ala Pro Cys Asn Thr Ser Gly Phe Asn Tyr Val Ser Val His Cys Pro
                1125                1130                1135
Asp Gly Ile His Asp Val Phe Ser Ser Arg Gly Pro Leu Pro Ala Tyr
            1140                1145                1150
Leu Gly Ser Lys Thr Ser Glu Ser Thr Ser Ile Leu Gln Pro Trp Glu
        1155                1160                1165
Arg Glu Ser Lys Val Pro Leu Ile Lys Arg Ala Thr Arg Leu Arg Asp
    1170                1175                1180
Ala Ile Ser Trp Phe Val Glu Pro Asp Ser Lys Leu Ala Met Thr Ile
1185                1190                1195                1200
Leu Ser Asn Ile His Ser Leu Thr Gly Glu Glu Trp Thr Lys Arg Gln
                1205                1210                1215
His Gly Phe Lys Arg Thr Gly Ser Ala Leu His Arg Phe Ser Thr Ser
            1220                1225                1230
Arg Met Ser His Gly Gly Phe Ala Ser Gln Ser Thr Ala Ala Leu Thr
        1235                1240                1245
Arg Leu Met Ala Thr Thr Asp Thr Met Arg Asp Leu Gly Asp Gln Asn
    1250                1255                1260
Phe Asp Phe Leu Phe Gln Ala Thr Leu Leu Tyr Ala Gln Ile Thr Thr
1265                1270                1275                1280
Thr Val Ala Arg Asp Gly Trp Ile Thr Ser Cys Thr Asp His Tyr His
                1285                1290                1295
Ile Ala Cys Lys Ser Cys Leu Arg Pro Ile Glu Glu Ile Thr Leu Asp

1300                1305                1310
Ser Ser Met Asp Tyr Thr Pro Pro Asp Val Ser His Val Leu Lys Thr
        1315                1320                1325
Trp Arg Asn Gly Glu Gly Ser Trp Gly Gln Glu Ile Lys Gln Ile Tyr
    1330                1335                1340
Pro Leu Glu Gly Asn Trp Lys Asn Leu Ala Pro Ala Glu Gln Ser Tyr
1345                1350                1355                1360
Gln Val Gly Arg Cys Ile Gly Phe Leu Tyr Gly Asp Leu Ala Tyr Arg
                1365                1370                1375
Lys Ser Thr His Ala Glu Asp Ser Ser Leu Phe Pro Leu Ser Ile Gln
            1380                1385                1390
Gly Arg Ile Arg Gly Arg Gly Phe Leu Lys Gly Leu Leu Asp Gly Leu
        1395                1400                1405
Met Arg Ala Ser Cys Cys Gln Val Ile His Arg Arg Ser Leu Ala His
    1410                1415                1420
Leu Lys Arg Pro Ala Asn Ala Val Tyr Gly Gly Leu Ile Tyr Leu Ile
1425                1430                1435                1440
Asp Lys Leu Ser Val Ser Pro Pro Phe Leu Ser Leu Thr Arg Ser Gly
                1445                1450                1455
Pro Ile Arg Asp Glu Leu Glu Thr Ile Pro His Lys Ile Pro Thr Ser
            1460                1465                1470
Tyr Pro Thr Ser Asn Arg Asp Met Gly Val Ile Val Arg Asn Tyr Phe
        1475                1480                1485
Lys Tyr Gln Cys Arg Leu Ile Glu Lys Gly Lys Tyr Arg Ser His Tyr
    1490                1495                1500
Ser Gln Leu Trp Leu Phe Ser Asp Val Leu Ser Ile Asp Phe Ile Gly
1505                1510                1515                1520
Pro Phe Ser Ile Ser Thr Thr Leu Leu Gln Ile Leu Tyr Lys Pro Phe
                1525                1530                1535
Leu Ser Gly Lys Asp Lys Asn Glu Leu Arg Glu Leu Ala Asn Leu Ser
            1540                1545                1550
Ser Leu Leu Arg Ser Gly Glu Gly Trp Glu Asp Ile His Val Lys Phe
        1555                1560                1565
Phe Thr Lys Asp Ile Leu Leu Cys Pro Glu Glu Ile Arg His Ala Cys
    1570                1575                1580
Lys Phe Gly Ile Ala Lys Asp Asn Asn Lys Asp Met Ser Tyr Pro Pro
1585                1590                1595                1600
Trp Gly Arg Glu Ser Arg Gly Thr Ile Thr Thr Ile Pro Val Tyr Tyr
                1605                1610                1615
Thr Thr Thr Pro Tyr Pro Lys Met Leu Glu Met Pro Pro Arg Ile Gln
            1620                1625                1630
Asn Pro Leu Leu Ser Gly Ile Arg Leu Gly Gln Leu Pro Thr Gly Ala
        1635                1640                1645
His Tyr Lys Ile Arg Ser Ile Leu His Gly Met Gly Ile His Tyr Arg
    1650                1655                1660
Asp Phe Leu Ser Cys Gly Asp Gly Ser Gly Gly Met Thr Ala Ala Leu
1665                1670                1675                1680
Leu Arg Glu Asn Val His Ser Arg Gly Ile Phe Asn Ser Leu Leu Glu
                1685                1690                1695
Leu Ser Gly Ser Val Met Arg Gly Ala Ser Pro Glu Pro Pro Ser Ala
            1700                1705                1710
Leu Glu Thr Leu Gly Gly Asp Lys Ser Arg Cys Val Asn Gly Glu Thr
        1715                1720                1725
Cys Trp Glu Tyr Pro Ser Asp Leu Cys Asp Pro Arg Thr Trp Asp Tyr
    1730                1735                1740
Phe Leu Arg Leu Lys Ala Gly Leu Gly Leu Gln Ile Asp Leu Ile Val
1745                1750                1755                1760
Met Asp Met Glu Val Arg Asp Ser Ser Thr Ser Leu Lys Ile Glu Thr
                1765                1770                1775
Asn Val Arg Asn Tyr Val His Arg Ile Leu Asp Glu Gln Gly Val Leu
            1780                1785                1790
Ile Tyr Lys Thr Tyr Gly Thr Tyr Ile Cys Glu Ser Glu Lys Asn Ala
        1795                1800                1805

Val Thr Ile Leu Gly Pro Met Phe Lys Thr Val Asp Leu Val Gln Thr
    1810                1815                1820
Glu Phe Ser Ser Ser Gln Thr Ser Glu Val Tyr Met Val Cys Lys Gly
1825                1830                1835                1840
Leu Lys Lys Leu Ile Asp Glu Pro Asn Pro Asp Trp Ser Ser Ile Asn
                1845                1850                1855
Glu Ser Trp Lys Asn Leu Tyr Ala Phe Gln Ser Ser Glu Gln Glu Phe
            1860                1865                1870
Ala Arg Ala Lys Lys Val Ser Thr Tyr Phe Thr Leu Thr Gly Ile Pro
        1875                1880                1885
Ser Gln Phe Ile Pro Asp Pro Phe Val Asn Ile Glu Thr Met Leu Gln
    1890                1895                1900
Ile Phe Gly Val Pro Thr Gly Val Ser His Ala Ala Ala Leu Lys Ser
1905                1910                1915                1920
Ser Asp Arg Pro Ala Asp Leu Leu Thr Ile Ser Leu Phe Tyr Met Ala
                1925                1930                1935
Ile Ile Ser Tyr Tyr Asn Ile Asn His Ile Arg Val Gly Pro Ile Pro
            1940                1945                1950
Pro Asn Pro Pro Ser Asp Gly Ile Ala Gln Asn Val Gly Ile Ala Ile
        1955                1960                1965
Thr Gly Ile Ser Phe Trp Leu Ser Leu Met Glu Lys Asp Ile Pro Leu
    1970                1975                1980
Tyr Gln Gln Cys Leu Ala Val Ile Gln Gln Ser Phe Pro Ile Arg Trp
1985                1990                1995                2000
Glu Ala Val Ser Val Lys Gly Gly Tyr Lys Gln Lys Trp Ser Thr Arg
                2005                2010                2015
Gly Asp Gly Leu Pro Lys Asp Thr Arg Ile Ser Asp Ser Leu Ala Pro
            2020                2025                2030
Ile Gly Asn Trp Ile Arg Ser Leu Glu Leu Val Arg Asn Gln Val Arg
        2035                2040                2045
Leu Asn Pro Phe Asn Glu Ile Leu Phe Asn Gln Leu Cys Arg Thr Val
    2050                2055                2060
Asp Asn His Leu Lys Trp Ser Asn Leu Arg Arg Asn Thr Gly Met Ile
2065                2070                2075                2080
Glu Trp Ile Asn Arg Arg Ile Ser Lys Glu Asp Arg Ser Ile Leu Met
                2085                2090                2095
Leu Lys Ser Asp Leu His Glu Glu Asn Ser Trp Arg Asp
            2100                2105

<210> 15
<211> 14086
<212> DNA
<213> Artificial Sequence

<220>
<223> VSV-NDV-HA-sPD1-Fc (nucleotide sequence)

<400> 15
atgtctgtta cagtcaagag aatcattgac aacacagtcg tagttccaaa acttcctgca      60

aatgaggatc cagtggaata cccggcagat tacttcagaa aatcaaagga gattcctctt     120

tacatcaata ctacaaaaag tttgtcagat ctaagaggat atgtctacca aggcctcaaa     180

tccggaaatg tatcaatcat acatgtcaac agctacttgt atggagcatt aaaggacatc     240

cggggtaagt tggataaaga ttggtcaagt ttcggaataa acatcgggaa agcaggggat     300

acaatcggaa tatttgacct tgtatccttg aaagccctgg acggcgtact tccagatgga     360

gtatcggatg cttccagaac cagcgcagat gacaaatggt tgcctttgta tctacttggc     420

ttatacagag tgggcagaac acaaatgcct gaatacagaa aaaagctcat ggatgggctg 480

acaaatcaat gcaaaatgat caatgaacag tttgaacctc ttgtgccaga aggtcgtgac 540

atttttgatg tgtggggaaa tgacagtaat tacacaaaaa ttgtcgctgc agtggacatg 600

ttcttccaca tgttcaaaaa acatgaatgt gcctcgttca gatacggaac tattgtttcc 660

agattcaaag attgtgctgc attggcaaca tttggacacc tctgcaaaat aaccggaatg 720

tctacagaag atgtaacgac ctggatcttg aaccgagaag ttgcagatga aatggtccaa 780

atgatgcttc caggccaaga aattgacaag gccgattcat acatgcctta tttgatcgac 840

tttggattgt cttctaagtc tccatattct tccgtcaaaa accctgcctt ccacttctgg 900

gggcaattga cagctcttct gctcagatcc accagagcaa ggaatgcccg acagcctgat 960

gacattgagt atacatctct tactacagca ggtttgttgt acgcttatgc agtaggatcc 1020

tctgccgact tggcacaaca gttttgtgtt ggagataaca aatacactcc agatgatagt 1080

accggaggat tgacgactaa tgcaccgcca caaggcagag atgtggtcga atggctcgga 1140

tggtttgaag atcaaaacag aaaaccgact cctgatatga tgcagtatgc gaaaagagca 1200

gtcatgtcac tgcaaggcct aagagagaag acaattggca agtatgctaa gtcagaattt 1260

gacaaatgac cctataattc tcagatcacc tattatatat tatgctacat atgaaaaaaa 1320

ctaacagata tcatggataa tctcacaaaa gttcgtgagt atctcaagtc ctattctcgt 1380

ctggatcagg cggtaggaga gatagatgag atcgaagcac aacgagctga aaagtccaat 1440

tatgagttgt tccaagagga tggagtggaa gagcatacta agccctctta ttttcaggca 1500

gcagatgatt ctgacacaga atctgaacca gaaattgaag acaatcaagg cttgtatgca 1560

ccagatccag aagctgagca agttgaaggc tttatacagg ggcctttaga tgactatgca 1620

gatgaggaag tggatgttgt atttacttcg gactggaaac agcctgagct tgaatctgac 1680

gagcatggaa agaccttacg gttgacatcg ccagagggtt taagtggaga gcagaaatcc 1740

cagtggcttt cgacgattaa agcagtcgtg caaagtgcca aatactggaa tctggcagag 1800

tgcacatttg aagcatcggg agaaggggtc attatgaagg agcgccagat aactccggat 1860

gtatataagg tcactccagt gatgaacaca catccgtccc aatcagaagc agtatcagat 1920

gtttggtctc tctcaaagac atccatgact ttccaaccca agaaagcaag tcttcagcct 1980

ctcaccatat ccttggatga attgttctca tctagaggag agttcatctc tgtcggaggt 2040

gacggacgaa tgtctcataa agaggccatc ctgctcggcc tgagatacaa aaagttgtac 2100

aatcaggcga gagtcaaata ttctctgtag actatgaaaa aaagtaacag atatcacgat 2160

ctaagtgtta tcccaatcca ttcatcatga gttccttaaa gaagattctc ggtctgaagg 2220

ggaaaggtaa gaaatctaag aaattaggga tcgcaccacc cccttatgaa gaggacacta 2280

gcatggagta tgctccgagc gctccaattg acaaatccta ttttggagtt gacgagatgg 2340

acacctatga tccgaatcaa ttaagatatg agaaattctt ctttacagtg aaaatgacgg 2400

ttagatctaa tcgtccgttc agaacatact cagatgtggc agccgctgta tcccattggg 2460

atcacatgta catcggaatg gcagggaaac gtcccttcta caaaatcttg gcttttttgg 2520

gttcttctaa tctaaaggcc actccagcgg tattggcaga tcaaggtcaa ccagagtatc 2580

acgctcactg cgaaggcagg gcttatttgc cacataggat ggggaagacc cctcccatgc 2640

tcaatgtacc agagcacttc agaagaccat tcaatatagg tctttacaag ggaacgattg 2700

agctcacaat gaccatctac gatgatgagt cactggaagc agctcctatg atctgggatc 2760

atttcaattc ttccaaattt tctgatttca gagagaaggc cttaatgttt ggcctgattg 2820

tcgagaaaaa ggcatctgga gcgtgggtcc tggactctat cggccacttc aaatgagcta 2880

gtctaacttc tagcttctga acaatccccg gtttactcag tctcccctaa ttccagcctc 2940

tcgaacaact aatatcctgt cttttctatc cctatgaaaa aaactaacag agatcgatct 3000

gtttacgcgt cgagatgggc tccagacctt ctaccaagaa cccagcacct atgatgctga 3060

ctatccgggt cgcgctggta ctgagttgca tctgcccggc aaactccatt gatggcaggc 3120

ctcttgcagc tgcaggaatt gtggttacag gagacaaagc agtcaacata tacacctcat 3180

cccagacagg atcaatcata gttaagctcc tcccgaatct gcccaaggat aaggaggcat 3240

gtgcgaaagc ccccttggat gcatacaaca ggacattgac cactttgctc accccccttg 3300

gtgactctat ccgtaggata caagagtctg tgactacatc tggagggcgg agacagaggc 3360

gctttatagg cgccattatt ggcggtgtgg ctcttggggt tgcaactgcc gcacaaataa 3420

cagcggccgc agctctgata caagccaaac aaaatgctgc caacatcctc cgacttaaag 3480

agagcattgc cgcaaccaat gaggctgtgc atgaggtcac tgacggatta tcgcaactag 3540

cagtggcagt tgggaagatg cagcagtttg ttaatgacca atttaataaa acagctcagg 3600

aattagactg catcaaaatt gcacagcaag ttggtgtaga gctcaacctg tacctaaccg 3660

aattgactac agtattcgga ccacaaatca cttcacctgc cttaaacaag ctgactattc 3720

aggcacttta caatctagct ggtgggaata tggattactt attgactaag ttaggtatag 3780

ggaacaatca actcagctca ttaatcggta gcggcttaat caccggtaac cctattctat 3840

acgactcaca gactcaactc ttgggtatac aggtaactgc accttcagtc gggaacctaa 3900

ataatatgcg tgccacctac ttggaaacct tatccgtaag cacaaccagg ggatttgcct 3960

cggcacttgt cccaaaagtg gtgacacagg tcggttctgt gatagaagaa cttgacacct 4020

catactgtat agaaactgac ttagatttat attgtacaag aatagtaacg ttccctatgt 4080

cccctggtat ttactcctgc ttgagcggca atacatcggc ctgtatgtac tcaaagaccg 4140

aaggcgcact tactacacca tatatgacta tcaaaggctc agtcatcgct aactgcaaga 4200

tgacaacatg tagatgtgta aaccccccgg gtatcatatc gcaaaactat ggagaagccg 4260

tgtctctaat agataaacaa tcatgcaatg ttttatcctt aggcgggata actttaaggc 4320

tcagtgggga attcgatgta acttatcaga agaatatctc aatacaagat tctcaagtaa 4380

taataacagg caatcttgat atctcaactg agcttgggaa tgtcaacaac tcgatcagta 4440

atgctttgaa taagttagag gaaagcaaca gaaaactaga caaagtcaat gtcaaactga 4500

ccagcacatc tgctctcatt acctatatcg ttttgactat catatctctt gtttttggta 4560

tacttagcct gattctagca tgctacctaa tgtacaagca aaaggcgcaa caaaagacct 4620

tattatggct tgggaataat accctagatc agatgagagc cactacaaaa atgtgagcta 4680

gcttaattaa tatgaaaaaa actaacagat atcatggacc gcgccgttag ccaagttgcg 4740

ttagagaatg atgaaagaga ggcaaaaaat acatggcgct tgatattccg gattgcaatc 4800

ttattcttaa cagtagtgac cttggctata tctgtagcct cccttttata tagcatgggg 4860

gctagcacac ctagcgatct tgtaggcata ccgactagga tttccagggc agaagaaaag 4920

attacatcta cacttggttc caatcaagat gtagtagata ggatatataa gcaagtggcc 4980

cttgagtctc cattggcatt gttaaatact gagaccacaa ttatgaacgc aataacatct 5040

ctctcttatc agattaatgg agctgcaaac aacagcgggt ggggggcacc tattcatgac 5100

ccagattata taggggggat aggcaaagaa ctcattgtag atgatgctag tgatgtcaca 5160

tcattctatc cctctgcatt tcaagaacat ctgaatttta tcccggcgcc tactacagga 5220

tcaggttgca ctcgaatacc ctcatttgac atgagtgcta cccattactg ctacacccat 5280

aatgtaatat tgtctggatg cagagatcac tcacactcac atcagtattt agcacttggt 5340

gtgctccgga catctgcaac agggagggta ttcttttcta ctctgcgttc catcaacctg 5400

gacgacaccc aaaatcggaa gtcttgcagt gtgagtgcaa ctcccctggg ttgtgatatg 5460

ctgtgctcga aagccacgga gacagaggaa gaagattata actcagctgt ccctacgcgg 5520

atggtacatg ggaggttagg gttcgacggc caatatcacg aaaaggacct agatgtcaca 5580

acattattcg gggactgggt ggccaactac ccaggagtag ggggtggatc ttttattgac 5640

agccgcgtat ggttctcagt ctacggaggg ttaaaaccca atacacccag tgacactgta 5700

caggaaggga aatatgtgat atacaagcga tacaatgaca catgcccaga tgagcaagac 5760

taccagattc gaatggccaa gtcttcgtat aagcctggac ggtttggtgg gaaacgcata 5820

cagcaggcta tcttatctat caaagtgtca acatccttag gcgaagaccc ggtactgact 5880

gtaccgccca acacagtcac actcatgggg gccgaaggca gaattctcac agtagggaca 5940

tcccatttct tgtatcagcg agggtcatca tacttctctc ccgcgttatt atatcctatg 6000

acagtcagca acaaaacagc cactcttcat agtccttata cattcaatgc cttcactcgg 6060

ccaggtagta tcccttgcca ggcttcagca agatgcccca actcgtgtgt tactggagtc 6120

tatacagatc catatcccct aatcttctat agaaaccaca ccttgcgagg ggtattcggg 6180

acaatgcttg atggtgaaca agcaagactt aaccctgcgt ctgcagtatt cgatagcaca 6240

tcccgcagtc gcataactcg agtgagttca agcagcatca aagcagcata cacaacatca 6300

acttgtttta aagtggtcaa gaccaataag acctattgtc tcagcattgc tgaaatatct 6360

aatactctct tcggagaatt cagaatcgtc ccgttactag ttgagatcct caaagatgac 6420

ggggttagag aagccaggtc tggctaggtt taaacagcta ggcgcgccta tgaaaaaaac 6480

taacagcaat catgcagatc ccacaggcgc cctggccagt cgtctgggcg gtgctacaac 6540

tgggctggcg gccaggatgg ttcttagact ccccagacag gccctggaac ccccccacct 6600

tctccccagc cctgctcgtg gtgaccgaag gggacaacgc caccttcacc tgcagcttct 6660

ccaacacatc ggagagcttc gtgctaaact ggtaccgcat gagccccagc aaccagacgg 6720

acaagctggc cgccttcccc gaggaccgca gccagcccgg ccaggactgc cgcttccgtg 6780

tcacacaact gcccaacggg cgtgacttcc acatgagcgt ggtcagggcc cggcgcaatg 6840

acagcggcac ctacctctgt ggggccatct ccctggcccc caagctgcag atcaaagaga 6900

gcctgcgggc agagctcagg gtgacagaga gaagggcaga agtgcccaca gcccacccca 6960

gcccctcacc caggccagcc ggccagttcc aaaccctggt gctcgaggac aaaactcaca 7020

catgcccacc gtgcccagca cctgaactcc tggggggacc gtcagtcttc ctcttccccc 7080

caaaacccaa ggacaccctc atgatctccc ggacccctga ggtcacatgc gtggtggtgg 7140

acgtgagcca cgaagaccct gaggtcaagt tcaactggta cgtggacggc gtggaggtgc 7200

ataatgccaa gacaaagccg cgggaggagc agtacaacag cacgtaccgt gtggtcagcg 7260

tcctcaccgt cctgcaccag gactggctga atggcaagga gtacaagtgc aaggtctcca 7320

acaaagccct cccagccccc atcgagaaaa ccatctccaa agccaaaggg cagccccgag 7380

aaccacaggt gtacaccctg cccccatccc gggaggagat gaccaagaac caggtcagcc 7440

tgacctgcct ggtcaaaggc ttctatccca gcgacatcgc cgtggagtgg gagagcaatg 7500

ggcagccgga gaacaactac aagaccacgc ctcccgtgct ggactccgac ggctccttct 7560

tcctctacag caagctcacc gtggacaaga gcaggtggca gcaggggaac gtcttctcat 7620

gctccgtgat gcacgaggct ctgcacaacc actacacgca gaagagcctc tccctgtctc 7680

cgggtaaatg acctagggct agcggcctca attatatttg agtttttaat ttttatgaaa 7740

aaaactaaca gcaatcatgg aagtccacga ttttgagacc gacgagttca atgatttcaa 7800

tgaagatgac tatgccacaa gagaattcct gaatcccgat gagcgcatga cgtacttgaa 7860

tcatgctgat tacaacctga attctcctct aattagtgat gatattgaca atttaatcag 7920

gaaattcaat tctcttccaa ttccctcgat gtgggatagt aagaactggg atggagttct 7980

tgagatgtta acgtcatgtc aagccaatcc catcccaaca tctcagatgc ataaatggat 8040

gggaagttgg ttaatgtctg ataatcatga tgccagtcaa gggtatagtt ttttacatga 8100

agtggacaaa gaggcagaaa taacatttga cgtggtggag accttcatcc gcggctgggg 8160

caacaaacca attgaataca tcaaaaagga aagatggact gactcattca aaattctcgc 8220

ttatttgtgt caaaagtttt tggacttaca caagttgaca ttaatcttaa atgctgtctc 8280

tgaggtggaa ttgctcaact tggcgaggac tttcaaaggc aaagtcagaa gaagttctca 8340

tggaacgaac atatgcagga ttagggttcc cagcttgggt cctactttta tttcagaagg 8400

atgggcttac ttcaagaaac ttgatattct aatggaccga aactttctgt taatggtcaa 8460

agatgtgatt atagggagga tgcaaacggt gctatccatg gtatgtagaa tagacaacct 8520

gttctcagag caagacatct tctcccttct aaatatctac agaattggag ataaaattgt 8580

ggagaggcag ggaaattttt cttatgactt gattaaaatg gtggaaccga tatgcaactt 8640

gaagctgatg aaattagcaa gagaatcaag gcctttagtc ccacaattcc ctcattttga 8700

aaatcatatc aagacttctg ttgatgaagg ggcaaaaatt gaccgaggta taagattcct 8760

ccatgatcag ataatgagtg tgaaaacagt ggatctcaca ctggtgattt atggatcgtt 8820

cagacattgg ggtcatcctt ttatagatta ttacactgga ctagaaaaat tacattccca 8880

agtaaccatg aagaaagata ttgatgtgtc atatgcaaaa gcacttgcaa gtgatttagc 8940

tcggattgtt ctatttcaac agttcaatga tcataaaaag tggttcgtga atggagactt 9000

gctccctcat gatcatccct ttaaaagtca tgttaaagaa aatacatggc ccacagctgc 9060

tcaagttcaa gattttggag ataaatggca tgaacttccg ctgattaaat gttttgaaat 9120

acccgactta ctagacccat cgataatata ctctgacaaa agtcattcaa tgaataggtc 9180

agaggtgttg aaacatgtcc gaatgaatcc gaacactcct atccctagta aaaaggtgtt 9240

gcagactatg ttggacacaa aggctaccaa ttggaaagaa tttcttaaag agattgatga 9300

gaagggctta gatgatgatg atctaattat tggtcttaaa ggaaaggaga gggaactgaa 9360

gttggcaggt agatttttct ccctaatgtc ttggaaattg cgagaatact ttgtaattac 9420

cgaatatttg ataaagactc atttcgtccc tatgtttaaa ggcctgacaa tggcggacga 9480

tctaactgca gtcattaaaa agatgttaga ttcctcatcc ggccaaggat tgaagtcata 9540

tgaggcaatt tgcatagcca atcacattga ttacgaaaaa tggaataacc accaaaggaa 9600

gttatcaaac ggcccagtgt tccgagttat gggccagttc ttaggttatc catccttaat 9660

cgagagaact catgaatttt ttgagaaaag tcttatatac tacaatggaa gaccagactt 9720

gatgcgtgtt cacaacaaca cactgatcaa ttcaacctcc caacgagttt gttggcaagg 9780

acaagagggt ggactggaag gtctacggca aaaaggatgg agtatcctca atctactggt 9840

tattcaaaga gaggctaaaa tcagaaacac tgctgtcaaa gtcttggcac aaggtgataa 9900

tcaagttatt tgcacacagt ataaaacgaa gaaatcgaga aacgttgtag aattacaggg 9960

tgctctcaat caaatggttt ctaataatga gaaaattatg actgcaatca aaatagggac 10020

agggaagtta ggacttttga taaatgacga tgagactatg caatctgcag attacttgaa 10080

ttatggaaaa ataccgattt tccgtggagt gattagaggg ttagagacca agagatggtc 10140

acgagtgact tgtgtcacca atgaccaaat acccacttgt gctaatataa tgagctcagt 10200

ttccacaaat gctctcaccg tagctcattt tgctgagaac ccaatcaatg ccatgataca 10260

gtacaattat tttgggacat ttgctagact cttgttgatg atgcatgatc ctgctcttcg 10320

tcaatcattg tatgaagttc aagataagat accgggcttg cacagttcta ctttcaaata 10380

cgccatgttg tatttggacc cttccattgg aggagtgtcg ggcatgtctt tgtccaggtt 10440

tttgattaga gccttcccag atcccgtaac agaaagtctc tcattctgga gattcatcca 10500

tgtacatgct cgaagtgagc atctgaagga gatgagtgca gtatttggaa accccgagat 10560

agccaagttt cgaataactc acatagacaa gctagtagaa gatccaacct ctctgaacat 10620

cgctatggga atgagtccag cgaacttgtt aaagactgag gttaaaaaat gcttaatcga 10680

atcaagacaa accatcagga accaggtgat taaggatgca accatatatt tgtatcatga 10740

agaggatcgg ctcagaagtt tcttatggtc aataaatcct ctgttcccta gatttttaag 10800

tgaattcaaa tcaggcactt ttttgggagt cgcagacggg ctcatcagtc tatttcaaaa 10860

ttctcgtact attcggaact cctttaagaa aaagtatcat agggaattgg atgatttgat 10920

tgtgaggagt gaggtatcct ctttgacaca tttagggaaa cttcatttga gaaggggatc 10980

atgtaaaatg tggacatgtt cagctactca tgctgacaca ttaagataca aatcctgggg 11040

ccgtacagtt attgggacaa ctgtacccca tccattagaa atgttgggtc cacaacatcg 11100

aaaagagact ccttgtgcac catgtaacac atcagggttc aattatgttt ctgtgcattg 11160

tccagacggg atccatgacg tctttagttc acggggacca ttgcctgctt atctagggtc 11220

taaaacatct gaatctacat ctattttgca gccttgggaa agggaaagca aagtcccact 11280

gattaaaaga gctacacgtc ttagagatgc tatctcttgg tttgttgaac ccgactctaa 11340

actagcaatg actatacttt ctaacatcca ctctttaaca ggcgaagaat ggaccaaaag 11400

gcagcatggg ttcaaaagaa cagggtctgc ccttcatagg ttttcgacat ctcggatgag 11460

ccatggtggg ttcgcatctc agagcactgc agcattgacc aggttgatgg caactacaga 11520

caccatgagg gatctgggag atcagaattt cgacttttta ttccaagcaa cgttgctcta 11580

tgctcaaatt accaccactg ttgcaagaga cggatggatc accagttgta cagatcatta 11640

tcatattgcc tgtaagtcct gtttgagacc catagaagag atcaccctgg actcaagtat 11700

ggactacacg cccccagatg tatcccatgt gctgaagaca tggaggaatg gggaaggttc 11760

gtggggacaa gagataaaac agatctatcc tttagaaggg aattggaaga atttagcacc 11820

tgctgagcaa tcctatcaag tcggcagatg tataggtttt ctatatggag acttggcgta 11880

tagaaaatct actcatgccg aggacagttc tctatttcct ctatctatac aaggtcgtat 11940

tagaggtcga ggtttcttaa aagggttgct agacggatta atgagagcaa gttgctgcca 12000

agtaatacac cggagaagtc tggctcattt gaagaggccg gccaacgcag tgtacggagg 12060

tttgatttac ttgattgata aattgagtgt atcacctcca ttcctttctc ttactagatc 12120

aggacctatt agagacgaat tagaaacgat tccccacaag atcccaacct cctatccgac 12180

aagcaaccgt gatatggggg tgattgtcag aaattacttc aaataccaat gccgtctaat 12240

tgaaaaggga aaatacagat cacattattc acaattatgg ttattctcag atgtcttatc 12300

catagacttc attggaccat tctctatttc caccaccctc ttgcaaatcc tatacaagcc 12360

atttttatct gggaaagata agaatgagtt gagagagctg gcaaatcttt cttcattgct 12420

aagatcagga gaggggtggg aagacataca tgtgaaattc ttcaccaagg acatattatt 12480

gtgtccagag gaaatcagac atgcttgcaa gttcgggatt gctaaggata ataataaaga 12540

catgagctat cccccttggg gaagggaatc cagagggaca attacaacaa tccctgttta 12600

ttatacgacc accccttacc caaagatgct agagatgcct ccaagaatcc aaaatcccct 12660

gctgtccgga atcaggttgg gccaattacc aactggcgct cattataaaa ttcggagtat 12720

attacatgga atgggaatcc attacaggga cttcttgagt tgtggagacg gctccggagg 12780

gatgactgct gcattactac gagaaaatgt gcatagcaga ggaatattca atagtctgtt 12840

agaattatca gggtcagtca tgcgaggcgc ctctcctgag ccccccagtg ccctagaaac 12900

tttaggagga gataaatcga gatgtgtaaa tggtgaaaca tgttgggaat atccatctga 12960

cttatgtgac ccaaggactt gggactattt cctccgactc aaagcaggct tggggcttca 13020

aattgattta attgtaatgg atatggaagt tcgggattct tctactagcc tgaaaattga 13080

gacgaatgtt agaaattatg tgcaccggat tttggatgag caaggagttt taatctacaa 13140

gacttatgga acatatattt gtgagagcga aaagaatgca gtaacaatcc ttggtcccat 13200

gttcaagacg gtcgacttag ttcaaacaga atttagtagt tctcaaacgt ctgaagtata 13260

tatggtatgt aaaggtttga agaaattaat cgatgaaccc aatcccgatt ggtcttccat 13320

caatgaatcc tggaaaaacc tgtacgcatt ccagtcatca gaacaggaat ttgccagagc 13380

aaagaaggtt agtacatact ttaccttgac aggtattccc tcccaattca ttcctgatcc 13440

ttttgtaaac attgagacta tgctacaaat attcggagta cccacgggtg tgtctcatgc 13500

ggctgcctta aaatcatctg atagacctgc agatttattg accattagcc ttttttatat 13560

ggcgattata tcgtattata acatcaatca tatcagagta ggaccgatac ctccgaaccc 13620

cccatcagat ggaattgcac aaaatgtggg gatcgctata actggtataa gcttttggct 13680

gagtttgatg gagaaagaca ttccactata tcaacagtgt ttagcagtta tccagcaatc 13740

attcccgatt aggtgggagg ctgtttcagt aaaaggagga tacaagcaga agtggagtac 13800

tagaggtgat gggctcccaa aagatacccg aatttcagac tccttggccc caatcgggaa 13860

ctggatcaga tctctggaat tggtccgaaa ccaagttcgt ctaaatccat tcaatgagat 13920

cttgttcaat cagctatgtc gtacagtgga taatcatttg aaatggtcaa atttgcgaag 13980

aaacacagga atgattgaat ggatcaatag acgaatttca aaagaagacc ggtctatact 14040

gatgttgaag agtgacctac acgaggaaaa ctcttggaga gattaa 14086

<210> 16
<211> 16659
<212> DNA
<213> Artificial Sequence

<220>
<223> Total nucleic acid sequence of rVSV-NDV-sPD1

<400> 16
cacctaaatt gtaagcgtta atattttgtt aaaattcgcg ttaaattttt gttaaatcag 60

ctcatttttt aaccaatagg ccgaaatcgg caaaatccct tataaatcaa aagaatagac 120

cgagataggg ttgagtgttg ttccagtttg gaacaagagt ccactattaa agaacgtgga 180

ctccaacgtc aaagggcgaa aaaccgtcta tcagggcgat ggcccactac gtgaaccatc 240

accctaatca agttttttgg ggtcgaggtg ccgtaaagca ctaaatcgga accctaaagg 300

gagcccccga tttagagctt gacggggaaa gccggcgaac gtggcgagaa aggaagggaa 360

gaaagcgaaa ggagcgggcg ctagggcgct ggcaagtgta gcggtcacgc tgcgcgtaac 420

caccacaccc gccgcgctta atgcgccgct acagggcgcg tcccattcgc cattcaggct 480

gcgcaactgt tgggaagggc gatcggtgcg ggcctcttcg ctattacgcc agctggcgaa 540

agggggatgt gctgcaaggc gattaagttg ggtaacgcca gggttttccc agtcacgacg 600

ttgtaaaacg acggccagtg aattgtaata cgactcacta taggacgaag acaaacaaac 660

cattattatc attaaaaggc tcaggagaaa ctttaacagt aatcaaaatg tctgttacag 720

tcaagagaat cattgacaac acagtcgtag ttccaaaact tcctgcaaat gaggatccag 780

tggaataccc ggcagattac ttcagaaaat caaaggagat tcctctttac atcaatacta 840

caaaaagttt gtcagatcta agaggatatg tctaccaagg cctcaaatcc ggaaatgtat 900

caatcataca tgtcaacagc tacttgtatg gagcattaaa ggacatccgg ggtaagttgg 960

ataaagattg gtcaagtttc ggaataaaca tcgggaaagc aggggataca atcggaatat 1020

ttgaccttgt atccttgaaa gccctggacg gcgtacttcc agatggagta tcggatgctt 1080

ccagaaccag cgcagatgac aaatggttgc ctttgtatct acttggctta tacagagtgg 1140

gcagaacaca aatgcctgaa tacagaaaaa agctcatgga tgggctgaca aatcaatgca 1200
aaatgatcaa tgaacagttt gaacctcttg tgccagaagg tcgtgacatt tttgatgtgt 1260
ggggaaatga cagtaattac acaaaaattg tcgctgcagt ggacatgttc ttccacatgt 1320
tcaaaaaaca tgaatgtgcc tcgttcagat acggaactat tgtttccaga ttcaaagatt 1380
gtgctgcatt ggcaacattt ggacacctct gcaaaataac cggaatgtct acagaagatg 1440
taacgacctg gatcttgaac cgagaagttg cagatgaaat ggtccaaatg atgcttccag 1500
gccaagaaat tgacaaggcc gattcataca tgccttattt gatcgacttt ggattgtctt 1560
ctaagtctcc atattcttcc gtcaaaaacc ctgccttcca cttctggggg caattgacag 1620
ctcttctgct cagatccacc agagcaagga atgcccgaca gcctgatgac attgagtata 1680
catctcttac tacagcaggt ttgttgtacg cttatgcagt aggatcctct gccgacttgg 1740
cacaacagtt ttgtgttgga gataacaaat acactccaga tgatagtacc ggaggattga 1800
cgactaatgc accgccacaa ggcagagatg tggtcgaatg gctcggatgg tttgaagatc 1860
aaaacagaaa accgactcct gatatgatgc agtatgcgaa aagagcagtc atgtcactgc 1920
aaggcctaag agagaagaca attggcaagt atgctaagtc agaatttgac aaatgaccct 1980
ataattctca gatcacctat tatatattat gctacatatg aaaaaaacta acagatatca 2040
tggataatct cacaaaagtt cgtgagtatc tcaagtccta ttctcgtctg gatcaggcgg 2100
taggagagat agatgagatc gaagcacaac gagctgaaaa gtccaattat gagttgttcc 2160
aagaggatgg agtggaagag catactaagc cctcttattt tcaggcagca gatgattctg 2220
acacagaatc tgaaccagaa attgaagaca atcaaggctt gtatgcacca gatccagaag 2280
ctgagcaagt tgaaggcttt atacaggggc ctttagatga ctatgcagat gaggaagtgg 2340
atgttgtatt tacttcggac tggaaacagc ctgagcttga atctgacgag catggaaaga 2400
ccttacggtt gacatcgcca gagggtttaa gtggagagca gaaatcccag tggctttcga 2460
cgattaaagc agtcgtgcaa agtgccaaat actggaatct ggcagagtgc acatttgaag 2520
catcgggaga aggggtcatt atgaaggagc gccagataac tccggatgta tataaggtca 2580
ctccagtgat gaacacacat ccgtcccaat cagaagcagt atcagatgtt tggtctctct 2640
caaagacatc catgactttc caacccaaga aagcaagtct tcagcctctc accatatcct 2700
tggatgaatt gttctcatct agaggagagt tcatctctgt cggaggtgac ggacgaatgt 2760
ctcataaaga ggccatcctg ctcggcctga gatacaaaaa gttgtacaat caggcgagag 2820
tcaaatattc tctgtagact atgaaaaaaa gtaacagata tcacgatcta agtgttatcc 2880
caatccattc atcatgagtt ccttaaagaa gattctcggt ctgaagggga aaggtaagaa 2940
atctaagaaa ttagggatcg caccaccccc ttatgaagag gacactagca tggagtatgc 3000

tccgagcgct ccaattgaca aatcctattt tggagttgac gagatggaca cctatgatcc 3060
gaatcaatta agatatgaga aattcttctt tacagtgaaa atgacggtta gatctaatcg 3120
tccgttcaga acatactcag atgtggcagc cgctgtatcc cattgggatc acatgtacat 3180
cggaatggca gggaaacgtc ccttctacaa aatcttggct tttttgggtt cttctaatct 3240
aaaggccact ccagcggtat tggcagatca aggtcaacca gagtatcacg ctcactgcga 3300
aggcagggct tatttgccac ataggatggg gaagacccct cccatgctca atgtaccaga 3360
gcacttcaga agaccattca atataggtct ttacaaggga acgattgagc tcacaatgac 3420
catctacgat gatgagtcac tggaagcagc tcctatgatc tgggatcatt tcaattcttc 3480
caaattttct gatttcagag agaaggcctt aatgtttggc ctgattgtcg agaaaaaggc 3540
atctggagcg tgggtcctgg actctatcgg ccacttcaaa tgagctagtc taacttctag 3600
cttctgaaca atccccggtt tactcagtct cccctaattc cagcctctcg aacaactaat 3660
atcctgtctt ttctatccct atgaaaaaaa ctaacagaga tcgatctgtt tacgcgtcga 3720
gatgggctcc agaccttcta ccaagaaccc agcacctatg atgctgacta tccgggtcgc 3780
gctggtactg agttgcatct gcccggcaaa ctccattgat ggcaggcctc ttgcagctgc 3840
aggaattgtg gttacaggag acaaagcagt caacatatac acctcatccc agacaggatc 3900
aatcatagtt aagctcctcc cgaatctgcc caaggataag gaggcatgtg cgaaagcccc 3960
cttggatgca tacaacagga cattgaccac tttgctcacc ccccttggtg actctatccg 4020
taggatacaa gagtctgtga ctacatctgg agggcggaga cagaggcgct ttataggcgc 4080
cattattggc ggtgtggctc ttggggttgc aactgccgca caaataacag cggccgcagc 4140
tctgatacaa gccaaacaaa atgctgccaa catcctccga cttaaagaga gcattgccgc 4200
aaccaatgag gctgtgcatg aggtcactga cggattatcg caactagcag tggcagttgg 4260
gaagatgcag cagtttgtta atgaccaatt taataaaaca gctcaggaat tagactgcat 4320
caaaattgca cagcaagttg gtgtagagct caacctgtac ctaaccgaat tgactacagt 4380
attcggacca caaatcactt cacctgcctt aaacaagctg actattcagg cactttacaa 4440
tctagctggt gggaatatgg attacttatt gactaagtta ggtataggga acaatcaact 4500
cagctcatta atcggtagcg gcttaatcac cggtaaccct attctatacg actcacagac 4560
tcaactcttg ggtatacagg taactgcacc ttcagtcggg aacctaaata atatgcgtgc 4620
cacctacttg gaaaccttat ccgtaagcac aaccagggga tttgcctcgg cacttgtccc 4680
aaaagtggtg acacaggtcg gttctgtgat agaagaactt gacacctcat actgtataga 4740
aactgactta gatttatatt gtacaagaat agtaacgttc cctatgtccc ctggtattta 4800
ctcctgcttg agcggcaata catcggcctg tatgtactca aagaccgaag gcgcacttac 4860
tacaccatat atgactatca aaggctcagt catcgctaac tgcaagatga caacatgtag 4920

atgtgtaaac cccccgggta tcatatcgca aaactatgga gaagccgtgt ctctaataga 4980

taaacaatca tgcaatgttt tatccttagg cgggataact ttaaggctca gtggggaatt 5040

cgatgtaact tatcagaaga atatctcaat acaagattct caagtaataa taacaggcaa 5100

tcttgatatc tcaactgagc ttgggaatgt caacaactcg atcagtaatg ctttgaataa 5160

gttagaggaa agcaacagaa aactagacaa agtcaatgtc aaactgacca gcacatctgc 5220

tctcattacc tatatcgttt tgactatcat atctcttgtt tttggtatac ttagcctgat 5280

tctagcatgc tacctaatgt acaagcaaaa ggcgcaacaa aagaccttat tatggcttgg 5340

gaataatacc ctagatcaga tgagagccac tacaaaaatg tgagctagct taattaatat 5400

gaaaaaaact aacagatatc atggaccgcg ccgttagcca agttgcgtta gagaatgatg 5460

aaagagaggc aaaaaataca tggcgcttga tattccggat tgcaatctta ttcttaacag 5520

tagtgacctt ggctatatct gtagcctccc ttttatatag catgggggct agcacaccta 5580

gcgatcttgt aggcataccg actaggattt ccagggcaga agaaaagatt acatctacac 5640

ttggttccaa tcaagatgta gtagatagga tatataagca agtggccctt gagtctccat 5700

tggcattgtt aaatactgag accacaatta tgaacgcaat aacatctctc tcttatcaga 5760

ttaatggagc tgcaaacaac agcgggtggg ggcacctatt catgacccca gattatatag 5820

gggggatagg caaagaactc attgtagatg atgctagtga tgtcacatca ttctatccct 5880

ctgcatttca agaacatctg aattttatcc cggcgcctac tacaggatca ggttgcactc 5940

gaataccctc atttgacatg agtgctaccc attactgcta cacccataat gtaatattgt 6000

ctggatgcag agatcactca cactcacatc agtatttagc acttggtgtg ctccggacat 6060

ctgcaacagg gagggtattc ttttctactc tgcgttccat caacctggac gacacccaaa 6120

atcggaagtc ttgcagtgtg agtgcaactc ccctgggttg tgatatgctg tgctcgaaag 6180

ccacggagac agaggaagaa gattataact cagctgtccc tacgcggatg gtacatggga 6240

ggttagggtt cgacggccaa tatcacgaaa aggacctaga tgtcacaaca ttattcgggg 6300

actgggtggc caactaccca ggagtagggg gtggatcttt tattgacagc cgcgtatggt 6360

tctcagtcta cggagggtta aaacccaata cacccagtga cactgtacag gaagggaaat 6420

atgtgatata caagcgatac aatgacacat gcccagatga gcaagactac cagattcgaa 6480

tggccaagtc ttcgtataag cctggacggt ttggtgggaa acgcatacag caggctatct 6540

tatctatcaa agtgtcaaca tccttaggcg aagacccggt actgactgta ccgcccaaca 6600

cagtcacact catgggggcc gaaggcagaa ttctcacagt agggacatcc catttcttgt 6660

atcagcgagg gtcatcatac ttctctcccg cgttattata tcctatgaca gtcagcaaca 6720

aaacagccac tcttcatagt ccttatacat tcaatgcctt cactcggcca ggtagtatcc 6780

cttgccaggc ttcagcaaga tgccccaact cgtgtgttac tggagtctat acagatccat 6840
atcccctaat cttctataga aaccacacct tgcgaggggt attcgggaca atgcttgatg 6900
gtgaacaagc aagacttaac cctgcgtctg cagtattcga tagcacatcc cgcagtcgca 6960
taactcgagt gagttcaagc agcatcaaag cagcatacac aacatcaact tgttttaaag 7020
tggtcaagac caataagacc tattgtctca gcattgctga aatatctaat actctcttcg 7080
gagaattcag aatcgtcccg ttactagttg agatcctcaa agatgacggg gttagagaag 7140
ccaggtctgg ctaggtttaa acagctaggc gcgcctatga aaaaaactaa cagcaatcat 7200
gcagatccca caggcgccct ggccagtcgt ctgggcggtg ctacaactgg gctggcggcc 7260
aggatggttc ttagactccc cagacaggcc ctggaacccc cccaccttct ccccagccct 7320
gctcgtggtg accgaagggg acaacgccac cttcacctgc agcttctcca acacatcgga 7380
gagcttcgtg ctaaactggt accgcatgag ccccagcaac cagacggaca agctggccgc 7440
cttccccgag gaccgcagcc agcccggcca ggactgccgc ttccgtgtca cacaactgcc 7500
caacgggcgt gacttccaca tgagcgtggt caggcccggg cgcaatgaca gcggcaccta 7560
cctctgtggg gccatctccc tggcccccaa ggcgcagatc aaagagagcc tgcgggcaga 7620
gctcagggtg acagagagaa gggcagaagt gcccacagcc caccccagcc cctcacccag 7680
gccagccggc cagttccaaa ccctggtgta acctagggct agcggcctca attatatttg 7740
agtttttaat ttttatgaaa aaaactaaca gcaatcatgg aagtccacga ttttgagacc 7800
gacgagttca atgatttcaa tgaagatgac tatgccacaa gagaattcct gaatcccgat 7860
gagcgcatga cgtacttgaa tcatgctgat tacaacctga attctcctct aattagtgat 7920
gatattgaca atttaatcag gaaattcaat tctcttccaa ttccctcgat gtgggatagt 7980
aagaactggg atggagttct tgagatgtta acgtcatgtc aagccaatcc catcccaaca 8040
tctcagatgc ataaatggat gggaagttgg ttaatgtctg ataatcatga tgccagtcaa 8100
gggtatagtt ttttacatga agtggacaaa gaggcagaaa taacatttga cgtggtggag 8160
accttcatcc gcggctgggg caacaaacca attgaataca tcaaaaagga aagatggact 8220
gactcattca aaattctcgc ttatttgtgt caaaagtttt tggacttaca caagttgaca 8280
ttaatcttaa atgctgtctc tgaggtggaa ttgctcaact tggcgaggac tttcaaaggc 8340
aaagtcagaa gaagttctca tggaacgaac atatgcagga ttagggttcc cagcttgggt 8400
cctactttta tttcagaagg atgggcttac ttcaagaaac ttgatattct aatggaccga 8460
aactttctgt taatggtcaa agatgtgatt atagggagga tgcaaacggt gctatccatg 8520
gtatgtagaa tagacaacct gttctcagag caagacatct tctcccttct aaatatctac 8580
agaattggag ataaaattgt ggagaggcag ggaaattttt cttatgactt gattaaaatg 8640
gtggaaccga tatgcaactt gaagctgatg aaattagcaa gagaatcaag gcctttagtc 8700

ccacaattcc ctcattttga aaatcatatc aagacttctg ttgatgaagg ggcaaaaatt 8760

gaccgaggta taagattcct ccatgatcag ataatgagtg tgaaaacagt ggatctcaca 8820

ctggtgattt atggatcgtt cagacattgg ggtcatcctt ttatagatta ttacactgga 8880

ctagaaaaat tacattccca agtaaccatg aagaaagata ttgatgtgtc atatgcaaaa 8940

gcacttgcaa gtgatttagc tcggattgtt ctatttcaac agttcaatga tcataaaaag 9000

tggttcgtga atggagactt gctccctcat gatcatccct ttaaaagtca tgttaaagaa 9060

aatacatggc ccacagctgc tcaagttcaa gattttggag ataaatggca tgaacttccg 9120

ctgattaaat gttttgaaat acccgactta ctagacccat cgataatata ctctgacaaa 9180

agtcattcaa tgaataggtc agaggtgttg aaacatgtcc gaatgaatcc gaacactcct 9240

atccctagta aaaaggtgtt gcagactatg ttggacacaa aggctaccaa ttggaaagaa 9300

tttcttaaag agattgatga gaagggctta gatgatgatg atctaattat tggtcttaaa 9360

ggaaaggaga gggaactgaa gttggcaggt agatttttct ccctaatgtc ttggaaattg 9420

cgagaatact ttgtaattac cgaatatttg ataaagactc atttcgtccc tatgtttaaa 9480

ggcctgacaa tggcggacga tctaactgca gtcattaaaa agatgttaga ttcctcatcc 9540

ggccaaggat tgaagtcata tgaggcaatt tgcatagcca atcacattga ttacgaaaaa 9600

tggaataacc accaaaggaa gttatcaaac ggcccagtgt tccgagttat gggccagttc 9660

ttaggttatc catccttaat cgagagaact catgaatttt ttgagaaaag tcttatatac 9720

tacaatggaa gaccagactt gatgcgtgtt cacaacaaca cactgatcaa ttcaacctcc 9780

caacgagttt gttggcaagg acaagagggt ggactggaag gtctacggca aaaaggatgg 9840

agtatcctca atctactggt tattcaaaga gaggctaaaa tcagaaacac tgctgtcaaa 9900

gtcttggcac aaggtgataa tcaagttatt tgcacacagt ataaaacgaa gaaatcgaga 9960

aacgttgtag aattacaggg tgctctcaat caaatggttt ctaataatga gaaaattatg 10020

actgcaatca aaatagggac agggaagtta ggacttttga taaatgacga tgagactatg 10080

caatctgcag attacttgaa ttatggaaaa ataccgattt tccgtggagt gattagaggg 10140

ttagagacca agagatggtc acgagtgact tgtgtcacca atgaccaaat acccacttgt 10200

gctaatataa tgagctcagt ttccacaaat gctctcaccg tagctcattt tgctgagaac 10260

ccaatcaatg ccatgataca gtacaattat tttgggacat ttgctagact cttgttgatg 10320

atgcatgatc ctgctcttcg tcaatcattg tatgaagttc aagataagat accgggcttg 10380

cacagttcta ctttcaaata cgccatgttg tatttggacc cttccattgg aggagtgtcg 10440

ggcatgtctt tgtccaggtt tttgattaga gccttcccag atcccgtaac agaaagtctc 10500

tcattctgga gattcatcca tgtacatgct cgaagtgagc atctgaagga gatgagtgca 10560

gtatttggaa accccgagat agccaagttt cgaataactc acatagacaa gctagtagaa 10620

gatccaacct ctctgaacat cgctatggga atgagtccag cgaacttgtt aaagactgag 10680

gttaaaaaat gcttaatcga atcaagacaa accatcagga accaggtgat taaggatgca 10740

accatatatt tgtatcatga agaggatcgg ctcagaagtt tcttatggtc aataaatcct 10800

ctgttcccta gatttttaag tgaattcaaa tcaggcactt ttttgggagt cgcagacggg 10860

ctcatcagtc tatttcaaaa ttctcgtact attcggaact cctttaagaa aaagtatcat 10920

agggaattgg atgatttgat tgtgaggagt gaggtatcct ctttgacaca tttagggaaa 10980

cttcatttga gaaggggatc atgtaaaatg tggacatgtt cagctactca tgctgacaca 11040

ttaagataca aatcctgggg ccgtacagtt attgggacaa ctgtacccca tccattagaa 11100

atgttgggtc cacaacatcg aaaagagact ccttgtgcac catgtaacac atcagggttc 11160

aattatgttt ctgtgcattg tccagacggg atccatgacg tctttagttc acggggacca 11220

ttgcctgctt atctagggtc taaaacatct gaatctacat ctattttgca gccttgggaa 11280

agggaaagca aagtcccact gattaaaaga gctacacgtc ttagagatgc tatctcttgg 11340

tttgttgaac ccgactctaa actagcaatg actatacttt ctaacatcca ctctttaaca 11400

ggcgaagaat ggaccaaaag gcagcatggg ttcaaaagaa cagggtctgc ccttcatagg 11460

ttttcgacat ctcggatgag ccatggtggg ttcgcatctc agagcactgc agcattgacc 11520

aggttgatgg caactacaga caccatgagg gatctgggag atcagaattt cgacttttta 11580

ttccaagcaa cgttgctcta tgctcaaatt accaccactg ttgcaagaga cggatggatc 11640

accagttgta cagatcatta tcatattgcc tgtaagtcct gtttgagacc catagaagag 11700

atcaccctgg actcaagtat ggactacacg cccccagatg tatcccatgt gctgaagaca 11760

tggaggaatg gggaaggttc gtggggacaa gagataaaac agatctatcc tttagaaggg 11820

aattggaaga atttagcacc tgctgagcaa tcctatcaag tcggcagatg tataggtttt 11880

ctatatggag acttggcgta tagaaaatct actcatgccg aggacagttc tctatttcct 11940

ctatctatac aaggtcgtat tagaggtcga ggtttcttaa aagggttgct agacggatta 12000

atgagagcaa gttgctgcca agtaatacac cggagaagtc tggctcattt gaagaggccg 12060

gccaacgcag tgtacggagg tttgatttac ttgattgata aattgagtgt atcacctcca 12120

ttcctttctc ttactagatc aggacctatt agagacgaat tagaaacgat tccccacaag 12180

atcccaacct cctatccgac aagcaaccgt gatatggggg tgattgtcag aaattacttc 12240

aaataccaat gccgtctaat tgaaaaggga aaatacagat cacattattc acaattatgg 12300

ttattctcag atgtcttatc catagacttc attggaccat tctctatttc caccaccctc 12360

ttgcaaatcc tatacaagcc atttttatct gggaaagata agaatgagtt gagagagctg 12420

gcaaatcttt cttcattgct aagatcagga gaggggtggg aagacataca tgtgaaattc 12480

ttcaccaagg acatattatt gtgtccagag gaaatcagac atgcttgcaa gttcgggatt 12540

gctaaggata ataataaaga catgagctat cccccttggg gaagggaatc cagagggaca 12600

attacaacaa tccctgttta ttatacgacc accccttacc caaagatgct agagatgcct 12660

ccaagaatcc aaaatcccct gctgtccgga atcaggttgg gccaattacc aactggcgct 12720

cattataaaa ttcggagtat attacatgga atgggaatcc attacaggga cttcttgagt 12780

tgtggagacg gctccggagg gatgactgct gcattactac gagaaaatgt gcatagcaga 12840

ggaatattca atagtctgtt agaattatca gggtcagtca tgcgaggcgc ctctcctgag 12900

ccccccagtg ccctagaaac tttaggagga gataaatcga gatgtgtaaa tggtgaaaca 12960

tgttgggaat atccatctga cttatgtgac ccaaggactt gggactattt cctccgactc 13020

aaagcaggct tggggcttca aattgattta attgtaatgg atatggaagt tcgggattct 13080

tctactagcc tgaaaattga gacgaatgtt agaaattatg tgcaccggat tttggatgag 13140

caaggagttt taatctacaa gacttatgga acatatattt gtgagagcga aaagaatgca 13200

gtaacaatcc ttggtcccat gttcaagacg gtcgacttag ttcaaacaga atttagtagt 13260

tctcaaacgt ctgaagtata tatggtatgt aaaggtttga agaaattaat cgatgaaccc 13320

aatcccgatt ggtcttccat caatgaatcc tggaaaaacc tgtacgcatt ccagtcatca 13380

gaacaggaat ttgccagagc aaagaaggtt agtacatact ttaccttgac aggtattccc 13440

tcccaattca ttcctgatcc ttttgtaaac attgagacta tgctacaaat attcggagta 13500

cccacgggtg tgtctcatgc ggctgcctta aaatcatctg atagacctgc agatttattg 13560

accattagcc ttttttatat ggcgattata tcgtattata acatcaatca tatcagagta 13620

ggaccgatac ctccgaaccc cccatcagat ggaattgcac aaaatgtggg gatcgctata 13680

actggtataa gcttttggct gagtttgatg gagaaagaca ttccactata tcaacagtgt 13740

ttagcagtta tccagcaatc attcccgatt aggtgggagg ctgtttcagt aaaaggagga 13800

tacaagcaga agtggagtac tagaggtgat gggctcccaa aagatacccg aatttcagac 13860

tccttggccc caatcgggaa ctggatcaga tctctggaat tggtccgaaa ccaagttcgt 13920

ctaaatccat tcaatgagat cttgttcaat cagctatgtc gtacagtgga taatcatttg 13980

aaatggtcaa atttgcgaag aaacacagga atgattgaat ggatcaatag acgaatttca 14040

aaagaagacc ggtctatact gatgttgaag agtgacctac acgaggaaaa ctcttggaga 14100

gattaaaaaa tcatgaggag actccaaact ttaagtatga aaaaaacttt gatccttaag 14160

accctcttgt ggtttttatt ttttatctgg ttttgtggtc ttcgtgggtc ggcatggcat 14220

ctccacctcc tcgcggtccg acctgggcat ccgaaggagg acgtcgtcca ctcggatggc 14280

taagggaggg gcccccgcgg ggctgctaac aaagcccgaa aggaagctga gttggctgct 14340

gccaccgctg agcaataact agcataaccc cttggggcct ctaaacgggt cttgaggggt 14400

tttttgctga aaggaggaac tatatccgga tcgagacctc gatactagtg cggtggagct 14460

ccagcttttg ttccctttag tgagggttaa tttcgagctt ggcgtaatca tggtcatagc 14520

tgtttcctgt gtgaaattgt tatccgctca caattccaca caacatacga gccggaagca 14580

taaagtgtaa agcctggggt gcctaatgag tgagctaact cacattaatt gcgttgcgct 14640

cactgcccgc tttccagtcg ggaaacctgt cgtgccagct gcattaatga atcggccaac 14700

gcgcggggag aggcggtttg cgtattgggc gctcttccgc ttcctcgctc actgactcgc 14760

tgcgctcggt cgttcggctg cggcgagcgg tatcagctca ctcaaaggcg gtaatacggt 14820

tatccacaga atcaggggat aacgcaggaa agaacatgtg agcaaaaggc cagcaaaagg 14880

ccaggaaccg taaaaaggcc gcgttgctgg cgtttttcca taggctccgc ccccctgacg 14940

agcatcacaa aaatcgacgc tcaagtcaga ggtggcgaaa cccgacagga ctataaagat 15000

accaggcgtt tccccctgga agctccctcg tgcgctctcc tgttccgacc ctgccgctta 15060

ccggatacct gtccgccttt ctcccttcgg gaagcgtggc gctttctcat agctcacgct 15120

gtaggtatct cagttcggtg taggtcgttc gctccaagct gggctgtgtg cacgaacccc 15180

ccgttcagcc cgaccgctgc gccttatccg gtaactatcg tcttgagtcc aacccggtaa 15240

gacacgactt atcgccactg gcagcagcca ctggtaacag gattagcaga gcgaggtatg 15300

taggcggtgc tacagagttc ttgaagtggt ggcctaacta cggctacact agaaggacag 15360

tatttggtat ctgcgctctg ctgaagccag ttaccttcgg aaaaagagtt ggtagctctt 15420

gatccggcaa acaaaccacc gctggtagcg gtggtttttt tgtttgcaag cagcagatta 15480

cgcgcagaaa aaaaggatct caagaagatc ctttgatctt ttctacgggg tctgacgctc 15540

agtggaacga aaactcacgt taagggattt tggtcatgag attatcaaaa aggatcttca 15600

cctagatcct tttaaattaa aaatgaagtt ttaaatcaat ctaaagtata tatgagtaaa 15660

cttggtctga cagttaccaa tgcttaatca gtgaggcacc tatctcagcg atctgtctat 15720

ttcgttcatc catagttgcc tgactccccg tcgtgtagat aactacgata cgggagggct 15780

taccatctgg ccccagtgct gcaatgatac cgcgagaccc acgctcaccg gctccagatt 15840

tatcagcaat aaaccagcca gccggaaggg ccgagcgcag aagtggtcct gcaactttat 15900

ccgcctccat ccagtctatt aattgttgcc gggaagctag agtaagtagt tcgccagtta 15960

atagtttgcg caacgttgtt gccattgcta caggcatcgt ggtgtcacgc tcgtcgtttg 16020

gtatggcttc attcagctcc ggttcccaac gatcaaggcg agttacatga tcccccatgt 16080

tgtgcaaaaa agcggttagc tccttcggtc ctccgatcgt tgtcagaagt aagttggccg 16140

cagtgttatc actcatggtt atggcagcac tgcataattc tcttactgtc atgccatccg 16200

taagatgctt ttctgtgact ggtgagtact caaccaagtc attctgagaa tagtgtatgc 16260

ggcgaccgag ttgctcttgc ccggcgtcaa tacgggataa taccgcgcca catagcagaa 16320

ctttaaaagt gctcatcatt ggaaaacgtt cttcggggcg aaaactctca aggatcttac 16380

cgctgttgag atccagttcg atgtaaccca ctcgtgcacc caactgatct tcagcatctt 16440

ttactttcac cagcgtttct gggtgagcaa aaacaggaag gcaaaatgcc gcaaaaaagg 16500

gaataagggc gacacggaaa tgttgaatac tcatactctt cctttttcaa tattattgaa 16560

gcatttatca gggttattgt ctcatgagcg gatacatatt tgaatgtatt tagaaaaata 16620

aacaaatagg ggttccgcgc acatttcccc gaaaagtgc 16659

<210> 17
<211> 11161
<212> DNA
<213> Vesicular stomatitis Indiana virus

<220>
<223> VSV Indiana complete genome

<400> 17
acgaagacaa acaaaccatt attatcatta aaaggctcag gagaaacttt aacagtaatc 60

aaaatgtctg ttacagtcaa gagaatcatt gacaacacag tcatagttcc aaaacttcct 120

gcaaatgagg atccagtgga atacccggca gattacttca gaaaatcaaa ggagattcct 180

ctttacatca atactacaaa aagtttgtca gatctaagag gatatgtcta ccaaggcctc 240

aaatccggaa atgtatcaat catacatgtc aacagctact tgtatggagc attaaaggac 300

atccggggta agttggataa agattggtca agtttcggaa taaacatcgg gaaagcaggg 360

gatacaatcg gaatatttga ccttgtatcc ttgaaagccc tggacggcgt acttccagat 420

ggagtatcgg atgcttccag aaccagcgca gatgacaaat ggttgccttt gtatctactt 480

ggcttataca gagtgggcag aacacaaatg cctgaataca gaaaaaagct catggatggg 540

ctgacaaatc aatgcaaaat gatcaatgaa cagtttgaac ctcttgtgcc agaaggtcgt 600

gacatttttg atgtgtgggg aaatgacagt aattacacaa aaattgtcgc tgcagtggac 660

atgttcttcc acatgttcaa aaaacatgaa tgtgcctcgt tcagatacgg aactattgtt 720

tccagattca aagattgtgc tgcattggca acatttggac acctctgcaa aataaccgga 780

atgtctacag aagatgtaac gacctggatc ttgaaccgag aagttgcaga tgaaatggtc 840

caaatgatgc ttccaggcca agaaattgac aaggccgatt catacatgcc ttatttgatc 900

gactttggat tgtcttctaa gtctccatat tcttccgtca aaaaccctgc cttccacttc 960

tgggggcaat tgacagctct tctgctcaga tccaccagag caaggaatgc ccgacagcct 1020

gatgacattg agtatacatc tcttactaca gcaggtttgt tgtacgctta tgcagtagga 1080

tcctctgccg acttggcaca acagttttgt gttggagata acaaatacac tccagatgat 1140

agtaccggag gattgacgac taatgcaccg ccacaaggca gagatgtggt cgaatggctc 1200

ggatggtttg aagatcaaaa cagaaaaccg actcctgata tgatgcagta tgcgaaaaga 1260

gcagtcatgt cactgcaagg cctaagagag aagacaattg gcaagtatgc taagtcagaa 1320

tttgacaaat gaccctataa ttctcagatc acctattata tattatgcta catatgaaaa 1380

aaactaacag atatcatgga taatctcaca aaagttcgtg agtatctcaa gtcctattct 1440

cgtctggatc aggcggtagg agagatagat gagatcgaag cacaacgagc tgaaaagtcc 1500

aattatgagt tgttccaaga ggatggagtg gaagagcata ctaagccctc ttattttcag 1560

gcagcagatg attctgacac agaatctgaa ccagaaattg aagacaatca aggtttgtat 1620

gcacaggatc cagaagctga gcaagttgaa ggctttatac aggggccttt agatgactat 1680

gcagatgagg aagtggatgt tgtatttact tcggactgga aaccacctga gcttgaatct 1740

gacgagcatg gaaagacctt acggttgaca tcgccagagg gtttaagtgg agagcagaaa 1800

tcccagtggc tttcgacgat taaagcagtc gtgcaaagtg ccaaatactg gaatctggca 1860

gagtgcacat ttgaagcatc gggagaaggg gtcattatga aggagcgcca gataactccg 1920

gatgtatata aggtcactcc agtgatgaac acacatccgt cccaatcaga agcagtatca 1980

gatgtttggt ctctctcaaa gacatccatg actttccaac ccaagaaagc aagtcttcag 2040

cctctcacca tatccttgga tgaattgttc tcatctagag gagagttcat ctctgtcgga 2100

ggtgacggac gaatgtctca taaagaggcc atcctgctcg gcctgagata caaaaagttg 2160

tacaatcagg cgagagtcaa atattctctg tagactatga aaaaaagtaa cagatatcac 2220

gatctaagtg ttatcccaat ccattcatca tgagttcctt aaagaagatt ctcggtctga 2280

aggggaaagg taagaaatct aagaaattag ggatcgcacc acccccttat gaagaggaca 2340

ctagcatgga gtatgctccg agcgctccaa ttgacaaatc ctattttgga gttgacgaga 2400

tggacaccta tgatccgaat caattaagat atgagaaatt cttctttaca gtgaaaatga 2460

cggttagatc taatcgtccg ttcagaacat actcagatgt ggcagccgct gtatcccatt 2520

gggatcacat gtacatcgga atggcaggga aacgtccctt ctacaaaatc ttggcttttt 2580

tgggttcttc taatctaaag gccactccag cggtattggc agatcaaggt caaccagagt 2640

atcacactca ctgcgaaggc agggcttatt tgccacatag gatggggaag acccctccca 2700

tgctcaatgt accagagcac ttcagaagac cattcaatat aggtctttac aagggaacga 2760

ttgagctcac aatgaccatc tacgatgatg agtcactgga agcagctcct atgatctggg 2820

atcatttcaa ttcttccaaa ttttctgatt tcagagagaa ggccttaatg tttggcctga 2880

ttgtcgagaa aaaggcatct ggagcgtggg tcctggattc tatcagccac ttcaaatgag 2940

ctagtctaac ttctagcttc tgaacaatcc ccggtttact cagtctctcc taattccagc 3000

ctctcgaaca actaatatcc tgtcttttct atccctatga aaaaaactaa cagagatcga 3060

tctgtttcct tgacactatg aagtgccttt tgtacttagc ctttttattc attggggtga 3120

attgcaagtt caccatagtt tttccacaca accaaaaagg aaactggaaa aatgttcctt 3180

ctaattacca ttattgcccg tcaagctcag atttaaattg gcataatgac ttaataggca 3240

cagccataca agtcaaaatg cccaagagtc acaaggctat tcaagcagac ggttggatgt 3300

gtcatgcttc caaatgggtc actacttgtg atttccgctg gtatggaccg aagtatataa 3360

cacagtccat ccgatccttc actccatctg tagaacaatg caaggaaagc attgaacaaa 3420

cgaaacaagg aacttggctg aatccaggct tccctcctca aagttgtgga tatgcaactg 3480

tgacggatgc cgaagcagtg attgtccagg tgactcctca ccatgtgctg gttgatgaat 3540

acacaggaga atgggttgat tcacagttca tcaacggaaa atgcagcaat tacatatgcc 3600

ccactgtcca taactctaca acctggcatt ctgactataa ggtcaaaggg ctatgtgatt 3660

ctaacctcat ttccatggac atcaccttct tctcagagga cggagagcta tcatccctgg 3720

gaaaggaggg cacagggttc agaagtaact actttgctta tgaaactgga ggcaaggcct 3780

gcaaaatgca atactgcaag cattggggag tcagactccc atcaggtgtc tggttcgaga 3840

tggctgataa ggatctcttt gctgcagcca gattccctga atgcccagaa gggtcaagta 3900

tctctgctcc atctcagacc tcagtggatg taagtctaat tcaggacgtt gagaggatct 3960

tggattattc cctctgccaa gaaacctgga gcaaaatcag agcgggtctt ccaatctctc 4020

cagtggatct cagctatctt gctcctaaaa acccaggaac cggtcctgct ttcaccataa 4080

tcaatggtac cctaaaatac tttgagacca gatacatcag agtcgatatt gctgctccaa 4140

tcctctcaag aatggtcgga atgatcagtg gaactaccac agaaagggaa ctgtgggatg 4200

actgggcacc atatgaagac gtggaaattg gacccaatgg agttctgagg accagttcag 4260

gatataagtt tcctttatac atgattggac atggtatgtt ggactccgat cttcatctta 4320

gctcaaaggc tcaggtgttc gaacatcctc acattcaaga cgctgcttcg caacttcctg 4380

atgatgagag tttatttttt ggtgatactg ggctatccaa aaatccaatc gagcttgtag 4440

aaggttggtt cagtagttgg aaaagctcta ttgcctcttt tttctttatc atagggttaa 4500

tcattggact attcttggtt ctccgagttg gtatccatct ttgcattaaa ttaaagcaca 4560

ccaagaaaag acagatttat acagacatag agatgaaccg acttggaaag taactcaaat 4620

cctgcacaac agattcttca tgtttggacc aaatcaactt gtgataccat gctcaaagag 4680

gcctcaatta tatttgagtt tttaattttt atgaaaaaaa ctaacagcaa tcatggaagt 4740

ccacgatttt gagaccgacg agttcaatga tttcaatgaa gatgactatg ccacaagaga 4800

attcctgaat cccgatgagc gcatgacgta cttgaatcat gctgattaca atttgaattc 4860

tcctctaatt agtgatgata ttgacaattt gatcaggaaa ttcaattctc ttccgattcc 4920

ctcgatgtgg gatagtaaga actgggatgg agttcttgag atgttaacat catgtcaagc 4980

caatcccatc tcaacatctc agatgcataa atggatggga agttggttaa tgtctgataa 5040

tcatgatgcc agtcaagggt atagtttttt acatgaagtg gacaaagagg cagaaataac 5100

atttgacgtg gtggagacct tcatccgcgg ctggggcaac aaaccaattg aatacatcaa 5160

aaaggaaaga tggactgact cattcaaaat tctcgcttat ttgtgtcaaa agtttttgga 5220

cttacacaag ttgacattaa tcttaaatgc tgtctctgag gtggaattgc tcaacttggc 5280

gaggactttc aaaggcaaag tcagaagaag ttctcatgga acgaacatat gcaggattag 5340

ggttcccagc ttgggtccta cttttatttc agaaggatgg gcttacttca agaaacttga 5400

tattctaatg gaccgaaact ttctgttaat ggtcaaagat gtgattatag ggaggatgca 5460

aacggtgcta tccatggtat gtagaataga caacctgttc tcagagcaag acatcttctc 5520

ccttctaaat atctacagaa ttggagataa aattgtggag aggcagggaa atttttctta 5580

tgacttgatt aaaatggtgg aaccgatatg caacttgaag ctgatgaaat tagcaagaga 5640

atcaaggcct ttagtcccac aattccctca ttttgaaaat catatcaaga cttctgttga 5700

tgaaggggca aaaattgacc gaggtataag attcctccat gatcagataa tgagtgtgaa 5760

aacagtggat ctcacactgg tgatttatgg atcgttcaga cattggggtc atccttttat 5820

agattattac actggactag aaaaattaca ttcccaagta accatgaaga aagatattga 5880

tgtgtcatat gcaaaagcac ttgcaagtga tttagctcgg attgttctat ttcaacagtt 5940

caatgatcat aaaaagtggt tcgtgaatgg agacttgctc cctcatgatc atccctttaa 6000

aagtcatgtt aaagaaaata catggcccac agctgctcaa gttcaagatt ttggagataa 6060

atggcatgaa cttccgctga ttaaatgttt tgaaataccc gacttactag acccatcgat 6120

aatatactct gacaaaagtc attcaatgaa taggtcagag gtgttgaaac atgtccgaat 6180

gaatccgaac actcctatcc ctagtaaaaa ggtgttgcag actatgttgg acacaaaggc 6240

taccaattgg aaagaatttc ttaaagagat tgatgagaag ggcttagatg atgatgatct 6300

aattattggt cttaaaggaa aggagaggga actgaagttg gcaggtagat ttttctccct 6360

aatgtcttgg aaattgcgag aatactttgt aattaccgaa tatttgataa agactcattt 6420

cgtccctatg tttaaaggcc tgacaatggc ggacgatcta actgcagtca ttaaaaagat 6480

gttagattcc tcatccggcc aaggattgaa gtcatatgag gcaatttgca tagccaatca 6540

cattgattac gaaaaatgga ataaccacca aaggaagtta tcaaacggcc cagtgttccg 6600

agttatgggc cagttcttag gttatccatc cttaatcgag agaactcatg aattttttga 6660

gaaaagtctt atatactaca atggaagacc agacttgatg cgtgttcaca acaacacact 6720

gatcaattca acctcccaac gagtttgttg gcaaggacaa gagggtggac tggaaggtct 6780

acggcaaaaa ggatggacta tcctcaatct actggttatt caaagagagg ctaaaatcag 6840

aaacactgct gtcaaagtct tggcacaagg tgataatcaa gttatttgca cacagtataa 6900

aacgaagaaa tcgagaaacg ttgtagaatt acagggtgct ctcaatcaaa tggtttctaa 6960

taatgagaaa attatgactg caatcaaaat agggacaggg aagttaggac ttttgataaa 7020

tgacgatgag actatgcaat ctgcagatta cttgaattat ggaaaaatac cgattttccg 7080

tggagtgatt agagggttag agaccaagag atggtcacga gtgacttgtg tcaccaatga 7140

ccaaataccc acttgtgcta atataatgag ctcagtttcc acaaatgctc tcaccgtagc 7200

tcattttgct gagaacccaa tcaatgccat gatacagtac aattattttg ggacatttgc 7260

tagactcttg ttgatgatgc atgatcctgc tcttcgtcaa tcattgtatg aagttcaaga 7320

taagataccg ggcttgcaca gttctacttt caaatacgcc atgttgtatt tggacccttc 7380

cattggagga gtgtcgggca tgtctttgtc caggtttttg attagagcct tcccagatcc 7440

cgtaacagaa agtctctcat tctggagatt catccatgta catgctcgaa gtgagcatct 7500

gaaggagatg agtgcagtat ttggaaaccc cgagatagcc aagtttcgaa taactcacat 7560

agacaagcta gtagaagatc caacctctct gaacatcgct atgggaatga gtccagcgaa 7620

cttgttaaag actgaggtta aaaaatgctt aatcgaatca agacaaacca tcaggaacca 7680

ggtgattaag gatgcaacca tatatttgta tcatgaagag gatcggctca gaagtttctt 7740

atggtcaata aatcctctgt tccctagatt tttaagtgaa ttcaaatcag gcactttttt 7800

gggagtcgca gacgggctca tcagtctatt tcaaaattct cgtactattc ggaactcctt 7860

taagaaaaag tatcataggg aattggatga tttgattgtg aggagtgagg tatcctcttt 7920

gacacattta gggaaacttc atttgagaag gggatcatgt aaaatgtgga catgttcagc 7980

tactcatgct gacacattaa gatacaaatc ctggggccgt acagttattg ggacaactgt 8040

accccatcca ttagaaatgt tgggtccaca acatcgaaaa gagactcctt gtgcaccatg 8100

taacacatca gggttcaatt atgtttctgt gcattgtcca gacgggatcc atgacgtctt 8160

tagttcacgg ggaccattgc ctgcttatct agggtctaaa acatctgaat ctacatctat 8220

tttgcagcct tgggaaaggg aaagcaaagt cccactgatt aaaagagcta cacgtcttag 8280

agatgctatc tcttggtttg ttgaacccga ctctaaacta gcaatgacta tactttctaa 8340

catccactct ttaacaggcg aagaatggac caaaaggcag catgggttca aaagaacagg 8400

gtctgccctt cataggtttt cgacatctcg gatgagccat ggtgggttcg catctcagag 8460

cactgcagca ttgaccaggt tgatggcaac tacagacacc atgagggatc tgggagatca 8520

gaatttcgac tttttattcc aagcaacgtt gctctatgct caaattacca ccactgttgc 8580

aagagacgga tggatcacca gttgtacaga tcattatcat attgcctgta agtcctgttt 8640

gagacccata gaagagatca ccctggactc aagtatggac tacacgcccc cagatgtatc 8700

ccatgtgctg aagacatgga ggaatgggga aggttcgtgg ggacaagaga taaaacagat 8760

ctatccttta gaagggaatt ggaagaattt agcacctgct gagcaatcct atcaagtcgg 8820

cagatgtata ggttttctat atggagactt ggcgtataga aaatctactc atgccgagga 8880

cagttctcta tttcctctat ctatacaagg tcgtattaga ggtcgaggtt tcttaaaagg 8940

gttgctagac ggattaatga gagcaagttg ctgccaagta atacaccgga gaagtctggc 9000

tcatttgaag aggccggcca acgcagtgta cggaggtttg atttacttga ttgataaatt 9060

gagtgtatca cctccattcc tttctcttac tagatcagga cctattagag acgaattaga 9120

aacgattccc cacaagatcc caacctccta tccgacaagc aaccgtgata tgggggtgat 9180

tgtcagaaat tacttcaaat accaatgccg tctaattgaa aagggaaaat acagatcaca 9240

ttattcacaa ttatggttat tctcagatgt cttatccata gacttcattg gaccattctc 9300

tatttccacc accctcttgc aaatcctata caagccattt ttatctggga aagataagaa 9360

tgagttgaga gagctggcaa atctttcttc attgctaaga tcaggagagg ggtgggaaga 9420

catacatgtg aaattcttca ccaaggacat attattgtgt ccagaggaaa tcagacatgc 9480

ttgcaagttc gggattgcta aggataataa taaagacatg agctatcccc cttggggaag 9540

ggaatccaga gggacaatta caacaatccc tgtttattat acgaccaccc cttacccaaa 9600

gatgctagag atgcctccaa gaatccaaaa tcccctgctg tccggaatca ggttgggcca 9660

attaccaact ggcgctcatt ataaaattcg gagtatatta catggaatgg gaatccatta 9720

cagggacttc ttgagttgtg gagacggctc cggagggatg actgctgcat tactacgaga 9780

aaatgtgcat agcagaggaa tattcaatag tctgttagaa ttatcagggt cagtcatgcg 9840

aggcgcctct cctgagcccc ccagtgccct agaaacttta ggaggagata aatcgagatg 9900

tgtaaatggt gaaacatgtt gggaatatcc atctgactta tgtgacccaa ggacttggga 9960

ctatttcctc cgactcaaag caggcttggg gcttcaaatt gatttaattg taatggatat 10020

ggaagttcgg gattcttcta ctagcctgaa aattgagacg aatgttagaa attatgtgca 10080

ccggattttg gatgagcaag gagttttaat ctacaagact tatggaacat atatttgtga 10140

gagcgaaaag aatgcagtaa caatccttgg tcccatgttc aagacggtcg acttagttca 10200

aacagaattt agtagttctc aaacgtctga agtatatatg gtatgtaaag gtttgaagaa 10260

attaatcgat gaacccaatc ccgattggtc ttccatcaat gaatcctgga aaaacctgta 10320

cgcattccag tcatcagaac aggaatttgc cagagcaaag aaggttagta catactttac 10380

cttgacaggt attccctccc aattcattcc tgatcctttt gtaaacattg agactatgct 10440

acaaatattc ggagtaccca cgggtgtgtc tcatgcggct gccttaaaat catctgatag 10500

acctgcagat ttattgacca ttagcctttt ttatatggcg attatatcgt attataacat 10560

caatcatatc agagtaggac cgatacctcc gaacccccca tcagatggaa ttgcacaaaa 10620

tgtggggatc gctataactg gtataagctt ttggctgagt ttgatggaga aagacattcc 10680

actatatcaa cagtgtttag cagttatcca gcaatcattc ccgattaggt gggaggctgt 10740

ttcagtaaaa ggaggataca agcagaagtg gagtactaga ggtgatgggc tcccaaaaga 10800

tacccgaact tcagactcct tggccccaat cgggaactgg atcagatctc tggaattggt 10860

ccgaaaccaa gttcgtctaa atccattcaa tgagatcttg ttcaatcagc tatgtcgtac 10920

agtggataat catttgaaat ggtcaaattt gcgaagaaac acaggaatga ttgaatggat 10980

caatagacga atttcaaaag aagaccggtc tatactgatg ttgaagagtg acctacacga 11040

ggaaaactct tggagagatt aaaaaatcat gaggagactc caaactttaa gtatgaaaaa 11100

aactttgatc cttaagaccc tcttgtggtt tttatttttt atctggtttt gtggtcttcg 11160

t 11161

<210> 18
<211> 511
<212> PRT
<213> Vesicular stomatitis Indiana virus

<220>
<223> VSV Indiana G protein

<400> 18
Met Lys Cys Phe Leu Tyr Leu Ala Phe Leu Phe Ile Gly Val Asn Cys
1               5                   10                  15
Lys Phe Thr Ile Val Phe Pro His Asn Gln Lys Gly Asn Trp Lys Asn
            20                  25                  30
Val Pro Ser Asn Tyr His Tyr Cys Pro Ser Ser Ser Asp Leu Asn Trp
        35                  40                  45
His Asn Asp Leu Ile Gly Thr Gly Leu Gln Val Lys Met Pro Lys Ser
    50                  55                  60
His Lys Ala Ile Gln Ala Asp Gly Trp Met Cys His Ala Ser Lys Trp
65                  70                  75                  80
Val Thr Thr Cys Asp Phe Arg Trp Tyr Gly Pro Lys Tyr Ile Thr His
                85                  90                  95
Ser Ile Arg Ser Phe Thr Pro Ser Val Glu Gln Cys Lys Glu Ser Ile
            100                 105                 110
Glu Gln Thr Lys Gln Gly Thr Trp Leu Asn Pro Gly Phe Pro Pro Gln
        115                 120                 125
Ser Cys Gly Tyr Ala Thr Val Thr Asp Ala Glu Ala Val Ile Val Gln
    130                 135                 140
Val Thr Pro His His Val Leu Val Asp Glu Tyr Thr Gly Glu Trp Val
145                 150                 155                 160
Asp Ser Gln Phe Ile Asn Gly Lys Cys Ser Asn Asp Ile Cys Pro Thr
                165                 170                 175
Val His Asn Ser Thr Thr Trp His Ser Asp Tyr Lys Val Lys Gly Leu
            180                 185                 190
Cys Asp Ser Asn Leu Ile Ser Thr Asp Ile Thr Phe Phe Ser Glu Asp
        195                 200                 205
Arg Glu Leu Ser Ser Leu Gly Lys Glu Gly Thr Gly Phe Arg Ser Asn
    210                 215                 220
Tyr Phe Ala Tyr Glu Thr Gly Asp Lys Ala Cys Lys Met Gln Tyr Cys
225                 230                 235                 240

Lys His Trp Gly Val Arg Leu Pro Ser Gly Val Trp Phe Glu Met Ala
            245                 250                 255
Asp Lys Asp Leu Phe Ala Ala Ala Arg Phe Pro Glu Cys Pro Glu Gly
            260                 265                 270
Ser Ser Ile Ser Ala Pro Ser Gln Thr Ser Val Asp Val Ser Leu Ile
        275                 280                 285
Gln Asp Val Glu Arg Ile Leu Asp Tyr Ser Leu Cys Gln Glu Thr Trp
    290                 295                 300
Ser Lys Ile Arg Ala Gly Leu Pro Ile Ser Pro Val Asp Leu Ser Tyr
305                 310                 315                 320
Leu Ala Pro Lys Asn Pro Gly Thr Gly Pro Ala Phe Thr Ile Ile Asn
                325                 330                 335
Gly Thr Leu Lys Tyr Phe Glu Thr Arg Tyr Ile Arg Val Asp Ile Ala
            340                 345                 350
Ala Pro Ile Leu Ser Arg Met Val Gly Met Ile Ser Gly Thr Thr Thr
        355                 360                 365
Glu Arg Glu Leu Trp Asp Asp Trp Ala Pro Tyr Glu Asp Val Glu Ile
    370                 375                 380
Gly Pro Asn Gly Val Leu Arg Thr Ser Ser Gly Tyr Lys Phe Pro Leu
385                 390                 395                 400
Tyr Met Ile Gly His Gly Met Leu Asp Ser Gly Leu His Leu Ser Ser
                405                 410                 415
Lys Ala Gln Val Phe Glu His Pro His Ile Gln Asp Ala Ala Ser Gln
            420                 425                 430
Leu Pro Asp Asp Glu Ile Leu Phe Phe Gly Asp Thr Gly Leu Ser Lys
        435                 440                 445
Asn Pro Ile Asp Phe Val Glu Gly Trp Phe Ser Ser Trp Lys Ser Ser
    450                 455                 460
Ile Ala Ser Phe Phe Phe Ile Ile Gly Leu Ile Ile Gly Leu Phe Leu
465                 470                 475                 480
Val Leu Arg Val Gly Ile Tyr Leu Tyr Ile Lys Leu Lys His Thr Lys
                485                 490                 495
Lys Arg Gln Ile Tyr Thr Asp Ile Glu Met Asn Arg Leu Gly Arg
            500                 505                 510

<210> 19
<211> 1992
<212> DNA
<213> Newcastle disease virus

<220>
<223> NDV HN protein (nucleic acid sequence)

<400> 19
acgggtagaa cggtaagaga ggccgcccct caattgcgag ccagacttca caacctccgt      60

tctaccgctt caccgacaac agtcctcaat catggaccgc gccgttagcc aagttgcgtt     120

agagaatgat gaaagagagg caaaaaatac atggcgcttg atattccgga ttgcaatctt     180

attcttaaca gtagtgacct tggctatatc tgtagcctcc cttttatata gcatgggggc     240

tagcacacct agcgatcttg taggcatacc gactaggatt tccagggcag aagaaaagat     300

tacatctaca cttggttcca atcaagatgt agtagatagg atatataagc aagtggccct     360

tgagtctcca ttggcattgt taaatactga gaccacaatt atgaacgcaa taacatctct     420

ctcttatcag attaatggag ctgcaaacaa cagcgggtgg ggggcaccta ttcatgaccc     480

agattatata ggggggatag gcaaagaact cattgtagat gatgctagtg atgtcacatc     540

attctatccc tctgcatttc aagaacatct gaattttatc ccggcgccta ctacaggatc 600

aggttgcact cgaataccct catttgacat gagtgctacc cattactgct acacccataa 660

tgtaatattg tctggatgca gagatcactc acactcatat cagtatttag cacttggtgt 720

gctccggaca tctgcaacag ggagggtatt cttttctact ctgcgttcca tcaacctgga 780

cgacacccaa aatcggaagt cttgcagtgt gagtgcaact cccctgggtt gtgatatgct 840

gtgctcgaaa gccacggaga cagaggaaga agattataac tcagctgtcc ctacgcggat 900

ggtacatggg aggttagggt tcgacggcca atatcacgaa aaggacctag atgtcacaac 960

attattcggg gactgggtgg ccaactaccc aggagtaggg ggtggatctt ttattgacag 1020

ccgcgtatgg ttctcagtct acggagggtt aaaacccaat tcacccagtg acactgtaca 1080

ggaagggaaa tatgtgatat acaagcgata caatgacaca tgcccagatg agcaagacta 1140

ccagattcga atggccaagt cttcgtataa gcctggacgg tttggtggga aacgcataca 1200

gcaggctatc ttatctatca aagtgtcaac atccttaggc gaagacccgg tactgactgt 1260

accgcccaac acagtcacac tcatgggggc cgaaggcaga attctcacag tagggacatc 1320

ccatttcttg tatcagcgag ggtcatcata cttctctccc gcgttattat atcctatgac 1380

agtcagcaac aaaacagcca ctcttcatag tccttataca ttcaatgcct tcactcggcc 1440

aggtagtatc ccttgccagg cttcagcaag atgccccaac tcgtgtgtta ctggagtcta 1500

tacagatcca tatcccctaa tcttctatag aaaccacacc ttgcgagggg tattcgggac 1560

aatgcttgat ggtgaacaag caagacttaa ccctgcgtct gcagtattcg atagcacatc 1620

ccgcagtcgc ataactcgag tgagttcaag cagcatcaaa gcagcataca caacatcaac 1680

ttgttttaaa gtggtcaaga ccaataagac ctattgtctc agcattgctg aaatatctaa 1740

tactctcttc ggagaattca gaatcgtccc gttactagtt gagatcctca aagatgacgg 1800

ggttagagaa gccaggtctg gctagttgag tcaactatga aagagttgga aagatggcat 1860

tgtatcacct atcttctgcg acatcaagaa tcaaaccgaa tgccggcgcg tgctcgaatt 1920

ccatgtcgcc agttgaccac aatcagccag tgctcatgcg atcagattaa gccttgtcaa 1980

tagtctcttg at 1992

<210> 20
<211> 15186
<212> DNA
<213> Newcastle disease virus

<220>
<223> NDV Hitchner B1 complete genome

<400> 20
accaaacaga gaatccgtaa gttacgataa aaggcgaagg agcaattgaa gtcgcacggg 60

tagaaggtgt gaatctcgag tgcgagcccg aagcacaaac tcgaggaagc cttctgccaa 120

catgtcttcc gtattcgacg agtacgaaca gctcctcgcg gctcagactc gccccaatgg 180

agctcatgga gggggggaga aagggagtac cttaaaagta gacgtcccgg tattcactct 240

taacagtgat gacccagaag ataggtggag ctttgtggta ttctgcctcc ggattgctgt 300

tagcgaagat gccaacaaac cactcaggca aggtgctctc atatctcttt tatgctccca 360

ctcacaggta atgaggaacc atgttgccct tgcagggaaa cagaatgaag ccacattggc 420

cgtgcttgag attgatggct ttgccaacgg cacgccccag ttcaacaata ggagtggagt 480

gtctgaagag agagcacaga gatttgcgat gatagcagga tctctccctc gggcatgcag 540

caacggcacc ccgttcgtca cagccggggc tgaagatgat gcaccagaag acatcaccga 600

taccctggag aggatcctct ctatccaggc tcaagtatgg gtcacagtag caaaagccat 660

gactgcgtat gagactgcag atgagtcgga aacaaggcga atcaataagt atatgcagca 720

aggcagggtc caaaagaaat acatcctcta ccccgtatgc aggagcacaa tccaactcac 780

gatcagacag tctcttgcag tccgcatctt tttggttagc gagctcaaga gaggccgcaa 840

cacggcaggt ggtacctcta cttattataa cctagtaggg gacgtagact catatatcag 900

gaataccggg cttactgcat tcttcttgac actcaagtac ggaatcaaca ccaagacatc 960

agcccttgca cttagtagcc tctcaggcga catccagaag atgaagcagc tcatgcgttt 1020

gtatcggatg aaaggagata atgcgccgta catgacatta cttggtgata gtgaccagat 1080

gagctttgcg cctgccgagt atgcacaact ttactccttt gccatgggta tggcatcagt 1140

cctagataaa ggtactggga aataccaatt tgccagggac tttatgagca catcattctg 1200

gagacttgga gtagagtacg ctcaggctca gggaagtagc attaacgagg atatggctgc 1260

cgagctaaag ctaaccccgg cagcaaggag gggcctggca gctgctgccc aacgagtctc 1320

cgaggtgacc agcagcatag acatgcctac tcaacaagtc ggagtcctca ctgggcttag 1380

cgagggggga tcccaagccc tacaaggcgg atcgaataga tcgcaagggc aaccagaagc 1440

cggggatggg gagacccaat tcctggatct gatgagagcg gtagcaaata gcatgaggga 1500

ggcgccaaac tctgcacagg gcactcccca atcggggcct cccccaactc ctgggccatc 1560

ccaagataac gacaccgact gggggtattg attgacaaaa cccagcctgc ttctacaaga 1620

acatcccaat gctctcaccc gtagtcgacc cctcgatttg cggctctata tgaccacacc 1680

ctcaaacaaa catccccctc tttcctccct ccccctgctg tacaactccg cacgccctag 1740

ataccacagg cacaccgcgg ctcactaaca atcaaaacag agccgaggga attagaaaaa 1800

agtacgggta gaagagggat attcagagat cagggcaagt ctcccgagtc tctgctctct 1860

cctctacctg atagaccagg acaaacatgg ccacctttac agatgcagag atcgacgagc 1920

tatttgagac aagtggaact gtcattgaca acataattac agcccagggt aaaccagcag 1980

agactgttgg aaggagtgca atcccacagg gcaagaccaa ggtgctgagc gcagcatggg 2040

agaagcatgg gagcatccag ccaccggcca gtcaagacaa cctcgatcga caggacagat 2100

ctgacaaaca accatccaca cccgagcaaa cgaccccgca cgacagcccg ccggccacat 2160

ccgctgacca gccccccacc caggccacag acgaagccgt cgacacacag ctcaggaccg 2220

gagcaagcaa ctctctgctg ttgatgcttg acaagctcag caataaatcg tccaatgcta 2280

aaaagggccc atggtcgagc ccccaagagg ggaatcacca acgtccgact caacagcagg 2340

ggagtcaacc cagtcgcgga aacagccagg aaagactgca gaaccaagtc aaggccgccc 2400

ctggaaacca gggcacagac gtgaacacag catatcatgg acaatgggag gagtcacaac 2460

tatcagctgg tgcaacccct catgctctcc gatcaaggca gagccaagac aatacccttg 2520

tatctgcgga tcatgtccag ccacctgtag actttgtgca agcgatgatg tctatgatgg 2580

gggcgatatc acagagagta agtaaggttg actatcagct agatcttgtc ttgaaacaga 2640

catcctccat ccctatgatg cggtccgaaa tccaacagct gaaaacatct gttgcagtca 2700

tggaagccaa cttgggaatg atgaagattc tggatcccgg ttgtgccaac atttcatctc 2760

tgagtgatct acgggcagtt gcccgatctc acccggtttt agtttcaggc cctggagacc 2820

catctcccta tgtgatacaa ggaggcgaaa tggcacttaa taaactttcg caaccagtgc 2880

cacatccatc tgaattgatt aaacccgcca ctgcatgcgg gcctgatata ggagtggaga 2940

gggacactgt ccgtgcattg atcatgtcac gcccaatgca cccgagttct tcagccaagc 3000

tcctaagcaa gttagatgca gccgggtcga tcgaggaaat caggaaaatc aagcgccttg 3060

ctctaaatgg ctaattacta ctgccacacg tagcgggtcc ctgtccactc ggcatcacac 3120

ggaatctgca ccgagttccc ccccgcagac ccaaggtcca actctagaag cggcaatcct 3180

ctctcgcttc ctcagcccca ctgaatgatc gcgtaaccgt aattaatcta gctacattaa 3240

ggattaagaa aaaatacggg tagaattgga gtgccccaat tgtgccaaga tggactcatc 3300

taggacaatt gggctgtact ttgattctgc ccattcttct agcaacctgt tagcatttcc 3360

gatcgtccta caagacacag gagatgggaa gaagcaaatc gccccgcaat ataggatcca 3420

gcgccttgac tcgtggactg atagtaagga agactcagta ttcatcacca cctatggatt 3480

catctttcaa gttgggaatg aggaagccac tgtcggcatg atcgatgata aacccaagcg 3540

cgagttactt tccgctgcga tgctctgcct aggaagcgtc ccaaataccg gagaccttgt 3600

tgagctggca agggcctgtc tcactatgat ggtcacatgc aagaagagtg caactaatac 3660

tgagagaatg gttttctcag tagtgcaggc accccaagtg ctgcaaagct gtagggttgt 3720

ggcaaataaa tactcatcag tgaatgcagt caagcacgtg aaagcgccag agaagatccc 3780

cgggagtgga accctagaat acaaggtgaa ctttgtctcc ttgactgtgg taccgaagaa 3840

ggatgtctac aagatcccag ctgcagtatt gaagatttct ggctcgagtc tgtacaatct 3900

tgcgctcaat gtcactatta atgtggaggt agacccgagg agtcctttgg ttaaatctct 3960

gtctaagtct gacagcggat actatgctaa cctcttcttg catattggac ttatgaccac 4020

cgtagatagg aaggggaaga aagtgacatt tgacaagctg gaaaagaaaa taaggagcct 4080

tgatctatct gtcgggctca gtgatgtgct cgggccttcc gtgttggtaa aagcaagagg 4140

tgcacggact aagcttttgg cacctttctt ctctagcagt gggacagcct gctatcccat 4200

agcaaatgct tctcctcagg tggccaagat actctggagt caaaccgcgt gcctgcggag 4260

cgttaaaatc attatccaag caggtaccca acgcgctgtc gcagtgaccg ctgaccacga 4320

ggttacctct actaagctgg agaaggggca cacccttgcc aaatacaatc cttttaagaa 4380

ataagctgcg tctctgagat tgcgctccgc ccactcaccc agatcatcat gacacaaaaa 4440

actaatctgt cttgattatt tacagttagt ttacctgtcc atcaagttag aaaaaacacg 4500

ggtagaagat tctggatccc ggttggcgcc ctccaggtgc aggatgggct ccagaccttc 4560

taccaagaac ccagcaccta tgatgctgac tatccgggtc gcgctggtac tgagttgcat 4620

ctgcccggca aactccattg atggcaggcc tcttgcagct gcaggaattg tggttacagg 4680

agacaaagca gtcaacatat acacctcatc ccagacagga tcaatcatag ttaagctcct 4740

cccgaatctg cccaaggata aggaggcatg tgcgaaagcc cccttggatg catacaacag 4800

gacattgacc actttgctca ccccccttgg tgactctatc cgtaggatac aagagtctgt 4860

gactacatct ggagggggga gacaggggcg ccttataggc gccattattg gcggtgtggc 4920

tcttggggtt gcaactgccg cacaaataac agcggccgca gctctgatac aagccaaaca 4980

aaatgctgcc aacatcctcc gacttaaaga gagcattgcc gcaaccaatg aggctgtgca 5040

tgaggtcact gacggattat cgcaactagc agtggcagtt gggaagatgc agcagtttgt 5100

taatgaccaa tttaataaaa cagctcagga attagactgc atcaaaattg cacagcaagt 5160

tggtgtagag ctcaacctgt acctaaccga attgactaca gtattcggac cacaaatcac 5220

ttcacctgcc ttaaacaagc tgactattca ggcactttac aatctagctg gtgggaatat 5280

ggattactta ttgactaagt taggtatagg gaacaatcaa ctcagctcat taatcggtag 5340

cggcttaatc accggtaacc ctattctata cgactcacag actcaactct tgggtataca 5400

ggtaactcta ccttcagtcg ggaacctaaa taatatgcgt gccacctact tggaaacctt 5460

atccgtaagc acaaccaggg gatttgcctc ggcacttgtc ccaaaagtgg tgacacaggt 5520

cggttctgtg atagaagaac ttgacacctc atactgtata gaaactgact tagatttata 5580

ttgtacaaga atagtaacgt tccctatgtc ccctggtatt tactcctgct tgagcggcaa 5640

tacatcggcc tgtatgtact caaagaccga aggcgcactt actacaccat atatgactat 5700

caaaggctca gtcatcgcta actgcaagat gacaacatgt agatgtgtaa accccccggg 5760

tatcatatcg caaaactatg gagaagccgt gtctctaata gataaacaat catgcaatgt 5820

tttatcctta ggcgggataa ctttaaggct cagtggggaa ttcgatgtaa cttatcagaa 5880

gaatatctca atacaagatt ctcaagtaat aataacaggc aatcttgata tctcaactga 5940

gcttgggaat gtcaacaact cgatcagtaa tgctttgaat aagttagagg aaagcaacag 6000

aaaactagac aaagtcaatg tcaaactgac cagcacatct gctctcatta cctatatcgt 6060

tttgactatc atatctcttg tttttggtat acttagcctg attctagcat gctacctaat 6120

gtacaagcaa aaggcgcaac aaaagacctt attatggctt gggaataata ccctagatca 6180

gatgagagcc actacaaaaa tgtgaacaca gatgaggaac gaaggtttcc ctaatagtaa 6240

tttgtgtgaa agttctggta gtctgtcagt tcggagagtt aagaaaaaac taccggttgt 6300

agatgaccaa aggacgatat acgggtagaa cggtaagaga ggccgcccct caattgcgag 6360

ccagacttca caacctccgt tctaccgctt caccgacaac agtcctcaat catggaccgc 6420

gccgttagcc aagttgcgtt agagaatgat gaaagagagg caaaaaatac atggcgcttg 6480

atattccgga ttgcaatctt attcttaaca gtagtgacct tggctatatc tgtagcctcc 6540

cttttatata gcatgggggc tagcacacct agcgatcttg taggcatacc gactaggatt 6600

tccagggcag aagaaaagat tacatctaca cttggttcca atcaagatgt agtagatagg 6660

atatataagc aagtggccct tgagtctcca ttggcattgt taaatactga gaccacaatt 6720

atgaacgcaa taacatctct ctcttatcag attaatggag ctgcaaacaa cagcgggtgg 6780

ggggcaccta ttcatgaccc agattatata ggggggatag gcaaagaact cattgtagat 6840

gatgctagtg atgtcacatc attctatccc tctgcatttc aagaacatct gaattttatc 6900

ccggcgccta ctacaggatc aggttgcact cgaataccct catttgacat gagtgctacc 6960

cattactgct acacccataa tgtaatattg tctggatgca gagatcactc acactcacat 7020

cagtatttag cacttggtgt gctccggaca tctgcaacag ggagggtatt cttttctact 7080

ctgcgttcca tcaacctgga cgacacccaa aatcggaagt cttgcagtgt gagtgcaact 7140

cccctgggtt gtgatatgct gtgctcgaaa gccacggaga cagaggaaga agattataac 7200

tcagctgtcc ctacgcggat ggtacatggg aggttagggt tcgacggcca atatcacgaa 7260

aaggacctag atgtcacaac attattcggg gactgggtgg ccaactaccc aggagtaggg 7320

ggtggatctt ttattgacag ccgcgtatgg ttctcagtct acggagggtt aaaacccaat 7380

acacccagtg acactgtaca ggaagggaaa tatgtgatat acaagcgata caatgacaca 7440

tgcccagatg agcaagacta ccagattcga atggccaagt cttcgtataa gcctggacgg 7500

tttggtggga aacgcataca gcaggctatc ttatctatca aagtgtcaac atccttaggc 7560

gaagacccgg tactgactgt accgcccaac acagtcacac tcatgggggc cgaaggcaga 7620

attctcacag tagggacatc ccatttcttg tatcagcgag ggtcatcata cttctctccc 7680

gcgttattat atcctatgac agtcagcgac aaaacagcca ctcttcatag tccttataca 7740

ttcaatgcct tcactcggcc aggtagtatc ccttgccagg cttcagcaag atgccccaac 7800

tcgtgtgtta ctggagtcta tacagatcca tatcccctaa tcttctatag aaaccacacc 7860

ttgcgagggg tattcgggac aatgcttgat ggtgaacaag caagacttaa ccctgcgtct 7920

gcagtattcg atagcacatc ccgcagtcgc ataactcgag tgagttcaag cagcatcaaa 7980

gcagcataca caacatcaac ttgttttaaa gtggtcaaga ccaataagac ctattgtctc 8040

agcattgctg aaatatctaa tactctcttc ggagaattca gaatcgtccc gttactagtt 8100

gagatcctca aagatgacgg ggttagagaa gccaggtctg gctagttgag tcaactatga 8160

aagagttgga aagatggcat tgtatcacct atcttctgcg acatcaagaa tcaaaccgaa 8220

tgccggcgcg tgctcgaatt ccatgtcgcc agttgaccac aatcagccag tgctcatgcg 8280

atcagattaa gccttgtcaa tagtctcttg attaagaaaa aatgtaagtg gcaatgagat 8340

acaaggcaaa acagctcacg gtaaataata cgggtaggac atggcgagct ccggtcctga 8400

aagggcagag catcagatta tcctaccaga gtcacacctg tcttcaccat tggtcaagca 8460

caaactactc tattattgga aattaactgg gctaccgctt cctgatgaat gtgacttcga 8520

ccacctcatt ctcagccgac aatggaaaaa aatacttgaa tcggcctctc ctgatactga 8580

gagaatgata aaactcggaa gggcagtaca ccaaactctt aaccacaatt ccagaataac 8640

cggagtactc caccccaggt gtttagaaga actggctaat attgaggtcc ctgattcaac 8700

caacaaattt cggaagattg agaagaagat ccaaattcac aacacgagat atggagaact 8760

gttcacaagg ctgtgtacgc atatagagaa gaaactgctg gggtcatctt ggtctaacaa 8820

tgtcccccgg tcagaggagt tcagcagcat tcgtacggat ccggcattct ggtttcactc 8880

aaaatggtcc acagccaagt ttgcatggct ccatataaaa cagatccaga ggcatctgat 8940

tgtggcagct aggacaaggt ctgcggccaa caaattggtg atgctaaccc ataaggtagg 9000

ccaagtcttt gtcactcctg aacttgttgt tgtgacgcat acgaatgaga acaagttcac 9060

atgtcttacc caggaacttg tattgatgta tgcagatatg atggagggca gagatatggt 9120

caacataata tcaaccacgg cggtgcatct cagaagctta tcagagaaaa ttgatgacat 9180

tttgcggtta atagacgctc tggcaaaaga cttgggtaat caagtctacg atgttgtatc 9240

actaatggag ggatttgcat acggagctgt ccagctactc gagccgtcag gtacatttgc 9300

gggagatttc ttcgcattca acctgcagga gcttaaagac attctaattg gcctcctccc 9360

caatgatata gcagaatccg tgactcatgc aatcgctact gtattctctg gtttagaaca 9420

gaatcaagca gctgagatgt tgtgcctgtt gcgtctgtgg ggtcacccac tgcttgagtc 9480

ccgtattgca gcaaaggcag tcaggagcca aatgtgcgca ccgaaaatgg tagactttga 9540

tatgatcctt caggtactgt ctttcttcaa gggaacaatc atcaacggat acagaaagaa 9600

gaatgcaggt gtgtggccgc gagtcaaagt ggatacaata tatgggaagg tcattgggca 9660

actacatgca gattcagcag agatttcaca cgatatcatg ttgagagagt ataagagttt 9720

atctgcactt gaatttgagc catgtataga atacgaccct gtcactaacc tgagcatgtt 9780

cctaaaagac aaggcaatcg cacaccccaa cgataattgg cttgcctcgt ttaggcggaa 9840

ccttctctcc gaagaccaga agaaacatgt aaaggaagcg acttcgacta accgcctctt 9900

gatagagttt ttagagtcaa atgattttga tccatataaa gagatggaat atctgacgac 9960

ccttgagtac cttagagatg acaatgtggc agtatcatac tcgctcaaag agaaggaagt 10020

gaaagttaat ggacggatct tcgctaagct gacaaagaag ttaaggaact gtcaggtgat 10080

ggcggaaggg atcctagccg atcagattgc acctttcttt cagggaaatg gagtcattca 10140

ggatagcata tccttgacca agagtatgct agcgatgagt caactgtctt ttaacagcaa 10200

taagaaacgt atcactgact gtaaagaaag agtatgttca aaccgcaatc atgatccgaa 10260

aagcaagaac cgtcggagag ttgcaacctt cataacaact gacctgcaaa agtactgtct 10320

taattggaga tatcagacga tcaaattgtt cgctcatgcc atcaatcagt tgatgggcct 10380

acctcatttc ttcgagtgga ttcacctaag actgatggac actacgatgt tcgtaggaga 10440

ccctttcaat cctccaagtg accctactga ctgtgacctc tcaagagtcc ctaatgatga 10500

catatatatt gtcagtgcca gagggggtat cgaaggatta tgccagaagc tatggacaat 10560

gatctcaatt gctgcaatcc aacttgctgc agctagatcg cattgtcgtg ttgcctgtat 10620

ggtacagggt gataatcaag taatagcagt aacgagagag gtaagatcag atgactctcc 10680

ggagatggtg ttgacacagt tgcatcaagc cagtgataat ttcttcaagg aattaatcca 10740

tgtcaatcat ttgattggcc ataatttgaa ggatcgtgaa accatcaggt cagacacatt 10800

cttcatatac agcaaacgaa tcttcaaaga tggagcaatc ctcagtcaag tcctcaaaaa 10860

ttcatctaaa ttagtgctag tgtcaggtga tctcagtgaa aacaccgtaa tgtcctgtgc 10920

caacattgcc tctactgtag cacggctatg cgagaacggg cttcccaaag acttctgtta 10980

ctatttaaac tatataatga gttgtgtgca gacatacttt gactctgagt tctccatcac 11040

caacaattcg caccccgatc ttaatcagtc gtggattgag gacatctctt ttgtgcactc 11100

atatgttctg actcctgccc aattaggggg actgagtaac cttcaatact caaggctcta 11160

cactagaaat atcggtgacc cggggactac tgcttttgca gagatcaagc gactagaagc 11220

agtgggacta ctgagtccta acattaggac taatatctta actaggccgc ctgggaatgg 11280

agattgggcc agtctgtgca acgacccata ctctttcaat tttgagactg ttgcaagccc 11340

aaacattgtt cttaagaaac atacgcaaag agtcctattt gaaacttgtt caaatccctt 11400

attgtctgga gtgcacacag aggataatga ggcagaagag aaggcattgg ctgaattctt 11460
gcttaatcaa gaggtgattc atccccgcgt tgcgcatgcc atcatggagg caagctctgt 11520
aggtaggaga aagcaaattc aagggcttgt tgacacaaca aacactgtaa ttaagattgc 11580
gcttactagg aggccattag gcatcaagag gctgatgcgg atagtcaatt attctagcat 11640
gcatgcaatg ctgtttagag acgatgtttt ttcctctagt agatccaacc accccttagt 11700
ctcttctaat atgtgttctc tgacactggc agactatgca cggaatagaa gctggtcacc 11760
tttgacggga ggcaggaaaa tactgggtgt atctaatcct gatacgatag aactcgtaga 11820
gggtgagatt cttagtgtaa gcggagggtg tacaagatgt gacagcggag atgaacaatt 11880
tacttggttc catcttccaa gcaatataga attgaccgat gacaccagca agaatcctcc 11940
gatgagggta ccatatctcg ggtcaaagac acaggagagg agagctgcct cacttgcgaa 12000
aatagctcat atgtcgccac atgtgaaggc tgccctaagg gcatcatccg tgttgatctg 12060
ggcttatggg gataatgaag taaattggac tgctgctctt acgattgcaa aatctcggtg 12120
taatgtaaac ttagagtatc ttcggttact gtccccttta cccacggctg ggaatcttca 12180
acatagacta gatgatggta taactcagat gacattcacc cctgcatctc tctacagggt 12240
gtcaccttac attcacatat ccaatgattc tcaaaggctg ttcactgaag aaggagtcaa 12300
agaggggaat gtggtttacc aacagatcat gctcttgggt ttatctctaa tcgaatcgat 12360
ctttccaatg acaacaacca gaacatatga tgagatcaca ctgcacctac atagtaaatt 12420
tagttgctgt atcagggaag cacctgttgc ggttcctttc gagctacttg gggtggcacc 12480
ggaactgagg acagtgacct caaataagtt tatgtatgat cctagccctg tatcggaggg 12540
agactttgcg agacttgact tagctatctt caagagttat gagcttaatc tggagtcata 12600
tcccacgata gagctaatga acattctttc aatatccagc gggaagttga ttggccagtc 12660
tgtggtttct tatgatgaag atacctccat aaagaatgat gccataatag tgtatgacaa 12720
tacccgaaat tggatcagtg aagctcagaa ttcagatgtg gtccgcctat ttgaatatgc 12780
agcacttgaa gtgctcctcg actgttctta ccaactctat tacctgagag taagagacct 12840
agacaatatt gtcttatata tgggtgattt atacaagaat atgccaggaa ttctactttc 12900
caacattgca gctacaatat ctcatcctgt cattcattca aggttacatg cagtgggcct 12960
ggtcaaccat gacggatcac accaacttgc agatacggat tttatcgaaa tgtctgcaaa 13020
actgttagta tcttgcaccc gacgtgtgat ctccggctta tattcaggaa ataagtatga 13080
tctgctgttc ccatctgtct tagatgataa cctgaatgag aagatgcttc agctgatatc 13140
ccggttatgc tgtctgtaca cggtactctt tgctacaaca agagaaatcc cgaaaataag 13200
aggcttaact gcagaagaga aatgttcaat actcactgag tatttactgt cggatgctgt 13260
gaaaccatta cttagccccg atcaagtgag ctctatcatg tctcctaaca taattacatt 13320

cccagctaat ctgtactaca tgtctcggaa gagcctcaat ttgatcaggg aaagggagga 13380

cagggatact atcctggcgt tgttgttccc ccaagagcca ttattagagt tcccttctgt 13440

gcaagatatt ggtgctcgag tgaaagatcc attcacccga caacctgcgg catttttgca 13500

agagttagat ttgagtgctc cagcaaggta tgacgcattc acacttagtc agattcatcc 13560

tgaactcaca tctccaaatc cggaggaaga ctacttagta cgatacttgt tcagagggat 13620

agggactgca tcttcctctt ggtataaggc atcccatctc ctttctgtac ccgaggtaag 13680

atgtgcaaga cacgggaact ccttatactt ggctgaagga agcggagcca tcatgagtct 13740

tcttgaactg catgtaccac atgaaactat ctattacaat acgctctttt caaatgagat 13800

gaaccccccg caacgacatt tcgggccgac cccaactcag tttttgaatt cggttgttta 13860

taggaatcta caggcggagg taacatgcaa ggatggattt gtccaagagt tccgtccatt 13920

atggagagaa aatacagagg aaagtgacct gacctcagat aaagcagtgg ggtatattac 13980

atctgcagta ccctacagat ctgtatcatt gctgcattgt gacattgaaa ttcctccagg 14040

gtccaatcaa agcttactag atcaactagc tatcaattta tctctgattg ccatgcattc 14100

tgtaagggag ggcggggtag taatcatcaa agtgttgtat gcaatgggat actactttca 14160

tctactcatg aacttgtttg ctccgtgttc cacaaaagga tatattctct ctaatggtta 14220

tgcatgtcga ggggatatgg agtgttacct ggtatttgtc atgggttacc tgggcgggcc 14280

tacatttgta catgaggtgg tgaggatggc aaaaactctg gtgcagcggc acggtacgct 14340

tttgtctaaa tcagatgaga tcacactgac caggttattc acctcacagc ggcagcgtgt 14400

gacagacatc ctatccagtc ctttaccaag attaataaag tacttgagga agaatattga 14460

cactgcgctg attgaagccg ggggacagcc cgtccgtcca ttctgtgcgg agagtctggt 14520

gagcacgcta gcgaacataa ctcagataac ccagatcatc gctagtcaca ttgacacagt 14580

catccggtct gtgatatata tggaagctga gggtgatctc gctgacacag tatttctatt 14640

taccccttac aatctctcta ctgacgggaa aaagaggaca tcacttaaac agtgcacgag 14700

acagatccta gaggttacaa tactaggtct tagagtcgaa aatctcaata aaataggcga 14760

tataatcagc ctagtgctta aaggcatgat ctccatggag gaccttatcc cactaaggac 14820

atacttgaag catagtacct gccctaaata tttgaaggct gtcctaggta ttaccaaact 14880

caaagaaatg tttacagaca cttctgtact gtacttgact cgtgctcaac aaaaattcta 14940

catgaaaact ataggcaatg cagtcaaagg atattacagt aactgtgact cctaacgaaa 15000

atcacatatt aataggctcc ttttttggcc aattgtattc ttgttgattt aattatatta 15060

tgttagaaaa aagttgaact ctgactcctt aggactcgaa ttcgaactca aataaatgtc 15120

tttaaaaaag gttgcgcaca attattcttg agtgtagtct cgtcattcac caaatctttg 15180

tttggt                                                            15186

<210> 21
<211> 1782
<212> DNA
<213> Newcastle disease virus

<220>
<223> NDV F (unmodified) protein (nucleotide sequence)

<400> 21
acgggtagaa gactctggat cccggttggc gccctccagg tgcaggatgg gctccagacc      60

ttttaccaag aacccagcac ctatgatgct gactatccgg gtcgcgctgg tattgagttg     120

catctgtccg gcaaactcca ttgatggcag gccttttgca gctgcaggaa ttgtggttac     180

aggagacaaa gcagtcaaca tatacacctc atcccagaca ggatcaatca tagttaagct     240

cctcccgaat ctgcccaagg ataaggaggc atgtgcgaaa gcccccttgg atgcatacaa      300

caggacattg accactttgc tcacccccct tggtgactct atccgtagga tacaagagtc     360

tgtgactaca tctggagggg ggagacaggg gcgccttata ggcgccatta ttggcggtgt     420

ggctcttggg gttgcaactg ccgcacaaat aacagcggcc gcagctctga tacaagccaa     480

acaaaatgct gccaacatcc tccgacttaa agagagcatt gccgcaacca atgaggctgt     540

gcatgaggtc actgacggat tatcccaact agcagtggca gttgggaaga tgcagcagtt     600

tgttaatgac caatttaata aaacagctca ggaattagac tgcataaaaa ttgcacagca     660

agttggtgta gagctcaacc tgtacctaac cgaattgact acagtattcg gaccacaaat     720

cacttcacct gccttaaaca agctgactat tcaggcactt tacaatctag ctggtgggaa     780

tatggattac ttattgacta agttaggtat agggaacaat caactcagct cattaatcgg     840

tagcggctta atcaccggta accctattct atacgactca cagactcaac tcttgggtat     900

acaggtaact ctaccttcag tcgggaacct aaataatatg cgtgccacct acttggaaac     960

cttatccgta agcacaacca ggggatttgc ctcggcactt gtcccaaaag tggtgacaca    1020

ggtcggttct gtgatagaag aacttgacac ctcatactgt atagaaactg acttagattt    1080

atattgtaca agaatagtaa cgttccctat gtcccctggt atttactcct gcttgagcgg    1140

caatacatcg gcctgtatgt actcaaagac cgaaggcgca cttactacac catatatgac    1200

tatcaaaggc tcagtcatcg ctaactgcaa gatgacaaca tgtagatgtg taaacccccc    1260

gggtatcata tcgcaaaact atggagaagc cgtgtctcta atagataaac aatcatgcaa    1320

tgttttatcc ttaggcggga taactttaag gctcagtggg gaattcgatg taacttatca    1380

gaagaatatc tcaatacaag attctcaagt aataataaca ggcaatcttg atatctcaac    1440

tgagcttggg aatgtcaaca actcgatcag taatgctttg aataagttag aggaaagcaa    1500

cagaaaacta gacaaagtca atgtcaaact gaccagcaca tctgctctca ttacctatat 1560

cgttttgact atcatatctc ttgtttttgg tatacttagc ctgattctag catgctacct 1620

aatgtacaag caaaaggcgc aacaaaagac cttattatgg cttgggaata ataccctaga 1680

tcagatgaga gccactacaa aaatgtgaac acagatgagg aacgaaggtt tccctaatag 1740

taatttgtgt gaaagttctg gtagtctgtc agttcggaga gt 1782

<210> 22
<211> 553
<212> PRT
<213> Newcastle disease virus

<220>
<223> NDV F (unmodified) protein (amino acid sequence)

<400> 22
Met Gly Ser Arg Pro Phe Thr Lys Asn Pro Ala Pro Met Met Leu Thr
1               5                   10                  15
Ile Arg Val Ala Leu Val Leu Ser Cys Ile Cys Pro Ala Asn Ser Ile
            20                  25                  30
Asp Gly Arg Pro Phe Ala Ala Ala Gly Ile Val Val Thr Gly Asp Lys
        35                  40                  45
Ala Val Asn Ile Tyr Thr Ser Ser Gln Thr Gly Ser Ile Ile Val Lys
    50                  55                  60
Leu Leu Pro Asn Leu Pro Lys Asp Lys Glu Ala Cys Ala Lys Ala Pro
65                  70                  75                  80
Leu Asp Ala Tyr Asn Arg Thr Leu Thr Thr Leu Leu Thr Pro Leu Gly
                85                  90                  95
Asp Ser Ile Arg Arg Ile Gln Glu Ser Val Thr Thr Ser Gly Gly Gly
            100                 105                 110
Arg Gln Gly Arg Leu Ile Gly Ala Ile Ile Gly Gly Val Ala Leu Gly
        115                 120                 125
Val Ala Thr Ala Ala Gln Ile Thr Ala Ala Ala Ala Leu Ile Gln Ala
    130                 135                 140
Lys Gln Asn Ala Ala Asn Ile Leu Arg Leu Lys Glu Ser Ile Ala Ala
145                 150                 155                 160
Thr Asn Glu Ala Val His Glu Val Thr Asp Gly Leu Ser Gln Leu Ala
                165                 170                 175
Val Ala Val Gly Lys Met Gln Gln Phe Val Asn Asp Gln Phe Asn Lys
            180                 185                 190
Thr Ala Gln Glu Leu Asp Cys Ile Lys Ile Ala Gln Gln Val Gly Val
        195                 200                 205
Glu Leu Asn Leu Tyr Leu Thr Glu Leu Thr Thr Val Phe Gly Pro Gln
    210                 215                 220
Ile Thr Ser Pro Ala Leu Asn Lys Leu Thr Ile Gln Ala Leu Tyr Asn
225                 230                 235                 240
Leu Ala Gly Gly Asn Met Asp Tyr Leu Leu Thr Lys Leu Gly Ile Gly
                245                 250                 255
Asn Asn Gln Leu Ser Ser Leu Ile Gly Ser Gly Leu Ile Thr Gly Asn
            260                 265                 270
Pro Ile Leu Tyr Asp Ser Gln Thr Gln Leu Leu Gly Ile Gln Val Thr
        275                 280                 285
Leu Pro Ser Val Gly Asn Leu Asn Asn Met Arg Ala Thr Tyr Leu Glu
    290                 295                 300
Thr Leu Ser Val Ser Thr Thr Arg Gly Phe Ala Ser Ala Leu Val Pro
305                 310                 315                 320
Lys Val Val Thr Gln Val Gly Ser Val Ile Glu Glu Leu Asp Thr Ser
                325                 330                 335

Tyr Cys Ile Glu Thr Asp Leu Asp Leu Tyr Cys Thr Arg Ile Val Thr
            340                 345                 350
Phe Pro Met Ser Pro Gly Ile Tyr Ser Cys Leu Ser Gly Asn Thr Ser
        355                 360                 365
Ala Cys Met Tyr Ser Lys Thr Glu Gly Ala Leu Thr Thr Pro Tyr Met
    370                 375                 380
Thr Ile Lys Gly Ser Val Ile Ala Asn Cys Lys Met Thr Thr Cys Arg
385                 390                 395                 400
Cys Val Asn Pro Pro Gly Ile Ile Ser Gln Asn Tyr Gly Glu Ala Val
                405                 410                 415
Ser Leu Ile Asp Lys Gln Ser Cys Asn Val Leu Ser Leu Gly Gly Ile
            420                 425                 430
Thr Leu Arg Leu Ser Gly Glu Phe Asp Val Thr Tyr Gln Lys Asn Ile
        435                 440                 445
Ser Ile Gln Asp Ser Gln Val Ile Ile Thr Gly Asn Leu Asp Ile Ser
    450                 455                 460
Thr Glu Leu Gly Asn Val Asn Asn Ser Ile Ser Asn Ala Leu Asn Lys
465                 470                 475                 480
Leu Glu Glu Ser Asn Arg Lys Leu Asp Lys Val Asn Val Lys Leu Thr
                485                 490                 495
Ser Thr Ser Ala Leu Ile Thr Tyr Ile Val Leu Thr Ile Ile Ser Leu
            500                 505                 510
Val Phe Gly Ile Leu Ser Leu Ile Leu Ala Cys Tyr Leu Met Tyr Lys
        515                 520                 525
Gln Lys Ala Gln Gln Lys Thr Leu Leu Trp Leu Gly Asn Asn Thr Leu
    530                 535                 540
Asp Gln Met Arg Ala Thr Thr Lys Met
545                 550

<210> 23
<211> 1662
<212> DNA
<213> Artificial Sequence

<220>
<223> NDV F3aa-modified fusion protein with L289A (nucleotide sequence)

<400> 23
atgggctcca gaccttctac caagaaccca gcacctatga tgctgactat ccgggtcgcg      60

ctggtactga gttgcatctg cccggcaaac tccattgatg gcaggcctct tgcagctgca     120

ggaattgtgg ttacaggaga caaagcagtc aacatataca cctcatccca gacaggatca     180

atcatagtta agctcctccc gaatctgccc aaggataagg aggcatgtgc gaaagccccc     240

ttggatgcat acaacaggac attgaccact ttgctcaccc cccttggtga ctctatccgt     300

aggatacaag agtctgtgac tacatctgga gggcggagac agaggcgctt tataggcgcc     360

attattggcg gtgtggctct tggggttgca actgccgcac aaataacagc ggccgcagct     420

ctgatacaag ccaaacaaaa tgctgccaac atcctccgac ttaaagagag cattgccgca     480

accaatgagg ctgtgcatga ggtcactgac ggattatcgc aactagcagt ggcagttggg     540

aagatgcagc agtttgttaa tgaccaattt aataaaacag ctcaggaatt agactgcatc     600

aaaattgcac agcaagttgg tgtagagctc aacctgtacc taaccgaatt gactacagta     660

ttcggaccac aaatcacttc acctgcctta aacaagctga ctattcaggc actttacaat     720

ctagctggtg ggaatatgga ttacttattg actaagttag gtatagggaa caatcaactc 780

agctcattaa tcggtagcgg cttaatcacc ggtaacccta ttctatacga ctcacagact 840

caactcttgg gtatacaggt aactgcacct tcagtcggga acctaaataa tatgcgtgcc 900

acctacttgg aaaccttatc cgtaagcaca accaggggat ttgcctcggc acttgtccca 960

aaagtggtga cacaggtcgg ttctgtgata gaagaacttg acacctcata ctgtatagaa 1020

actgacttag atttatattg tacaagaata gtaacgttcc ctatgtcccc tggtatttac 1080

tcctgcttga gcggcaatac atcggcctgt atgtactcaa agaccgaagg cgcacttact 1140

acaccatata tgactatcaa aggctcagtc atcgctaact gcaagatgac aacatgtaga 1200

tgtgtaaacc ccccgggtat catatcgcaa aactatggag aagccgtgtc tctaatagat 1260

aaacaatcat gcaatgtttt atccttaggc gggataactt taaggctcag tggggaattc 1320

gatgtaactt atcagaagaa tatctcaata caagattctc aagtaataat aacaggcaat 1380

cttgatatct caactgagct tgggaatgtc aacaactcga tcagtaatgc tttgaataag 1440

ttagaggaaa gcaacagaaa actagacaaa gtcaatgtca aactgaccag cacatctgct 1500

ctcattacct atatcgtttt gactatcata tctcttgttt ttggtatact tagcctgatt 1560

ctagcatgct acctaatgta caagcaaaag gcgcaacaaa agaccttatt atggcttggg 1620

aataataccc tagatcagat gagagccact acaaaaatgt ga 1662

<210> 24
<211> 1662
<212> DNA
<213> Artificial Sequence

<220>
<223> NDV F3aa-modifed fusion protein (nucleotide sequence)

<400> 24
atgggctcca gaccttctac caagaaccca gcacctatga tgctgactat ccgggtcgcg 60

ctggtactga gttgcatctg cccggcaaac tccattgatg gcaggcctct tgcagctgca 120

ggaattgtgg ttacaggaga caaagcagtc aacatataca cctcatccca gacaggatca 180

atcatagtta agctcctccc gaatctgccc aaggataagg aggcatgtgc gaaagccccc 240

ttggatgcat acaacaggac attgaccact ttgctcaccc cccttggtga ctctatccgt 300

aggatacaag agtctgtgac tacatctgga gggcggagac agaggcgctt tataggcgcc 360

attattggcg gtgtggctct tggggttgca actgccgcac aaataacagc ggccgcagct 420

ctgatacaag ccaaacaaaa tgctgccaac atcctccgac ttaaagagag cattgccgca 480

accaatgagg ctgtgcatga ggtcactgac ggattatcgc aactagcagt ggcagttggg 540

aagatgcagc agtttgttaa tgaccaattt aataaaacag ctcaggaatt agactgcatc 600

aaaattgcac agcaagttgg tgtagagctc aacctgtacc taaccgaatt gactacagta 660

ttcggaccac aaatcacttc acctgcctta aacaagctga ctattcaggc actttacaat 720

ctagctggtg ggaatatgga ttacttattg actaagttag gtatagggaa caatcaactc 780

agctcattaa tcggtagcgg cttaatcacc ggtaacccta ttctatacga ctcacagact 840

caactcttgg gtatacaggt aactctacct tcagtcggga acctaaataa tatgcgtgcc 900

acctacttgg aaaccttatc cgtaagcaca accaggggat ttgcctcggc acttgtccca 960

aaagtggtga cacaggtcgg ttctgtgata gaagaacttg acacctcata ctgtatagaa 1020

actgacttag atttatattg tacaagaata gtaacgttcc ctatgtcccc tggtatttac 1080

tcctgcttga gcggcaatac atcggcctgt atgtactcaa agaccgaagg cgcacttact 1140

acaccatata tgactatcaa aggctcagtc atcgctaact gcaagatgac aacatgtaga 1200

tgtgtaaacc ccccgggtat catatcgcaa aactatggag aagccgtgtc tctaatagat 1260

aaacaatcat gcaatgtttt atccttaggc gggataactt taaggctcag tggggaattc 1320

gatgtaactt atcagaagaa tatctcaata caagattctc aagtaataat aacaggcaat 1380

cttgatatct caactgagct tgggaatgtc aacaactcga tcagtaatgc tttgaataag 1440

ttagaggaaa gcaacagaaa actagacaaa gtcaatgtca aactgaccag cacatctgct 1500

ctcattacct atatcgtttt gactatcata tctcttgttt ttggtatact tagcctgatt 1560

ctagcatgct acctaatgta caagcaaaag gcgcaacaaa agaccttatt atggcttggg 1620

aataataccc tagatcagat gagagccact acaaaaatgt ga 1662

<210> 25
<211> 553
<212> PRT
<213> Artificial Sequence

<220>
<223> NDV F3aa-modified fusion protein (amino acid sequence)

<400> 25
Met Gly Ser Arg Pro Phe Thr Lys Asn Pro Ala Pro Met Met Leu Thr
1               5                   10                  15
Ile Arg Val Ala Leu Val Leu Ser Cys Ile Cys Pro Ala Asn Ser Ile
            20                  25                  30
Asp Gly Arg Pro Phe Ala Ala Ala Gly Ile Val Val Thr Gly Asp Lys
        35                  40                  45
Ala Val Asn Ile Tyr Thr Ser Ser Gln Thr Gly Ser Ile Ile Val Lys
    50                  55                  60
Leu Leu Pro Asn Leu Pro Lys Asp Lys Glu Ala Cys Ala Lys Ala Pro
65                  70                  75                  80
Leu Asp Ala Tyr Asn Arg Thr Leu Thr Thr Leu Leu Thr Pro Leu Gly
                85                  90                  95
Asp Ser Ile Arg Arg Ile Gln Glu Ser Val Thr Thr Ser Gly Gly Arg
            100                 105                 110
Arg Gln Arg Arg Phe Ile Gly Ala Ile Ile Gly Gly Val Ala Leu Gly
        115                 120                 125

Val Ala Thr Ala Ala Gln Ile Thr Ala Ala Ala Ala Leu Ile Gln Ala
    130                 135                 140
Lys Gln Asn Ala Ala Asn Ile Leu Arg Leu Lys Glu Ser Ile Ala Ala
145                 150                 155                 160
Thr Asn Glu Ala Val His Glu Val Thr Asp Gly Leu Ser Gln Leu Ala
                165                 170                 175
Val Ala Val Gly Lys Met Gln Gln Phe Val Asn Asp Gln Phe Asn Lys
            180                 185                 190
Thr Ala Gln Glu Leu Asp Cys Ile Lys Ile Ala Gln Gln Val Gly Val
        195                 200                 205
Glu Leu Asn Leu Tyr Leu Thr Glu Leu Thr Thr Val Phe Gly Pro Gln
    210                 215                 220
Ile Thr Ser Pro Ala Leu Asn Lys Leu Thr Ile Gln Ala Leu Tyr Asn
225                 230                 235                 240
Leu Ala Gly Gly Asn Met Asp Tyr Leu Leu Thr Lys Leu Gly Ile Gly
                245                 250                 255
Asn Asn Gln Leu Ser Ser Leu Ile Gly Ser Gly Leu Ile Thr Gly Asn
            260                 265                 270
Pro Ile Leu Tyr Asp Ser Gln Thr Gln Leu Leu Gly Ile Gln Val Thr
        275                 280                 285
Leu Pro Ser Val Gly Asn Leu Asn Asn Met Arg Ala Thr Tyr Leu Glu
    290                 295                 300
Thr Leu Ser Val Ser Thr Thr Arg Gly Phe Ala Ser Ala Leu Val Pro
305                 310                 315                 320
Lys Val Val Thr Gln Val Gly Ser Val Ile Glu Glu Leu Asp Thr Ser
                325                 330                 335
Tyr Cys Ile Glu Thr Asp Leu Asp Leu Tyr Cys Thr Arg Ile Val Thr
            340                 345                 350
Phe Pro Met Ser Pro Gly Ile Tyr Ser Cys Leu Ser Gly Asn Thr Ser
        355                 360                 365
Ala Cys Met Tyr Ser Lys Thr Glu Gly Ala Leu Thr Thr Pro Tyr Met
    370                 375                 380
Thr Ile Lys Gly Ser Val Ile Ala Asn Cys Lys Met Thr Thr Cys Arg
385                 390                 395                 400
Cys Val Asn Pro Pro Gly Ile Ile Ser Gln Asn Tyr Gly Glu Ala Val
                405                 410                 415
Ser Leu Ile Asp Lys Gln Ser Cys Asn Val Leu Ser Leu Gly Gly Ile
            420                 425                 430
Thr Leu Arg Leu Ser Gly Glu Phe Asp Val Thr Tyr Gln Lys Asn Ile
        435                 440                 445
Ser Ile Gln Asp Ser Gln Val Ile Ile Thr Gly Asn Leu Asp Ile Ser
    450                 455                 460
Thr Glu Leu Gly Asn Val Asn Asn Ser Ile Ser Asn Ala Leu Asn Lys
465                 470                 475                 480
Leu Glu Glu Ser Asn Arg Lys Leu Asp Lys Val Asn Val Lys Leu Thr
                485                 490                 495
Ser Thr Ser Ala Leu Ile Thr Tyr Ile Val Leu Thr Ile Ile Ser Leu
            500                 505                 510
Val Phe Gly Ile Leu Ser Leu Ile Leu Ala Cys Tyr Leu Met Tyr Lys
        515                 520                 525
Gln Lys Ala Gln Gln Lys Thr Leu Leu Trp Leu Gly Asn Asn Thr Leu
    530                 535                 540
Asp Gln Met Arg Ala Thr Thr Lys Met
545                 550

<210> 26
<211> 510
<212> DNA
<213> Homo sapiens

<220>
<223> Human soluble PD1 (sPD1)

<400> 26
atgcagatcc cacaggcgcc ctggccagtc gtctgggcgg tgctacaact gggctggcgg 60

ccaggatggt tcttagactc cccagacagg ccctggaacc cccccacctt ctccccagcc 120

ctgctcgtgg tgaccgaagg ggacaacgcc accttcacct gcagcttctc caacacatcg 180

gagagcttcg tgctaaactg gtaccgcatg agccccagca accagacgga caagctggcc 240

gccttccccg aggaccgcag ccagcccggc caggactgcc gcttccgtgt cacacaactg 300

cccaacgggc gtgacttcca catgagcgtg gtcagggccc ggcgcaatga cagcggcacc 360

tacctctgtg gggccatctc cctggccccc aaggcgcaga tcaaagagag cctgcgggca 420

gagctcaggg tgacagagag aagggcagaa gtgcccacag cccaccccag cccctcaccc 480

aggccagccg gccagttcca aaccctggtg 510

## Description

### FIELD OF THE INVENTION

The present invention relates to a recombinant oncolytic virus. The present invention further relates to a nucleic acid encoding a recombinant oncolytic virus. The present invention also relates to a vector comprising a nucleic acid encoding a recombinant oncolytic virus. Furthermore, the present invention relates to a pharmaceutical composition comprising a recombinant oncolytic virus.

### BACKGROUND OF THE INVENTION

Despite many decades of intensive research, cancer remains a major worldwide health concern and imposes a heavy societal burden, both epidemiologically and financially. Cancer immunotherapy is rapidly transforming the face of medical oncology as an exciting paradigm shift in treating cancer by exploiting the immune system of the patient as the basis for the therapy. Oncolytic viruses (OVs) have recently earned their place as an exciting subset of cancer immunotherapeutics. OVs offer a novel, multi-mechanistic approach through the onset of direct tumor cell oncolysis, modulation of the tumor microenvironment, and the potential to stimulate adaptive immune responses directed against tumor cells. Therefore, OV approaches, particularly in the context of rationally designed combination regimens with other immunotherapeutics, are currently under intense investigation.

Cancer immunotherapy encompasses a wide range of approaches that have the common aim of modulating a patient's immune system to recognize and reject invading tumor cells. Vaccination approaches and adoptive immune cell therapies, such as chimeric antigen receptor (CAR) T cells, rely on the identification of suitable tumor-associated antigens (TAAs) and neoantigens to target. Due to intratumoral heterogeneity and the process of cancer immune-editing, strategies which target a single antigen can result in selection for tumor cells which do not express the targeted antigen and can escape the therapy. Furthermore, since each tumor has its own distinct gene signature, targeted therapies often require expensive and time-consuming screening of tumor biopsies and subsequent production of personalized treatments.

Immune checkpoints are compensatory controls that function to prevent constant activation of an immune inflammatory response to foreign antigens by suppressing T cell activation and creating a state of T-cell "exhaustion". Tumor cells utilize these immune checkpoints to create an immune-suppressive microenvironment, in which they can escape immune clearance and continue to invade the host. Immune checkpoints have increasingly been considered as promising targets for cancer immunotherapy. Blockade using antibodies against key checkpoint molecules have entered the market for limited cancer indications, but only seem to be effective in small subsets of cancer patients, generally in tumors which are highly inflamed and have a high mutational load. Furthermore, the high doses of these antibodies required for systemic application can result in severe immune related toxicities.

Oncolytic viruses (OVs) offer an elegant multimodal approach to cancer therapy through their ability to cause direct tumor cell lysis, while stimulating immune responses directed against the tumor. Nevertheless, the potential of OVs as monotherapeutics is limited by the relatively rapid onset of antiviral immune responses and insufficient adoptive immune stimulation to provide systemic tumor clearance and protection from relapse. Over the last decade, significant progress has been made in the development of enhanced OV therapies, and several vectors have entered clinical trials. To date, a single OV has received Federal Drug Administration (FDA) and European Medical Association (EMA) approval for use as a clinical agent. This virus is a recombinant herpes simplex virus I (HSV-I) vector and is currently approved only for one single indication which is non-resectable melanoma. Furthermore, US 10604574 B2 relates to oncolytic viral delivery of therapeutic polypeptides. However, clinical trial results are often disappointing due to inadequate tumor responses to most OV therapies in immune competent hosts.

The present inventors have previously engineered a hybrid oncolytic virus technology (WO 2017/198779), which combines the beneficial features of oncolytic vesicular stomatitis virus (VSV) with those of Newcastle disease virus (NDV), while eliminating the safety concerns of each.

Recently, a strategy to combine OVs with immune checkpoint therapies has emerged. The rationale is that the local virus infection within the tumor can transform the "cold" tumor microenvironment to an inflamed, "hot" environment marked by high levels of cytokines and immune cell infiltration, thereby sensitizing the tumor to the effects of immune checkpoint blockade. It has been postulated that tumor therapy with oncolytic viruses leads to upregulation of the ligand, PD-L1, of the co-inhibitory T-cell receptor, programmed cell death protein 1 (PD-1), within the tumor, and that blockade of the interaction between PD-1 and PD-L1 enhances the oncolytic immunotherapeutic effect. A strategy utilizing an oncolytic myxoma virus expressing sPD-1 has been published [1].

However, various hurdles must be addressed in order to fully exploit the potential of the combination of OV with an immune checkpoint therapy. Firstly, a challenge is that conventional immune checkpoint modulating drugs consist of antibodies, which need to be administered systemically and at high concentrations, often leading to intolerable side effects. Secondly, the prohibitively high costs of immunotherapeutics substantially limit the feasibility of combining two individual drug products in this class. Thirdly, in order to optimize the synergistic effects, a challenge is to choose an efficient oncolytic virus, which combines a potent tumor debulking effect with immunogenic cell death and inflammation.

Thus, there is a need in the art for improved means and methods for oncolytic virotherapy as well as for improved oncolytic viruses. The aim of the present invention is to provide a superior cancer therapeutic that combines optimal viral-mediated oncolysis with potent immune-mediated effects, in the absence of systemic toxicity.

### SUMMARY OF THE INVENTION

In the following, the elements of the invention will be described. These elements are listed with specific embodiments, however, it should be understood that they may be combined in any manner and in any number to create additional embodiments. The variously described examples and preferred embodiments should not be construed to limit the present invention to only the explicitly described embodiments. This description should be understood to support and encompass embodiments which combine two or more of the explicitly described embodiments or which combine the one or more of the explicitly described embodiments with any number of the disclosed and/or preferred elements. Furthermore, any permutations and combinations of all described elements in this application should be considered disclosed by the description of the present application unless the context indicates otherwise.

In a first aspect, the present invention relates to a recombinant oncolytic virus,
comprising a vesicular stomatitis virus (VSV),
wherein the glycoprotein (G protein) of VSV is deleted, and which comprises a modified fusion protein (F protein) of Newcastle disease virus (NDV), and the hemagglutinin neuraminidase (HN) protein of NDV,
further comprising soluble PD-1 (sPD-1).

In one embodiment, said recombinant virus further comprises a Fc domain or a fragment thereof, preferably a Fc domain of human IgG1, IgG2, IgG3, and/or IgG4, or a fragment thereof.

In one embodiment, said Fc domain or fragment thereof is fused to said sPD-1.

In one embodiment, said sPD-1 is a high affinity sPD-1 (HA-sPD-1), preferably having an affinity to its ligands PD-L1 and PD-L2 which is at least 2-fold higher, such as 45- and 30-fold higher, respectively, than an affinity of a wildtype sPD-1 to said ligands, and/or has an affinity with a K_{d} of < 3.2 µM with regard to PD-L1 and/or < 0.1 µM with regard to PD-L2.

In one embodiment, said sPD-1 comprises a mutation at a position selected from 132 and 41 of SEQ ID NO. 2, preferably a mutation selected from A132L, L41I, and L41V.

In one embodiment, said sPD-1 comprises or consists of a sequence having SEQ ID NO. 2, and optionally further comprises a mutation at a position selected from 132 and 41 of SEQ ID NO. 2, preferably a mutation selected from A132L, L41I, and L41V.

In one embodiment, said sPD-1 is HA-sPD-1-A132L having a sequence of SEQ ID NO. 3.

In one embodiment, the modified fusion protein (F protein) of NDV is the F3aa-modified F protein having a sequence of SEQ ID NO. 25,
and/or comprises at least one amino acid substitution in the protease cleavage site, preferably in position L289 of SEQ ID NO. 25, more preferably L289A;
and/or the modified fusion protein (F protein) of NDV is the F3aa-modified F protein with an amino acid substitution L289A having SEQ ID NO. 4;
and/or the G protein of VSV is replaced by the modified fusion protein having a sequence of SEQ ID NO. 4 and HN protein of NDV having a sequence of SEQ ID NO. 5.

In a further aspect, the present invention relates to a nucleic acid encoding a recombinant oncolytic virus as defined above.

In one embodiment, said nucleic acid comprises a nucleic acid encoding a Fc domain or fragment thereof, preferably having a sequence of SEQ ID NO. 7, which is fused to a nucleic acid encoding said sPD-1, preferably having a sequence of SEQ ID NO. 6, wherein, preferably, a fusion product thereof has a nucleic acid sequence of SEQ ID NO. 8.

In this aspect, said recombinant oncolytic virus, said Fc domain, said fragment, and said sPD-1 are as defined above.

In a further aspect, the present invention relates to a vector comprising a nucleic acid as defined above, preferably having a sequence of SEQ ID NO. 9,
optionally further comprising any of
- a reporter gene,
   such as any of HSV1-sr39TK, sodium iodide symporter (NIS), somatostatin receptor 2 (SSTR2), luciferase (Firefly or Renilla), green fluorescence protein (GFP), lacZ, and tyrosinase;
- a gene to be delivered to a tumor cell and/or tumor tissue, such as any of
   an immune stimulating gene, e.g. IFN-α, IFN-β, or granulocyte macrophage colony-stimulating factor (GM-CSF), IL-12, or IL-15;
   an immune checkpoint inhibitory antibody, e.g. PD-1, PD-1-L, CTLA-4, LAG-3, or B7-H3; and/or
   a tumor associated antigen (TAA) for vaccination (specific for the tumor being targeted);
- or combinations thereof.

In a further aspect, the present invention relates to a nucleic acid as defined above or a vector as defined above, comprising or consisting of the nucleotide sequence of SEQ ID NO. 9 or 15,
or a nucleotide sequence having at least 60%, preferably at least 70% or 80%, more preferably at least 90% or 95% sequence identity to the nucleotide sequence of SEQ ID NO. 9 or 15,
or comprising or consisting of the nucleotide of SEQ ID NO. 16,
or a nucleotide sequence having at least 60%, preferably at least 70% or 80%, more preferably at least 90% or 95% sequence identity to the nucleotide sequence of SEQ ID NO. 16.

In a preferred embodiment, the nucleic acid, as defined above, or the vector, as defined above, comprise or consist of the nucleotide sequence of SEQ ID NO. 9 or 15, or a nucleotide sequence having at least 60%, preferably at least 70% or 80%, more preferably at least 90% or 95% sequence identity to the nucleotide sequence of SEQ ID NO. 9 or 15.

In a further aspect, the present invention relates to a pharmaceutical composition, comprising
(i) the recombinant oncolytic virus as defined above, the nucleic acid as defined above, or the vector as defined above; and
(ii) optionally, pharmaceutically acceptable carrier(s) and/or excipient(s),
(iii) optionally, a further drug, such as
   a chemotherapeutic agent,
   a radiotherapeutic agent,
   a tumor vaccine,
   an immune checkpoint inhibitor, such as anti-CTLA4,
   an adoptive cell therapy system, such as T cells or dendritic cells,
   a cell carrier system,
   a small molecule inhibitor,
   an embolization agent,
   a shielding polymer.

In one embodiment, the pharmaceutical composition as defined above is formulated for any of systemic delivery, tumor injection, intravenous administration, and intra-arterial administration,
and/or for an intradermal, subcutaneous, intramuscular, intravenous, intraosseous, intraperitoneal, intrathecal, epidural, intracardiac, intraarticular, intracavernous, intracerebral, intracerebroventricular, or intravitreal injection.

In a further aspect, the present invention relates to a recombinant oncolytic virus, as defined above, a nucleic acid, as defined above, a vector, as defined above, or a pharmaceutical composition, as defined above, for use in medicine.

In a further aspect, the present invention relates to a recombinant oncolytic virus, as defined above, a nucleic acid, as defined above, a vector, as defined above, or a pharmaceutical composition, as defined above, for use in the prevention and/or treatment of cancer, such as in the prevention and/or treatment of a cancer selected from hepatocellular carcinoma, pancreatic cancer, and melanoma.

In one embodiment, the recombinant oncolytic virus for use, as defined above, the nucleic acid for use, as defined above, the vector for use, as defined above, or the pharmaceutical composition for use, as defined above, is used in combination with other therapies, such as
cell carrier systems, e.g. T cells, dendritic cells, NK cells, mesenchymal stem cells, immunotherapies, e.g. tumor vaccines or immune checkpoint inhibitors, adoptive cell therapies, such as with T cells or dendritic cells, and/or
standard tumor therapies, e.g. radiofrequency ablation, chemotherapy, embolization, small molecule inhibitors.

In a further aspect, the present invention relates to a recombinant oncolytic virus, as defined above, a nucleic acid, as defined above, a vector, as defined above, or a pharmaceutical composition, as defined above, for use in the diagnosis of cancer, such as in the diagnosis of a cancer selected from hepatocellular carcinoma, pancreatic cancer, and melanoma.

In a further aspect, the present invention relates to a use of the recombinant oncolytic virus, as defined above, or the nucleic acid, as defined above, or the vector, as defined above, or the pharmaceutical composition, as defined above, as gene delivery tool, (noninvasive) imaging of virus biodistribution, and/or for tumor detection. In one embodiment, such use is an in-vitro-use.

In a further aspect, the present invention relates to a method of diagnosis, prevention and/or treatment of a cancer comprising administering to a subject in need thereof a therapeutically amount of the recombinant oncolytic virus, as defined above, or the nucleic acid, as defined above, or the vector, as defined above, or the pharmaceutical composition, as defined above.

In one embodiment, said administering is systemic, intravenous, intra-arterial, via injection into tumor, and/or via intradermal, subcutaneous, intramuscular, intravenous, intraosseous, intraperitoneal, intrathecal, epidural, intracardiac, intraarticular, intracavernous, intracerebral, intracerebroventricular and intravitreal injection(s).

In this aspect, said cancer and said administering are as defined above.

In a further aspect, the present invention relates to a use of a recombinant oncolytic virus, as defined above, or a nucleic acid, as defined above, or a vector, as defined above, or a pharmaceutical composition, as defined above, for the manufacture of a medicament for diagnosing, preventing and/or treating cancer. In this aspect, said cancer and said diagnosing, preventing and/or treating are as defined above.

### DETAILED DESCRIPTION

The enhanced virus of the present invention, which is a modified VSV-NDV, offers several beneficial features over conventional oncolytic viruses, such as rapid and efficient tumor cell oncolysis generated by the induction of cell-cell fusion reactions, while maintaining an exceptional safety profile. Not only do the infected cells fuse with neighboring tumor cells, thereby killing them, but they also set off a series of events which causes the patient's immune system to launch an attack on remaining uninfected tumor cells, creating an inflammatory tumor microenvironment. Since VSV-NDV can replicate well in all tumor cells, it has far-reaching therapeutic effects in a multitude of tumor indications.

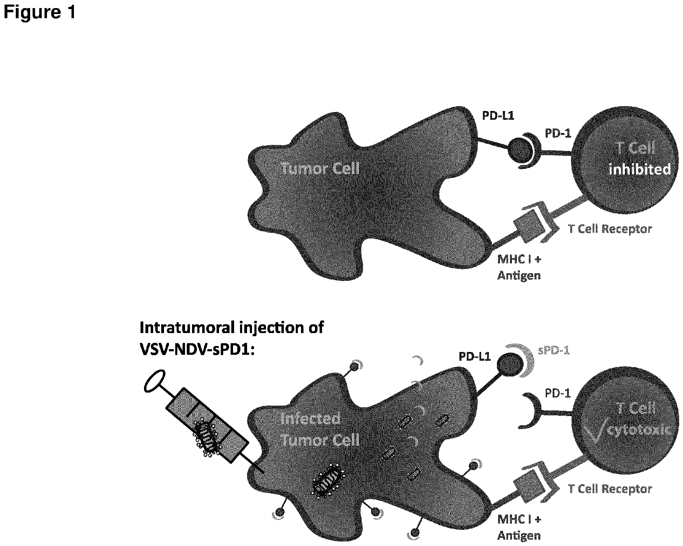

The present invention provides a potent oncolytic virus with an effective immune checkpoint blocking molecule within a single therapeutic agent. The present inventors use the safe and immune-stimulating rVSV-NDV virus as a vector to express a soluble PD-1 (sPD-1) molecule, containing the signaling domain and the soluble extracellular domain of human PD-1. The recombinant vector infects tumor cells, where it replicates and evokes secretion of sPD-1. Then, sPD-1 attaches to its ligands, PD-L1 and PD-L2, on the surface of surrounding tumor cells and immune cells (i.e. dendritic cells), and thereby acts as a decoy to compete for T cell binding to their inhibitory ligands, thereby allowing the T cells to remain active, without the need for antibodies (Figure 1).

Compared to traditional immune checkpoint blockade (ICB) therapy via antibodies, the invention is inventive in that it combines oncolytic virus therapy and ICB into a single therapeutic agent, which acts to simultaneously debulk the tumor, via direct oncolytic effects, while alleviating the immune suppression within the microenvironment. Furthermore, this approach allows self-amplification of therapeutic agents through virus replication and local release of the PD-1 directly at the tumor site, which is a major safety benefit compared to an antibody approach.

The present invention has the advantages that, firstly, the vector contains a highly fusogenic NDV fusion (F) protein, such as NDV/F3aa(L289A), which induces a potent immunogenic cell death and optimally synergizes with immune checkpoint blockade; secondly, the sPD-1 used in the construct is preferably a "*high* a*ffinity*" version having increased affinity compared to wild type sPD-1. Such high affinity version is, for example, produced via an introduction of a single alanine to leucine substitution at amino acid 132 (A132L) to result in a 45- and 30-fold higher affinity binding to its ligands, PD-L1 and PD-L2 [2], respectively, or is, for example, a high affinity consensus variant encoding an isoleucine or valine at position 41 having a 40,000-fold higher affinity for PD-L1 than the wildtype [3]. Thirdly, HA-sPD-1 is fused to the Fc domain of human IgG for enhanced stability. In one embodiment, the Fc region directly follows the sPD-1 sequence in frame, wherein the stop codon of sPD-1 is removed in order to generate a sPD-1-Fc fusion protein with a Fc domain. In one embodiment, a high affinity sPD-1 (HA-sPD-1) has an increased affinity compared to wild type sPD-1 and/or compared to wild type PD-1, such as a twofold increase in the affinity to the respective ligand(s). In one embodiment, a "high affinity" version of sPD-1 has an affinity which is at least twice as high as the affinity of wild type sPD-1, such as an 5 times higher affinity, 10 times higher affinity, 30 times higher affinity, or 45 times higher affinity. In one embodiment, a wild type human PD-1 has a K_{d} of 6.36 µM and 0.19 µM with regard to PD-L1 and PD-L2, respectively. In one embodiment, a HA-sPD-1 (high affinity sPD-1) has an affinity with a K_{d} of < 3.2 µM with regard to PD-L1 and < 0.1 µM with regard to PD-L2, preferably an affinity with a K_{d} of < 0.5 µM with regard to PD-L1 and < 0.01 µM with regard to PD-L2, such as 0.14 µM and 0.0065 µM with regard to PD-L1 and PD-L2, respectively.

The binding of the wildtype sPD-1 to its ligand is likely too weak to support its application as a therapeutically effective approach. The advantage of the present invention is that the high affinity version of sPD-1 used in a vector of the present invention is more effective and thus has higher efficiency in cancer treatment.

The vector of the present invention offers several advantages over antibody-based ICB therapies. For example, it offers a local release of PD-1 specifically at the tumor site, which has the potential to avoid the severe side effects that are attributed to the systemic delivery of these antibodies. It also provides a synergistic mechanism of action through tumor debulking, induction of antitumor immune responses, and therapeutic modulation of immune suppression within the tumor microenvironment. Furthermore, the rVSV-NDV-sPD-1 drug product can potentially be produced for a fraction of the price of PD-1 antibodies, and it is more cost-effective than administration of combination therapy of an OV in addition to a separate PD-1 antibody. Furthermore, the vector of the present invention is advantageously safe for human use.

The present invention has further advantages, such as advantages over an oncolytic myxoma virus expressing a sPD-1. Firstly, the rVSV-NDV vector is an optimal oncolytic virus platform, as it is extremely safe and highly effective in tumor killing through direct oncolytic effects and induction of highly immunogenic cell death, leading to abscopal effects. These superior therapeutic effects are attributed to the VSV glycoprotein substitution with the envelope proteins of NDV (comprising the fusion (F) and hemagglutinin neuraminidase (HN) proteins), and more specifically, through the use of the modified hyperfusogenic fusion (F) protein that is engineered into the virus construct. In one embodiment, the modified fusion protein (F protein) of NDV is the F3aa-modified F protein e.g. having a sequence of SEQ ID NO. 25, optionally further comprising at least one amino acid substitution in the protease cleavage site, preferably in position L289 of SEQ ID NO. 25, more preferably L289A. In one embodiment, using the L289A-modified version of the NDV fusion (F) protein, namely NDV/F3aa(L289A), such as a protein having an amino acid sequence of SEQ ID NO. 4, provides increased hyperfusogenicity. These properties are the basis for the rVSV-NDV being an ideal OV vector for combination with ICB. Moreover, the rVSV-NDV-sPD-1 vector utilizes sPD-1, preferably a high affinity version of human sPD-1, which offers a stronger interaction of the sPD-1 with its ligands and thus a greater potential for therapeutic effects. Finally, a high affinity sPD-1 is fused with a Fc domain of human IgG, which allows for greater stability for an improved pharmacokinetic profile in vivo. In one embodiment, a nucleic acid and/or a vector of the invention comprise a nucleotide sequence having SEQ ID NO. 9 or 15, wherein SEQ ID NO. 9 and 15 both encode VSV-NDV-HAsPD1-Fc, wherein SEQ ID NO. 9 further comprises non-coding regions.

Concentrations, amounts, and other numerical data may be expressed or presented herein in a range format. It is to be understood that such a range format is used merely for convenience and brevity and thus should be interpreted flexibly to include not only the numerical values explicitly recited as the limits of the range, but also to include all the individual numerical values or sub-ranges encompassed within that range as if each numerical value and sub-range is explicitly recited. As an illustration, a numerical range of "20 to 100 nucleotides" should be interpreted to include not only the explicitly recited values of 20 to 100, but also include individual values and sub-ranges within the indicated range. Thus, included in this numerical range are individual values such as 20, 21, 22, 23, 24, 25, 26, 27, 28, 29 ,... 97, 98, 100 and sub-ranges such as from 25 to 35, from 20 to 40, from 25 to 50, etc. This same principle applies to ranges reciting only one numerical value, such as "at least 25 nucleotides". Furthermore, such an interpretation should apply regardless of the breadth of the range or the characteristics being described.

The term "VSV", as used herein, relates to Vesicular stomatitis virus (VSV) which is a negative-strand RNA virus of the Rhabdovirus family. VSV vectors are very attractive oncolytic agents due to their inherent tumor specificity and rapid replication cycle, which results in high intratumoral titers and subsequent tumor cell lysis. The genome of VSV is a single molecule of negative-sense RNA that encodes five major proteins: glycoprotein (G), large polymerase protein (L), phosphoprotein (P), matrix protein (M) and nucleoprotein (N). The total genome is about 11,000 nucleotides. The VSV G protein enables viral entry. It mediates viral attachment to an LDL receptor (LDLR) or an LDLR family member present on the host cell. Following binding the VSV-LDLR complex is rapidly endocytosed. It then mediates fusion of the viral envelope with the endosomal membrane. VSV enters the cell through partially clathrin-coated vesicles; virus-containing vesicles contain more clathrin and clathrin adaptor than conventional vesicles. Virus-containing vesicles recruit components of the actin machinery for their interaction, thus inducing its own uptake. Replication occurs in the cytoplasm. The VSV L protein is encoded by half the genome, and combines with the phosphoprotein to catalyze replication of the mRNA. The VSV M protein is encoded by an mRNA that is 831 nucleotides long and translates to a 229 amino acid-protein. The predicted M protein sequence does not contain any long hydrophobic or nonpolar domains that might promote membrane association. The protein is rich in basic amino acids and contains a highly basic amino terminal domain. The term "rVSV" relates to recombinant vesicular stomatitis virus (VSV).

| | |
|---|---|
| VSV Indiana complete genome | SEQ ID NO. 17 |
| NCBI GenBank accession No. J02428.1 | |
| VSV Indiana G protein | SEQ ID NO. 18 |
| See GenBank accession No. X03633.1 for nucleotide and amino acid sequence. | |

The term "NDV", as used herein, relates to Newcastle disease virus (NDV) which is an avian virus of the Paramyxovirus family. Members of this family have a single stranded linear RNA. The total genome is about 16,000 nucleotides. Replication of the virus takes place in the cytoplasm of the host cell. It is a negative-stand RNA virus and has been developed as an oncolytic virus, due to its innate ability to replicate and cause lysis in tumor cells, while leaving healthy cells unharmed. Phase I-II clinical trials suggest that there is minimal toxicity related to the therapy. A major benefit of NDV as an oncolytic agent is that the viral envelope, which is comprised of a hemagglutinin-neuraminidase (HN) and fusion (F) protein, mediates not only virus attachment and fusion to the target cell, but it causes fusion of infected cells to their neighboring uninfected cells, providing a potent mechanism for viral spread and tumor cell killing. Furthermore, new evidence indicates that the syncytia formation caused by cell-cell fusion results in a multimodal cell death response, which can synergize with the direct oncolytic effect of the virus for a potent mechanism of tumor destruction.

Two proteins of Newcastle disease virus are inserted in the envelope. They are the haemagglutinin/neuraminidase protein (HN) and the fusion protein (F). These two proteins are important in determining the virulence of the virus and how the virus infects host cells. The haemagglutinin/neuraminidase protein has two sections that are of interest: (1) The haemagglutinin section, which is an attachment protein and binds to receptors on the outside of the membrane of host cells including red blood cells. (2) The neuraminidase section is the active site of an enzyme that aids in the release of the virus from the membrane of host cells. The activity of this enzyme affects the time taken for the virus to elute from red blood cells.

The fusion protein F fuses the virus envelope to the membrane of the host cell. This allows penetration of the host cell by the viral genome. In order for fusion to occur, the shape of the native fusion protein must be changed. This change happens when a host cell protease cleaves the protein at a specific cleavage site. After this has happened, the fusion protein is activated and can then fuse to the membrane of the cell. The sequence of the amino acids around the cleavage site determines the range of proteases that can activate cleavage of the protein. This sequence therefore determines the virulence. NDV F protein is responsible for viral fusion with the cell membrane and for viral spread from cell to cell via formation of syncytia. The presence of a multibasic cleavage site within the F protein allows for protein cleavage and activation by a broad range of proteases and is a determinant of virulence in velogenic viral strains. The inventors have previously demonstrated that a single amino acid substitution from leucine to alanine at amino acid 289 (L289A) in the F3aa-modified fusion protein results in substantially greater syncytial formation and tumor necrosis than the virus bearing only the F3aa mutation, without any additional toxicity [4]. The fusogenic and oncolytic activity of the rNDV/F3aa strain can be further enhanced by a point mutation in the F protein at residue 289 from leucine to alanine, generating rNDV/F3aa (L289A). In an orthotopic immunocompetent liver tumor rat model, administration of the mutant virus via hepatic arterial infusion resulted in significant syncytia formation and necrosis, which translated to a significant 20% prolongation of survival over treatment with the original rNDV/F3aa virus [4].

| | |
|---|---|
| NDV Hitchner B1 complete genome | SEQ ID NO. 20 |
| GenBank accession No. AF375823 | |
| NDV HN protein | SEQ ID NOs. 5 and 19 |
| See GenBank accession No. AF375823 and NCBI Gene ID acid sequence. 912270 for nucleic acid and amino | |
| NDV F protein | SEQ ID NOs. 21 and 22 |
| See GenBank accession No. AF375823 and NCBI Gene ID acid sequence. 912271 for nucleic acid and amino | |
| NDV F3aa-modified fusion protein | SEQ ID NOs. 24 and 25 |
| NDV F3aa-modified fusion protein with L289A | SEQ ID NOs. 23 and 4 |

As discussed above, the present invention provides recombinant oncolytic VSV viruses, wherein the glycoprotein protein of VSV is pseudotyped, and which further comprise a soluble PD-1. The concept of exchanging the glycoprotein ("pseudotyping") of a virus with that of a heterologous virus has previously been demonstrated as an effective means of altering virus tropism. Using this approach, the viral backbone is kept intact, and therefore, virus replication in susceptible cells should be minimally effected.

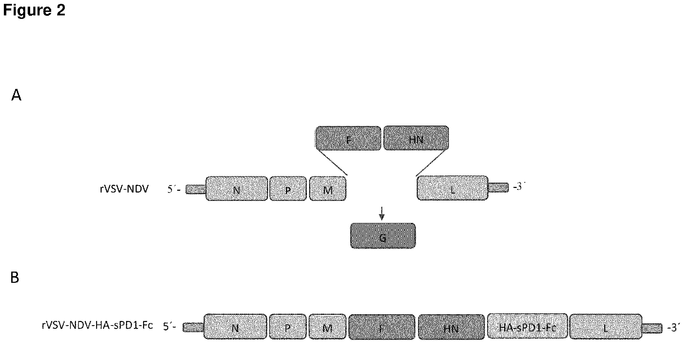

In one embodiment, the G protein of VSV is replaced by the modified fusion protein, preferably the modified fusion (F) protein having an amino acid substitution at position L289 e.g. L289A, and HN protein of NDV. In one embodiment, the recombinant oncolytic virus furthermore comprises the remaining proteins of VSV, namely the large polymerase protein (L), phosphoprotein (P), matrix protein (M) and nucleoprotein (N). For example, the endogenous glycoprotein of VSV can be deleted from a plasmid encoding the full-length VSV genome. The NDV glycoprotein, comprising a modified fusion protein (NDV/F(L289A)) and hemagglutinin-neuraminidase (NDV/HN), can be inserted as discrete transcription units between the VSV matrix (M) and large polymerase (L) genes (see Figure 2A and WO 2017/198779). In one embodiment, a modified fusion protein (F protein) of NDV is a F3aa-modified F protein, optionally comprising at least one amino acid substitution in the protease cleavage site, preferably in position L289, such as L289A.

In an embodiment of the vector, preferably recombinant VSV (rVSV) vector, of the present invention, the modified fusion protein (F protein) of NDV comprises or consists of the amino acid sequence of SEQ ID NO. 25 [= aa sequence of F3aa protein] or SEQ ID NO. 4 [= aa sequence of F3aa protein/L289A],
or an amino acid sequence having at least 60%, or preferably at least 70% or 80% or 90% or 95% sequence identity to the amino acid sequence of SEQ ID NOs. 25 or 4,
and/or wherein the modified fusion protein (F protein) of NDV is encoded by a nucleotide sequence of SEQ ID NO. 24 [=nucleotide sequence of F3aa protein] or SEQ ID NO. 23 [= nucleotide sequence of F3aa protein/L289A],
or a nucleotide sequence having at least 60%, or preferably at least 70% or 80% or 90% or 95% sequence identity to the nucleotide sequence of SEQ ID NOs. 24 or 23.

In an embodiment of the vector, preferably rVSV vector, of the present invention, the HN protein of NDV comprises or consists of the amino acid sequence of SEQ ID NO. 5,
or an amino acid sequence having at least 60%, or preferably at least 70% or 80% or 90% or 95% sequence identity to the amino acid sequence of SEQ ID NO. 5,
and/or the HN protein of NDV is encoded by a nucleotide sequence of SEQ ID NO. 19 or a nucleotide sequence having at least 60%, or preferably at least 70% or 80% or 90% or 95% sequence identity to the nucleotide sequence of SEQ ID NO. 19.

The present invention comprises *nucleic acids* encoding the oncolytic viruses of the present invention. The present invention further comprises *vectors* comprising the nucleic acids of the present invention.

In one embodiment, the vector of the present invention further comprises any of
- a reporter gene,
   such as any of HSV1-sr39TK, the sodium iodide symporter (NIS), somatostatin receptor 2 (SSTR2), luciferase (Firefly or Renilla), green fluorescence protein (GFP), lacZ, and tyrosinase,
- a gene to be delivered to a tumor cell and/or tumor tissue, such as any of
   an immune stimulating gene, e.g. IFN-α, IFN-β, or granulocyte macrophage colony-stimulating factor (GM-CSF), IL-12, or IL-15;
   an immune checkpoint inhibitory antibody, such as PD-1, PD-L1, CTLA-4, LAG-3, or B7-H3; and
   a tumor associated antigen (TAA) for vaccination (specific for the tumor being targeted);
- and combinations thereof.

The present invention provides the recombinant oncolytic viruses, the nucleic acids of the present invention, the vectors of the present invention, and/or the pharmaceutical composition of the present invention for use in medicine. The present invention further provides the recombinant oncolytic viruses, the nucleic acids of the present invention, the vectors of the present invention, and/or the pharmaceutical composition of the present invention for use in the diagnosis, prevention and/or treatment of cancer. The present invention also provides the recombinant oncolytic viruses, the nucleic acids of the present invention, the vectors of the present invention, and/or the pharmaceutical composition of the present invention for use in oncolytic therapy, particularly oncolytic virotherapy. The term "oncolytic virotherapy", as used herein, refers to therapy of cancer by administration of oncolytic viruses, nucleic acids encoding them or respective vectors to induce tumor regression. In one embodiment, the recombinant oncolytic viruses of the present invention, the nucleic acids of the present invention, the vectors of the present invention, and/or the pharmaceutical composition of the present invention are provided for use in combination with other therapies, such as
cell carrier systems, e.g. T cells, dendritic cells, NK cells, mesenchymal stem cells, immunotherapies, e.g. tumor vaccines or immune checkpoint inhibitors, adoptive cell therapies, e.g. with T cells or dendritic cells, and/or
standard tumor therapies, e.g. radiofrequency ablation, chemotherapy, embolization, small molecule inhibitors.

In one embodiment, an administration of a recombinant oncolytic virus, a nucleic acid, a vector, and/or a pharmaceutical composition to a patient in need thereof is systemic, intravenous, intra-arterial, via injection into tumor, and/or via intradermal, subcutaneous, intramuscular, intravenous, intraosseous, intraperitoneal, intrathecal, epidural, intracardiac, intraarticular, intracavernous, intracerebral, intracerebroventricular, and intravitreal injection(s). In a preferred embodiment, the pharmaceutical composition comprising the recombinant oncolytic virus of the present invention is formulated as a liquid composition for administration, for example intravenous or intratumoral administration. Such composition is administered to a patient in need thereof at a suitable interval, such as once in a week for a period of 1 to 15 weeks.

The present invention further provides a method of diagnosis, prevention and/or treatment of cancer comprising the step of administering to a subject in need thereof a therapeutically effective amount of the recombinant oncolytic virus, the nucleic acid or the vector of the present invention or the pharmaceutical composition of the present invention. A therapeutically effective amount of a recombinant oncolytic virus, nucleic acid or vector of the present invention is the amount which results in the desired therapeutic result, in particular tumor regression.

The recombinant viruses, nucleic acids, vectors or their pharmaceutical composition(s) are preferably administered in multiple cycles over a period of time, such as for several days up to several weeks. In one embodiment, a pharmaceutical composition of the present invention comprises carrier(s) and/or excipient(s), such as a physiological buffer, and/or a polymer and/or lipid for improved stability and tumor transduction efficiency.

The vector of the present invention comprises, inserted into the VSV G-deleted vector, a modified hyperfusogenic F protein together with a NDV HN attachment protein. By creating a hybrid of these two potent oncolytic vectors, the positive features of each virus were merged, while simultaneously eliminating the safety concerns of each. The resulting vector has the VSV backbone and, therefore, maintains the rapid replication cycle of wildtype VSV. Furthermore, due to the incorporation of the HN and hyperfusogenic F proteins of NDV, the recombinant virus induces enhanced syncytia formation, allowing for efficient intratumoral spread of the virus and a potent mechanism of tumor cell death and induction of antitumor immune responses. Using this strategy, the benefit of a fusogenic virus can be achieved without the environmental threat associated with NDV. Furthermore, since the endogenous VSV glycoprotein has been deleted, there is no neurotoxicity associated with the vector. Finally, since NDV attaches to target cells via sialic acid residues, which are upregulated on tumor cells, the present invention achieves additional transductional tumor targeting with the pseudotyped vector. The specific virus modification allows to provide a highly potent virus due to the substitution of the VSV envelope protein with that of NDV, in addition to the virus being safer. A mutated version of the NDV F protein has been introduced for further improving the efficacy of the resulting recombinant virus, without negatively impacting safety. In additional thereto, the vector further comprises the highly affine soluble PD-1 which allows for a highly efficient inhibition of an immune checkpoint.

The benefit of the vector having a glycoprotein exchange is three-fold:
1. The neurotropism associated with the endogenous VSV glycoprotein can be averted by the deletion of the VSV envelope and the introduction of the non-neurotropic NDV envelope proteins;
2. Tumor cells can be targeted via upregulation of sialic acid residues, which are the natural receptor for NDV; and
3. Viral spread and tumor cell killing can be significantly enhanced via introduction of the highly fusogenic mutant version of the NDV F protein.

The virus of the present invention offers improved safety and enhanced efficacy over other vectors. Furthermore, using the rVSV-NDV vector backbone is advantageous due to its hyperfusogenic feature, lack of pre-existing immunity in the general population, and no expected attenuation compared to VSV or NDV. Furthermore, an advantage of fusion of sPD-1 with Fc is, firstly, that the stability of sPD-1 is increased and, secondly, that the Fc domain enables the complex to interact with Fc receptors on immune cells, which further contributes to the immune-therapy. Preferably, the recombinant oncolytic virus of the present invention is a recombinant oncolytic vesicular stomatitis virus (VSV). In one embodiment, the recombinant oncolytic virus of the present invention comprises an oncolytic virus, as defined in WO 2017/198779, further comprising soluble PD-1 (sPD-1), preferably high affinity sPD-1 (HA-sPD-1), such as a high affinity sPD-1 variant having SEQ ID NO. 3, optionally further comprising a Fc domain, wherein said optional Fc domain is preferably fused to said sPD-1. In one embodiment, the recombinant oncolytic virus of the present invention comprises an oncolytic virus, as defined in WO 2017/198779, further comprising sPD-1, preferably a high affinity sPD-1 (HA-sPD-1), such as a high affinity sPD-1 variant having SEQ ID NO. 3, further comprising a Fc domain which is fused to said sPD-1. In one embodiment, the virus of the present invention comprises a N protein, P protein, M protein, and L protein of VSV, a modified F protein and HN protein of NDV, and a HA-sPD-1-Fc fusion protein and/or genes encoding such proteins.

The term "Fc domain", as used herein, relates to the fragment crystallizable region of an antibody which is the tail region of an antibody that interacts with cell surface receptors, such as Fc receptors and some proteins of the complement system. In one embodiment, a Fc domain is any of a human IgG1-Fc domain, IgG2-Fc domain, IgG3-Fc domain, and IgG4-Fc domain. In one embodiment, when referring to a "Fc domain" and/or to a "Fc domain or fragment thereof", the term also comprises biologically functional fragments of a Fc domain, such as a CH3 fragment of a FC domain, i.e. the term comprises entire Fc domains and fragments thereof, such as CH3 fragments. A fragment of a Fc domain is typically a biologically functional fragment of a Fc domain, i.e. the fragment has the capacity of interacting with cell surface receptors, such as Fc receptors, and/or of interacting with components of the complement system, for example a CH3 fragment.

In one embodiment, a patient's response to infection with the recombinant virus of the present invention induces a highly inflamed tumor microenvironment, thereby sensitizing the tumor to immune checkpoint inhibition. In one embodiment, the terms "subject" and "patient" are used interchangeably, and preferably relate to a mammalian patient, more preferably a human patient.

In one embodiment, the term "soluble PD-1" relates to a soluble form of programmed cell death protein 1 (PD-1). PD-1 is an immune checkpoint, namely a protein that has a role in regulating the immune system's response by down-regulating the immune system and promoting self-tolerance by suppressing T cell inflammatory activity. PD-1 has two ligands, namely PD-L1 and PD-L2, which are members of the B7 family. PD-1 typically has a sequence as shown in SEQ ID NO. 1. PD-1 is a protein on the surface of cells and soluble PD-1 is a soluble form thereof which can be secreted from cells. Soluble PD-1 typically has a sequence as shown in SEQ ID NO. 2. In one embodiment, soluble PD-1 is a high affinity sPD-1 having a sequence of SEQ ID NO. 3. In one embodiment, a virus of the present invention and/or a pharmaceutical composition of the present invention comprise(s) sPD-1 having at least 60 %, preferably 80 %, more preferably 95 % or more such as 99 %, sequence identity to an amino acid sequence having any of SEQ ID NO. 1-3, preferably having SEQ ID NO. 3. In one embodiment, a virus of the present invention, a nucleic acid of the present invention, a vector of the present invention, and/or a pharmaceutical composition of the present invention comprise(s) sPD-1 having at least 60 %, preferably 80 %, more preferably 95 % or more such as 99 %, sequence identity to a nucleic acid encoding an amino acid sequence having any of SEQ ID NO. 1-3, preferably having SEQ ID NO. 3, such as a nucleic acid having a sequence of SEQ ID NO. 6. Due to the codon redundancy of the genetic code, when referring to a nucleic acid, alternative nucleic acid sequences are also encompassed which encode the same amino acid sequence but which comprise alternative codons.

The term "fused" and/or "fusion protein", as used herein in the context of sPD-1 and Fc, relates to the fusion of proteins, such as sPD-1 and Fc, created through the joining of two or more genes that originally coded for separate proteins. Translation of such a fusion gene, for example sPD-1-Fc, results in a single or multiple polypeptides with functional properties derived from each of the original proteins. Fusion of proteins typically involves removing the stop codon from a cDNA sequence coding for the first protein, then appending the cDNA sequence of the second protein in frame through a method known to a person skilled in the art, such as ligation or overlap extension PCR. The resulting DNA sequence will then be expressed by a cell as a single protein. The protein can be engineered to include the full sequence of both original proteins, or only a portion of either. Optionally, linkers can be included between the components of a fusion protein. In one embodiment, the term "fusion product" relates to a product created by fusing two or more genes, such as a fusion gene comprising a nucleic acid encoding a Fc domain, preferably having a sequence of SEQ ID NO. 7, and comprising a nucleic acid encoding sPD-1, preferably having a sequence of SEQ ID NO. 6, and/or to a product encoded by such fused genes. In one embodiment, an exemplary fusion product of HA-sPD-1 and Fc has a nucleic acid sequence of SEQ ID NO. 8 and/or an amino acid sequence of SEQ ID NO. 13. In one embodiment, a Fc domain or fragment thereof is fused to sPD-1, e.g. HA-sPD-1, which means that a nucleic acid encoding a Fc domain or fragment thereof and a nucleic acid encoding a sPD-1, such as an HA-sPD-1, are fused. In one embodiment, when used in the context of the fusion (F) protein of NDV, the term "fusion protein" does not relate to two or more proteins being joined together, but rather, the term relates to a protein which *induces* fusion of the viral envelope with the cellular receptor.

The term "high affinity", as used herein, relates to an increased affinity of a modified protein, such as HA-sPD-1, to its binding partner, such as PD-L1, compared to the affinity of the respective wildtype protein, such as sPD-1, to the respective binding partner. In one embodiment, a "high affinity" version of sPD-1 (HA-sPD-1) has an increased affinity, such as a twofold increased affinity, to its ligands compared to wild type sPD-1. Such high affinity version is, for example, produced via an introduction of a single alanine to leucine substitution at amino acid 132 (A132L) of sPD-1 which results in a higher affinity, such as a 45- and 30-fold higher affinity binding to its ligands, PD-L1 and PD-L2, respectively, or is, for example, a high affinity consensus variant encoding an isoleucine or valine at position 41 resulting in a higher affinity, such as a 40,000-fold higher affinity for PD-L1 compared to wildtype sPD-1. In one embodiment, a high affinity sPD-1 has a K_{d} of < 3.2 µM with regard to PD-L1 and < 0.1 µM with regard to PD-L2. In one embodiment, a mutation is preferably a point mutation.
SEQ ID NO. 1 represents the amino acid sequence of human PD-1.
SEQ ID NO. 2 represents the amino acid sequence of human PD-1 (soluble).
SEQ ID NO. 3 represents the amino acid sequence of an exemplary high affinity PD-1 (soluble) which is HA-sPD-1-A132L.
SEQ ID NO. 4 represents the amino acid sequence of F3aa-modified fusion protein (F protein) of NDV having amino acid substitution L289A.
SEQ ID NO. 5 represents the amino acid sequence of the HN protein of NDV.
SEQ ID NO. 6 represents the nucleic acid sequence encoding an exemplary high affinity PD-1 (soluble) which is HA-sPD-1-A132L.
SEQ ID NO. 7 represents the nucleic acid sequence of the human IgG1-Fc; CH2 and CH3 domains of the human IgGi heavy chain and the hinge region.
SEQ ID NO. 8 represents the nucleic acid sequence of an exemplary high affinity soluble PD-1-IgG1-Fc fusion gene.
SEQ ID NO. 9 represents the nucleic acid sequence of an exemplary vector of the present invention comprising a construct VSV-NDV-HA-sPD-1-Fc.
SEQ ID NO. 10 represents the amino acid sequence of a N protein.
SEQ ID NO. 11 represents the amino acid sequence of a P protein.
SEQ ID NO. 12 represents the amino acid sequence of a M protein.
SEQ ID NO. 13 represents the amino acid sequence of a HA-sPD-1-Fc protein.
SEQ ID NO. 14 represents the amino acid sequence of a L protein.
SEQ ID NO. 15 represents the nucleic acid sequence of the construct VSV-NDV-HA-sPD-1-Fc.
SEQ ID NO. 16 represents the total nucleic acid sequence of rVSV-NDV-sPD-1, wherein said sPD-1 is normal sPD-1 without high-affinity modification, and wherein said sequence does not comprise a Fc-encoding sequence.
SEQ ID NO. 17 represents the complete genome of VSV Indiana.
SEQ ID NO. 18 represents the amino acid sequence of VSV Indiana G protein.
SEQ ID NO. 19 represents the nucleic acid sequence of the NDV HN protein.
SEQ ID NO. 20 represents the complete genome of NDV Hitchner Bi.
SEQ ID NO. 21 represents the nucleic acid sequence of the NDV F (unmodified) protein.
SEQ ID NO. 22 represents the amino acid sequence of the NDV F (unmodified) protein.
SEQ ID NO. 23 represents the nucleic acid sequence of the NDV F3aa-modified fusion protein with L289A.
SEQ ID NO. 24 represents the nucleic acid sequence of the NDV F3aa-modifed fusion protein.
SEQ ID NO. 25 represents the amino acid sequence of the NDV F3aa-modified fusion protein.
SEQ ID NO. 26 represents the nucleic acid sequence of human soluble PD-1 (sPD-1).

### BRIEF DESCRIPTION OF THE FIGURES

The present invention is now further described by reference to the following figures. All methods mentioned in the figure descriptions below were carried out as described in detail in the examples.
**Figure 1** **shows interference of the PD-1/PD-L1 interaction and a soluble PD-1.** When exhausted T cells come into contact with tumor cells expressing PD-L1, the PD-1 on the T cell engages and becomes inactivated, allowing the tumor cell to evade immune clearance. Local expression of a soluble PD-1 competes with the PD-1 expressed by the T cells for binding to its PD-L1 ligand and interferes with the interaction, enabling the T cell to remain functional and elicit its cytotoxic effector functions against the tumor cell [5].
**Figure 2** **shows the rVSV-NDV backbone (A) and the rVSV-NDV-sPD-1 construct of the present invention (B).**
   A) Recombinant pseudotyped VSV construct expressing the glycoprotein of NDV (see also WO 2017/198779 A1). The endogenous glycoprotein of VSV was deleted from a plasmid encoding the full-length VSV genome. The NDV glycoprotein, comprising a modified fusion protein (NDV/F(L289A)) and hemagglutinin-neuraminidase (NDV/HN), was inserted as discrete transcription units between the VSV matrix (M) and large polymerase (L) genes. The respective pseudotyped VSV vector was rescued using an established reverse-genetics system. B) The construct of the present invention comprises a soluble human PD-1 gene, preferably the high affinity soluble human PD-1 gene (HA-sPD-1), fused with a Fc domain of human IgG. The high affinity, soluble human PD-1 gene (HA-sPD-1), fused with the Fc domain of human IgG, was cloned as an additional transcription unit between the NDV attachment protein (HN) and the VSV large polymerase (L) genes. The resultant viral genome is shown.

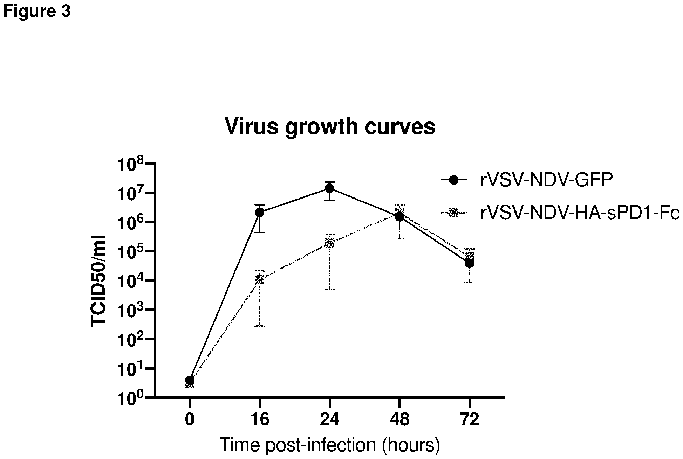

**Figure 3** **shows that rVSV-NDV-HA-sPD-1-Fc replicates with slight attenuation in mouse melanoma cells.** The mouse melanoma cell line, B16-OVA, was infected with rVSV-NDV-GFP or rVSV-NDV-HA-sPD-1-Fc at a multiplicity of infection (MOI) of 0.01. After a 1 hour infection, the cells were washed and fresh medium was added to the cells. At various time-points post-infection aliquots of the supernatant were collected for measurements of viral titers by TCID50 assay. Experiments were performed in triplicate, and data are presented as mean +/- standard error of the mean.

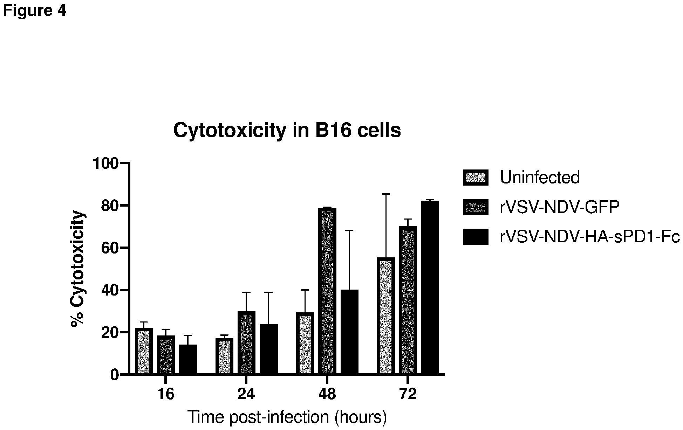

**Figure 4** **shows that rVSV-NDV-HA-sPD-1-Fc efficiently kills mouse melanoma cells.** The mouse melanoma cell line, B16-OVA, was infected with rVSV-NDV-GFP or rVSV-NDV-HA-sPD-1-Fc at a multiplicity of infection (MOI) of 0.01. After a 1 hour infection, the cells were washed and fresh medium was added to the cells. At various time-points post-infection aliquots of the supernatant were collected for LDH assay for cytotoxicity. Experiments were performed in triplicate, and data are presented as mean +/- standard error of the mean.

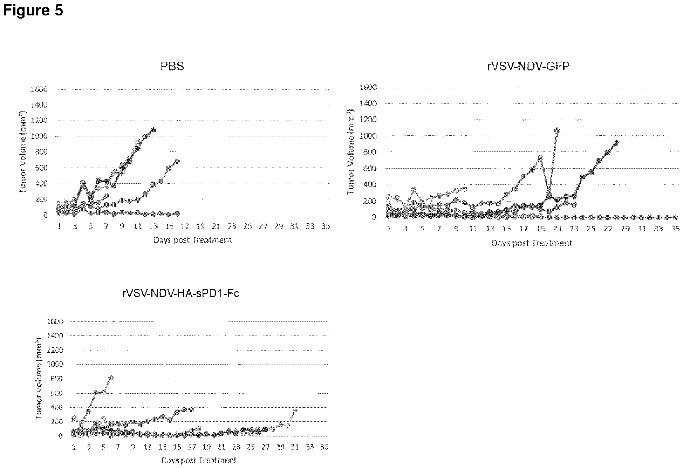

**Figure 5** **shows that rVSV-NDV-HA-sPD-1-Fc causes delayed tumor growth in immune-competent mice bearing syngeneic B16-OVA melanoma cells.** Male C57Bl/6 mice were implanted with 2.4 x 10⁵ B16-OVA cells subcutaneously in the flanks. On days 7, 10, and 13 post-tumor implantation, PBS or 10⁷ TCID50 of rVSV-NDV-GFP or rVSV-NDV-HA-sPD-1-Fc was injected into the tumor in a 50µl volume. Tumor size was monitored daily, and tumor volumes were calculated using the formula: 4/3^{∗}PI()^{∗}((L+W)/4)³. Individual tumor growth curves for each treatment are shown. Each curve represents an individual mouse.

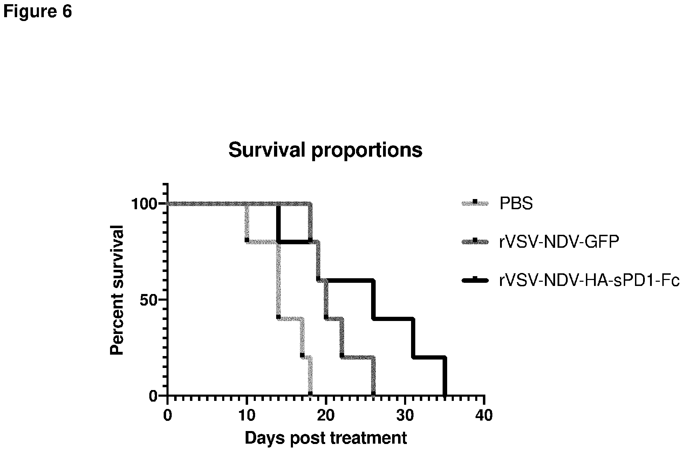

**Figure 6** **shows that intratumoral injection of rVSV-NDV-HA-sPD-1-Fc significantly prolongs survival of B16-OVA-bearing mice.** Male C57Bl/6 mice were implanted with 2.4 x 10⁵ B16-OVA cells subcutaneously in the flanks. On days 7, 10, and 13 post-tumor implantation, PBS or 10⁷ TCID50 of rVSV-NDV-GFP or rVSV-NDV-HA-sPD-1-Fc was injected into the tumor in a 50µl volume. Mice were monitored daily and euthanized when tumor diameters reached 1.5cm or if the skin ruptured due to tumor growth. Survival times post-treatment were plotted as a Kaplan-Meier survival curves and statistical significance was determined by log-rank test. The p value for rVSV-NDV-HA-sPD-1-Fc versus PBS < 0.05.

In the following, reference is made to the examples, which are given to illustrate, not to limit the present invention.

### EXAMPLES

### Example 1: Preparation of the rVSV-NDV-sPD-1 virus

In order to engineer the rVSV-NDV-sPD-1 virus, the signaling and extracellular domains of PD-1 were first amplified by RT-PCR from human PBMCs. The RT-PCR product generated served as a template for a round of overlapping PCR to introduce the A132L mutation for high affinity, which generated 2 overlapping PCR fragments containing the mutated base pairs. These fragments were annealed and subjected to a final elongation step to produce the full-length HA-sPD-1 gene. The HA-sPD-1 fragment was cloned into pFUSE-hIgG1-Fc1 in order to create a fusion gene of the HA sPD-1 with the human Fc fragment. In order to insert the HA-sPD-1-Fc construct into rVSV-NDV, the appropriate restriction sites were introduced using forward and reverse oligonucleotides and amplified by PCR. Finally, the insert was ligated into the full-length VSV-NDV genome as an additional transcription unit via the multi-cloning site between the NDV-HN and the VSV-L genes. The virus construct is depicted in Figure 2B. The corresponding infectious recombinant virus was rescued using an established method of reverse genetics.

### Example 2: Characterization of the rVSV-NDV-sPD-1 virus

Characterization of the recombinant VSV-NDV-HA-sPD-1-Fc virus was carried out in the mouse melanoma cell line B16-OVA. Growth kinetics of the virus were compared to the parental rVSV-NDV virus and revealed a slight attenuation attributed to the expression of the transgene, and a peak in replication was reached at approximately 48-hours post-infection at a multiplicity of infection (MOI) of 0.01 (Figure 3). In line with these findings, cytotoxicity assays in the same cells revealed a slight delay in tumor cell killing by infection with rVSV-NDV-HA-sPD-1-Fc, but nearly complete killing of the cell monolayer by 72-hours post-infection (Figure 4). Accordingly, the VSV-NDV-HA-sPD-1-Fc virus provides enhanced efficiency after 72 hours post-infection compared to the virus without HA-sPD-1-Fc.

### Example 3: Anticancer effect of the rVSV-NDV-sPD-1 virus in vivo

In order to determine in vivo efficacy, experiments were performed in immune-competent C57Bl/6 mice bearing subcutaneous B16-OVA tumors in their flanks. On days 7, 10, and 13 after tumor implantation, PBS or 10⁷ TCID50 of rVSV-NDV-GFP or rVSV-NDV-HA-sPD-1-Fc was injected into the tumor in a 50/µl volume. Tumors were measured daily, and tumor volumes were calculated using the following formula: V = 4/3^{∗}PI()^{∗}((L+W)/4)³. The data reveal a significant delay in tumor growth in those mice treated with rVSV-NDV-GFP, which was even more pronounced in mice receiving rVSV-NDV-HA-sPD-1-Fc, compared to PBS (Figure 5).

### Example 4: Increased survival of mice treated with rVSV-NDV-HA-sPD-1-Fc

In a survival study, mice were euthanized at humane endpoints when tumors reached a diameter of 1.5 cm or if tumor growth led to skin ruptures. Survival times with respect to the first treatment dose were plotted and revealed a significant survival prolongation of mice treated with rVSV-NDV-HA-sPD-1-Fc compared to PBS, with a median survival time of 26 versus 14 days, respectively (Figure 6). The light grey line represents survival time after treatment with PBS; medium grey represents that of rVSV-NDV-GFP treatment; black represents that of rVSV-NDV-HA-sPD-1-Fc treatment. The data demonstrate that treatment with rVSV-NDV-HA-sPD-1-Fc results in substantially delayed tumor growth, resulting in prolonged survival, compared to treatment with buffer or the parental rVSV-NDV virus.

### REFERENCES

[1] Bartee MY, Dunlap KM, Bartee E. Tumor-Localized Secretion of Soluble PD1 Enhances Oncolytic Virotherapy. Cancer Res. 2017;77(11):2952-63.
[2] Lazar-Molnar E, Scandiuzzi L, Basu I, Quinn T, Sylvestre E, Palmieri E, et al. Structure-guided development of a high-affinity human Programmed Cell Death-i: Implications for tumor immunotherapy. EBioMedicine. 2017;17:30-44.
[3] Maute RL, Gordon SR, Mayer AT, McCracken MN, Natarajan A, et al. PD-1 variants for immunotherapy and PET imaging. PNAS. Nov 2015, 112(47)E6506-E6514; DOI: 10.1073/pnas.1519623112.
[4] Altomonte, J., S. Marozin, et al. (2010). "Engineered newcastle disease virus as an improved oncolytic agent against hepatocellular carcinoma." Mol Ther 18(2):275-284.
[5] Malini Guha, The Pharmaceutical Journal (2014).

The features of the present invention disclosed in the specification, the claims, and/or in the accompanying figures may, both separately and in any combination thereof, be material for realizing the invention in various forms thereof.

## Claims

1. A recombinant oncolytic virus,
comprising a vesicular stomatitis virus (VSV),
wherein the glycoprotein (G protein) of VSV is deleted, and which comprises
a modified fusion protein (F protein) of Newcastle disease virus (NDV), and
the hemagglutinin neuraminidase (HN) protein of NDV,
further comprising soluble PD-1 (sPD-1).

2. The recombinant oncolytic virus according to claim 1, wherein said recombinant virus further comprises a Fc domain or a fragment thereof, preferably a Fc domain of human IgG1, IgG2, IgG3, and/or IgG4, or a fragment thereof.

3. The recombinant oncolytic virus according to claim 1 or 2, wherein said Fc domain or fragment thereof is fused to said sPD-1.

4. The recombinant oncolytic virus according to any of the foregoing claims, wherein said sPD-1 is a high affinity sPD-1 (HA-sPD-1), preferably having an affinity to its ligands PD-L1 and PD-L2 which is at least 2-fold higher, such as 45- and 30-fold higher, respectively, than an affinity of a wildtype sPD-1 to said ligands, and/or has an affinity with a K_{d} of < 3.2 µM with regard to PD-L1 and/or < 0.1 µM with regard to PD-L2.

5. The recombinant oncolytic virus according to any of the foregoing claims, wherein said sPD-1 comprises a mutation at a position selected from 132 and 41 of SEQ ID NO. 2, preferably a mutation selected from A132L, L41I, and L41V.

6. The recombinant oncolytic virus according to any of the foregoing claims, wherein said sPD-1 is HA-sPD-1-A132L having a sequence of SEQ ID NO. 3.

7. The recombinant oncolytic virus according to any of the foregoing claims, wherein the modified fusion protein (F protein) of NDV is the F3aa-modified F protein having a sequence of SEQ ID NO. 25,
and/or comprises at least one amino acid substitution in the protease cleavage site, preferably in position L289 of SEQ ID NO. 25, more preferably L289A;
and/or wherein the modified fusion protein (F protein) of NDV is the F3aa-modified F protein with an amino acid substitution L289A having SEQ ID NO. 4;
and/or wherein the G protein of VSV is replaced by the modified fusion protein having a sequence of SEQ ID NO. 4 and HN protein of NDV having a sequence of SEQ ID NO. 5.

8. A nucleic acid encoding a recombinant oncolytic virus of any of the foregoing claims.

9. The nucleic acid according to claim 8, comprising a nucleic acid encoding a Fc domain or fragment thereof, preferably having a sequence of SEQ ID NO. 7, which is fused to a nucleic acid encoding said sPD-1, preferably having a sequence of SEQ ID NO. 6, wherein, preferably, a fusion product thereof has a nucleic acid sequence of SEQ ID NO. 8.

10. A vector comprising a nucleic acid of claim 8 or 9, preferably having a sequence of SEQ ID NO. 9,
optionally further comprising any of
- a reporter gene,
such as any of HSV1-sr39TK, sodium iodide symporter (NIS), somatostatin receptor 2 (SSTR2), luciferase (Firefly or Renilla), green fluorescence protein (GFP), lacZ, and tyrosinase;
- a gene to be delivered to a tumor cell and/or tumor tissue, such as any of
an immune stimulating gene, e.g. IFN-α, IFN-β, or granulocyte macrophage colony-stimulating factor (GM-CSF), IL-12, or IL-15;
an immune checkpoint inhibitory antibody, e.g. PD-1, PD-L1, CTLA-4, LAG-3, or B7-H3; and/or
a tumor associated antigen (TAA) for vaccination (specific for the tumor being targeted);
- or combinations thereof.

11. The nucleic acid according to any of claims 8-9 or the vector according to claim 10, comprising or consisting of the nucleotide sequence of SEQ ID NO. 9 or 15,
or a nucleotide sequence having at least 60%, preferably at least 70% or 80%, more preferably at least 90% or 95% sequence identity to the nucleotide sequence of SEQ ID NO. 9 or 15,
or comprising or consisting of the nucleotide sequence of SEQ ID NO. 16,
or a nucleotide sequence having at least 60%, preferably at least 70% or 80%, more preferably at least 90% or 95% sequence identity to the nucleotide sequence of SEQ ID NO. 16.

12. A pharmaceutical composition, comprising
(i) the recombinant oncolytic virus of any of claims 1-7, the nucleic acid of any of claims 8-9 and 11, or the vector of any of claims 10-11; and
(ii) optionally, pharmaceutically acceptable carrier(s) and/or excipient(s),
(iii) optionally, a further drug, such as
a chemotherapeutic agent,
a radiotherapeutic agent,
a tumor vaccine,
an immune checkpoint inhibitor, such as anti-CTLA4,
an adoptive cell therapy system, such as T cells or dendritic cells,
a cell carrier system,
a small molecule inhibitor,
an embolization agent,
a shielding polymer.

13. The pharmaceutical composition according to claim 12, formulated for any of systemic delivery, tumor injection, intravenous administration, and intra-arterial administration,
and/or for an intradermal, subcutaneous, intramuscular, intravenous, intraosseous, intraperitoneal, intrathecal, epidural, intracardiac, intraarticular, intracavernous, intracerebral, intracerebroventricular, or intravitreal injection.

14. The recombinant oncolytic virus of any of claims 1-7, the nucleic acid of any of claims 8-9 and 11, the vector of any of claims 10-11, or the pharmaceutical composition of any of claims 12-13 for use in medicine.

15. The recombinant oncolytic virus of any of claims 1-7, the nucleic acid of any of claims 8-9 and 11, the vector of any of claims 10-11, or the pharmaceutical composition of any of claims 12-13 for use in the prevention and/or treatment of cancer, such as in the prevention and/or treatment of a cancer selected from hepatocellular carcinoma, pancreatic cancer, and melanoma.

16. Use of the recombinant oncolytic virus of any of claims 1-7, the nucleic acid of any of claims 8-9 and 11, the vector of any of claims 10-11, or the pharmaceutical composition of any of claims 12-13, as gene delivery tool, (noninvasive) imaging of virus biodistribution, and/or for tumor detection.
